# EUROPÄISCHE PATENTSCHRIFT

(11) **EP 2 148 909 B1**
(45) Veröffentlichungstag und Bekanntmachung des Hinweises auf die Patenterteilung: **30.05.2012**
(21) Anmeldenummer: 08749228.6
(22) Anmeldetag: 29.04.2008
(51) Int. Cl.: C09K 11/06, H01L 51/50

(54) **BENZANTHRACEN-DERIVATE FÜR ORGANISCHE ELEKTROLUMINESZENZVORRICHTUNGEN**
BENZANTHRACENE DERIVATIVES FOR ORGANIC ELECTROLUMINESCENT DEVICES
DERIVE BENZANTHRACENE POUR DES DISPOSITIFS ÉLECTROLUMINESCENTS ORGANIQUES

(30) Priorität: 29.05.2007 DE 102007024850
(43) Veröffentlichungstag der Anmeldung: 03.02.2010
(73) Patentinhaber: Merck Patent GmbH, 64293 Darmstadt (DE)
(72) Erfinder: STOESSEL, Philipp, 60487 Frankfurt/Main (DE); BUESING, Arne, 65929 Frankfurt am Main (DE); HEIL, Holger, 64295 Darmstadt (DE)
(86) Internationale Anmeldenummer: PCT/EP2008/003474
(87) Internationale Veröffentlichungsnummer: WO 2008/145239

(56) Entgegenhaltungen:
- EP-A- 0 728 828
- WO-A-2005/090365
- US-A1- 2006 216 633
- YAO, TUANLI; CAMPO, MARINO A.; LAROCK, RICHARD C.: "Synthesis of polycyclic aromatics and heteroaromatics via electrophilic cyclization" JOURNAL OF ORGANIC CHEMISTRY, Bd. 70, Nr. 9, 2005, Seiten 3511-3517, XP002493803
- LAROCK, RICHARD C.; DOTY, MARK J.; HAN, XIAOJUN: "Synthesis of Isocoumarins and .alpha.-Pyrones via Palladium-Catalyzed Annulation of Internal Alkynes" JOURNAL OF ORGANIC CHEMISTRY, Bd. 64, Nr. 24, 1999, Seiten 8770-8779, XP002493796
- LAROCK, RICHARD C.; DOTY, MARK J.; TIAN, QINGPING; ZENNER, JOHN M.: "Synthesis of Polycyclic Aromatic Hydrocarbons by Pd-Catalyzed Annulation of Alkynes" JOURNAL OF ORGANIC CHEMISTRY, Bd. 62, Nr. 22, 1997, Seiten 7536-7537, XP002493797
- CHAI, CHRISTINA L. L.; CHRISTEN, DETLEV; HALTON, BRIAN; NEIDLEIN, RICHARD; STARR, MALCOLM A. E.: "Studies in the cycloproparene series: reactions with radicals" AUSTRALIAN JOURNAL OF CHEMISTRY, Bd. 48, Nr. 3, 30. Oktober 1999 (1999-10-30), Seiten 577-591, XP008095814 in der Anmeldung erwähnt
- ZHDANOV, YU. A.; VERIN, S. V.; KOROBKA, I. V.; KUZNETSOV, E. V.: "2-Benzopyrylium salts. 33. 4,1'-Dimerization of 2-benzopyrylium salts; formation of benz[a]anthracenes" CHEMISTRY OF HETEROCYCLIC COMPOUNDS, Bd. 9, 1988, Seiten 974-978, XP002493943
- CHO, BONGSUP P.; HARVEY, RONALD G.: "Polycyclic fluoranthene hydrocarbons. 2. A new general synthesis" JOURNAL OF ORGANIC CHEMISTRY, Bd. 52, Nr. 26, 1987, Seiten 5668-5678, XP002493799
- GATES, MARSHALL: "Condensation of naphthoquinones with polar ethylenes. A reexamination" JOURNAL OF ORGANIC CHEMISTRY, Bd. 47, Nr. 3, 1982, Seiten 578-582, XP002493800
- MARUYAMA, KAZUHIRO; OTSUKI, TETSUO: "Photochemical reaction of 2-alkoxy-3-bromo-1,4-naphthoquinone with 1,1-diarylethylene. Novel synthetic method of 5-aryl-7,12-benz(a)anthraquinones" CHEMISTRY LETTERS, Bd. 4, Nr. 1, 5. Januar 1975 (1975-01-05), Seiten 87-88, XP008095825 in der Anmeldung erwähnt
- CLAR E ET AL: "Asymmetric annellation effects. VI", TETRAHEDRON,, vol. 18, no. 202, 1 January 1962 (1962-01-01), pages 1471-1475, XP002493801, DOI: DOI:10.1016/S0040-4020(01)99303-2
- SMITH W MAYO JR ET AL: "Isocyanates of 9-methyl- and 9,10-dimethyl-1,2-benzanthracene [12-methyl-and 7,12-dimethylbenz[a]anthracene]", JOURNAL OF THE AMERICAN CHEMICAL SOCIETY,, vol. 73, 1 January 1951 (1951-01-01), pages 319-322, XP002493802, DOI: DOI:10.1021/JA01145A105

## Beschreibung

Die vorliegende Erfindung betrifft organische Halbleiter und deren Verwendung in organischen elektronischen Vorrichtungen.

Organische Halbleiter werden für eine Reihe verschiedenartiger elektronischer Anwendungen entwickelt. Der Aufbau organischer Elektrolumineszenzvorrichtungen (OLEDs), in denen diese organischen Halbleiter als funktionelle Materialien eingesetzt werden, ist beispielsweise in US 4539507, US 5151629, EP 0676461 und WO 98/27136 beschrieben. Allerdings sind noch weitere Verbesserungen erforderlich, bevor diese Vorrichtungen für hochwertige und langlebige Displays verwendet werden können. So stellen insbesondere die unzureichende Lebensdauer und die unzureichende Effizienz blau emittierender organischer Elektrolumineszenzvorrichtungen derzeit noch ein Problem dar, welches noch nicht zufrieden stellend gelöst ist. Weiterhin ist es erforderlich, dass die Verbindungen eine hohe thermische Stabilität und eine hohe Glasübergangstemperatur aufweisen und sich unzersetzt sublimieren lassen. Insbesondere für Anwendung bei erhöhter Temperatur ist eine hohe Glasübergangstemperatur für die Erreichung hoher Lebensdauern essentiell.

Für fluoreszierende OLEDs werden gemäß dem Stand der Technik vor allem kondensierte Aromaten, insbesondere Anthracenderivate, als Host-Materialien vor allem für blau emittierende Elektrolumineszenzvorrichtungen verwendet, z. B. 9,10-Bis(2-naphthyl)anthracen (US 5935721). In WO 03/095445 und in CN 1362464 werden 9,10-Bis-(1-naphthyl)anthracen-Derivate für die Verwendung in OLEDs offenbart. Weitere Anthracenderivate sind in WO 01/076323, in WO 01/021729, in WO 04/013073, in WO 041018588, in WO 03/087023 oder in WO 04/018587 offenbart. Host-Materialien, basierend auf Arylsubstituierten Pyrenen und Chrysenen, werden in WO 04/016575 offenbart. Es ist für hochwertige Anwendungen erforderlich, verbesserte Host-Materialien zur Verfügung zu haben.

Als Stand der Technik bei blau emittierenden Verbindungen kann die Verwendung von Arylvinylaminen genannt werden (z. B. WO 04/013073, WO 04/016575, WO 04/018587). Diese Verbindungen sind jedoch thermisch instabil und lassen sich nicht unzersetzt verdampfen, was einen hohen technischen Aufwand für die OLED-Herstellung erfordert und somit einen technischen Nachteil darstellt. Daher ist es für hochwertige Anwendungen notwendig, verbesserte Emitter besonders im Bezug auf Device- und Sublimationsstabilität sowie Emissionsfarbe zur Verfügung zu haben.

Es besteht also weiterhin Bedarf an verbesserten Materialien, insbesondere Host-Materialien für fluoreszierende Emitter, vor allem für blau fluoreszierende Emitter, und fluoreszierenden Materialien, die thermisch stabil sind, die in organischen elektronischen Vorrichtungen zu guten Effizienzen und gleichzeitig zu hohen Lebensdauern führen, die bei der Herstellung und beim Betrieb der Vorrichtung zu reproduzierbaren Ergebnissen führen und die synthetisch einfach zugänglich sind. Auch bei Loch- und Elektronentransportmaterialien sind weitere Verbesserungen erforderlich.

Überraschend wurde gefunden, dass Benz[a]anthracenderivate, welche in mindestens einer der Positionen 2, 3, 4, 5 oder 6 mit einer aromatischen oder heteroaromatischen Gruppe, mit einer Diarylaminogruppe oder mit einer der anderen unten definierten Gruppen substituiert sind, sich sehr gut für die Verwendung in organischen Elektrolumineszenzvorrichtungen eignen. Mit diesen Verbindungen ist eine Steigerung der Effizienz und vor allem der Lebensdauer der organischen elektronischen Vorrichtung im Vergleich zu Materialien gemäß dem Stand der Technik möglich. Dies trifft insbesondere auf blau fluoreszierende Vorrichtungen zu. Weiterhin weisen diese Verbindungen eine hohe thermische Stabilität auf. Generell sind diese Materialien sehr gut für die Verwendung in organischen elektronischen Vorrichtungen geeignet, da sie eine hohe Glasübergangstemperatur aufweisen. Diese Materialien und deren Verwendung in organischen elektronischen Vorrichtungen sind daher der Gegenstand der vorliegenden Erfindung.

In der Literatur wurden bereits vereinzelt Benz[a]anthracenderivate, welche in diesen Positionen mit aromatischen Substituenten substituiert sind, beschrieben (z. B. K. Maruyama et al., Chem. Lett. 1975, (1), 87-88; C. L. L. Chai et al., Austr. J. Chem. 1995, 48(3), 577-591, M. C. Kloetzel et al., J. Org. Chem. 1961, 26, 1748-1754 etc.). Jedoch wurde zu diesen Verbindungen nur die Synthese und die Reaktivität untersucht. Die Anwendung dieser Verbindungen in organischen elektronischen Vorrichtungen wurde nicht vorgeschlagen. Weiterhin wurde in WO 05/090365 eine Vielzahl von Organosilanverbindungen mit polycyclischen aromatischen Gruppen unter anderem auch für die Verwendung in organischen Elektrolumineszenzvorrichtungen offenbart, darunter neben etlichen anderen Verbindungen auch eine Verbindung, welche ein Aryksubstituiertes Benzanthracen ist. Der besondere Effekt dieser Verbindungen wird dabei auf die Anwesenheit der Organosilylgruppe zurückgeführt und nicht auf das substituierte Benzanthracen-Grundgerüst.

In WO 2005/090365 werden Organosilanverbindungen offenbart, welche Benzanthracenreste tragen können. Die Verbindungen enthalten mindestens eine hydrolysierbare Gruppe zur Anbindung an Obertlächen. Weiterhin wird die Verwendung der Verbindungen in elektronischen Vorrichtungen offenbart.

Yao et al., J. Org. Chem. 2005, 70, 3511 offenbaren Syntheseverfahren für bestimmte Benzanthracenderivate.

In US 2006/0216633 werden bestimmte Bis-Anthracenderivate zur Verwendung in OLEDs offenbart.

Larock et al., J. Org. Chem. 1999, 64, 8770, und J. Org. Chem. 1997, 62, 7536, offenbaren Syntheseverfahren für bestimmte Benzanthracenderivate.

In Chai et al., Aust. J. Chem., 1995, 48, 577, werden bestimmte Benzanthracenderivate im Rahmen von Reaktivitäts-Studien hergestellt. Zhdanov et al., Chemistry of Heterocyclic Compounds, 1988, 9, 974, Cho et al., J. Org, Chem. 1987, 52, 5668, Gates et al., J. Org. Chem. 1982, 47, 578, und Maruyama et al., Chem. Lett. 1975, 4, 87, offenbaren die Synthese von bestimmten Benzanthracen-Derivaten.

Clar et al., Tetrahedron 1962, 18, 1471, offenbaren ein Benzanthracenderivat als Intermediat in der Synthese einer Azinverbindung.

Smith et al., J. Am. Chem. Soc. 1951, 73, 319, offenbaren Benzanthracenderivate für die Konjugation mit Proteinen.

Der Übersichtlichkeit halber werden im Folgenden die Struktur und die Nummerierung des Benz[a]anthracens dargestellt:

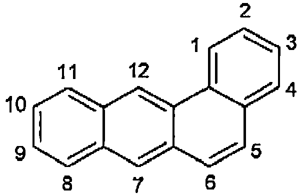

Gegenstand der Erfindung sind ungeladene Verbindungen der Formel (1), 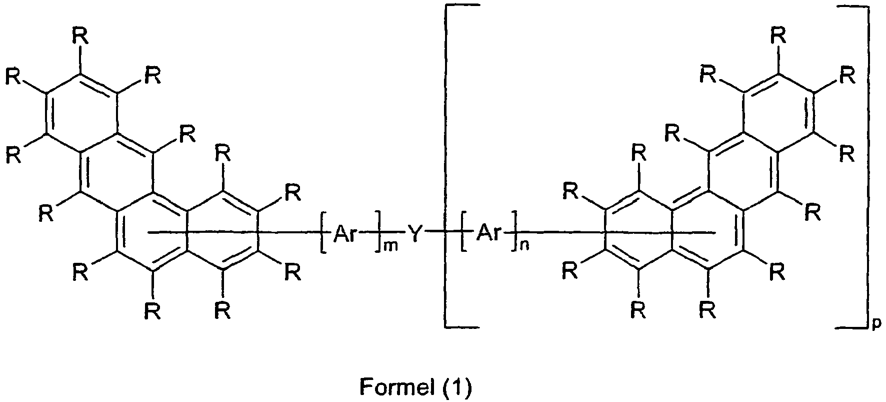 wobei die Gruppe Ar bzw. Y über eine der Positionen 2, 3, 4, 5 oder 6 des Benz[a]anthracens gebunden ist und entsprechend an dieser Position kein Rest R gebunden ist und wobei für die Symbole und Indizes gilt:
- Ar: ist bei jedem Auftreten gleich oder verschieden ein bivalentes aromatisches oder heteroaromatisches Ringsystem mit 5 bis 40 aromatischen Ringatomen, welches mit einem oder mehreren Resten R substituiert sein kann;
- Y: ist, abhängig vom Index p, ein mono-, bi-, tri-, tetra-, penta- oder hexavalentes aromatisches oder heteroaromatisches Ringsystem mit 5 bis 40 aromatischen Ringatomen, welches durch einen oder mehrere Reste R substituiert sein kann; oder Y ist, wenn p = 0 ist, eine Gruppe N(Ar¹)₂, C(=O)Ar¹ oder P(=O)(Ar¹)₂; oder Y ist, wenn p = 1 ist, eine Einfachbindung oder C=O, O, S, SO, SO₂, NR¹, NAr¹, PAr¹, P(=O)Ar¹, P(=S)Ar¹, O-B(Ar¹)-O, O-BR¹-O, BAr¹, -CR¹=CR¹-, -C≡C-, eine Alkylen- oder Alkylidengruppe mit 1 bis 20 C-Atomen, die jeweils auch mit einem oder mehreren Resten R¹ substituiert sein kann, wobei eine oder mehrere nicht benachbarte CH₂-Gruppen durch R¹C=CR¹, C≡C, Si(R¹)₂, Ge(R¹)₂, Sn(R¹)₂, C=O, C=S, C=Se, C=NR¹, P(=O)(R¹), SO, SO₂, NR¹, O, S oder CONR¹ ersetzt sein können und wobei ein oder mehrere H-Atome durch F, Cl, Br, I, CN oder NO₂ ersetzt sein können; oder Y ist, wenn p = 2 ist, gleich B, B₃O₃, CR¹, CAr¹, N, P, P=O oder P=S;
- R: ist gleich oder verschieden bei jedem Auftreten H, D, F, Cl, Br, I, CHO, N(Ar¹)₂, N(R²)₂, C(=O)Ar¹, P(Ar¹)₂, P(=O)(Ar¹)₂, S(=O)Ar¹, S(=O)₂Ar¹, CR¹=CR¹Ar¹, CN, NO₂, Si(R¹)₃, B(OAr¹)₂, B(OR¹)₂, OSO₂R¹, OH, eine geradkettige Alkyl- oder Thioalkoxygruppe mit 1 bis 40 C-Atomen oder eine geradkettige Alkoxygruppe mit 2 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R¹ substituiert sein können, wobei eine oder mehrere nicht benachbarte CH₂-Gruppen durch R¹C=CR¹, C≡C, Si(R¹)₂, Ge(R¹)₂, Sn(R¹)₂, C=O, C=S, C=Se, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere nicht-aromatische Reste R substituiert sein kann, oder eine Aryloxy- oder Heteroaryloxygruppe mit 5 bis 60 aromatischen Ringatomen, die durch einen oder mehrere nicht-aromatische Reste R substituiert sein kann, oder eine Kombination dieser Systeme; dabei können zwei oder mehrere benachbarte Substituenten R auch miteinander ein mono- oder polycyclisches aliphatisches Ringsystem bilden;
- Ar¹: ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R substituiert sein kann; dabei können auch zwei Reste Ar¹, welche an dasselbe Stickstoff- oder Phosphoratom binden, durch eine Einfachbindung oder eine Brücke, ausgewählt aus B(R¹), C(R¹)₂, Si(R¹)₂, C=O, C=NR¹, C=C(R¹)₂, O, S, S=O, SO₂, N(R¹), P(R¹) und P(=O)R¹, miteinander verknüpft sein;
- R¹: ist bei jedem Auftreten gleich oder verschieden H oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere benachbarte Substituenten R¹ auch miteinander ein mono- oder polycyclisches aliphatisches oder aromatisches Ringsystem bilden;
- m, n: ist bei jedem Auftreten gleich oder verschieden 0, 1, 2 oder 3;
- p: ist 0, 1, 2, 3, 4 oder 5;
dabei sind die folgenden Verbindungen von der Erfindung ausgenommen:

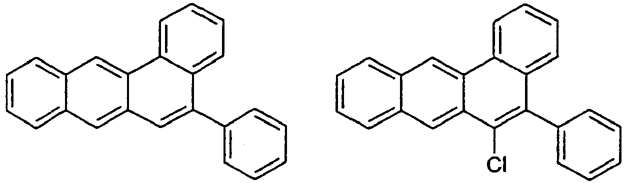

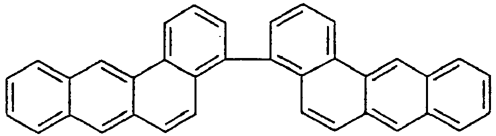

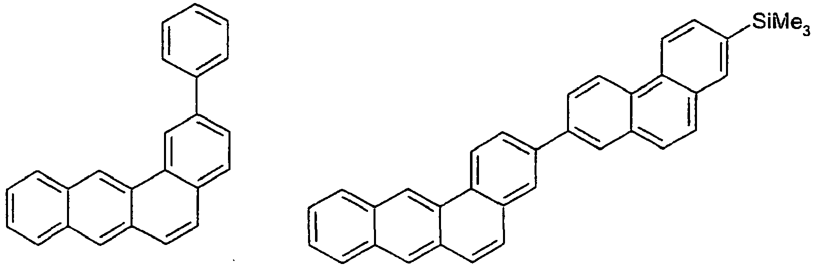

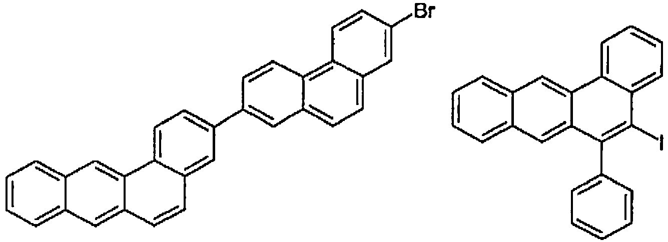

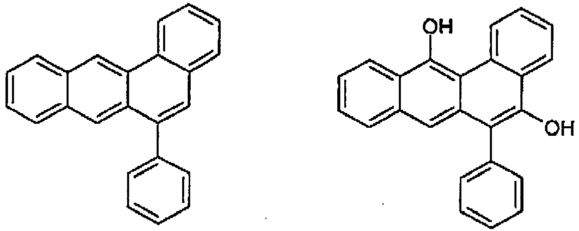

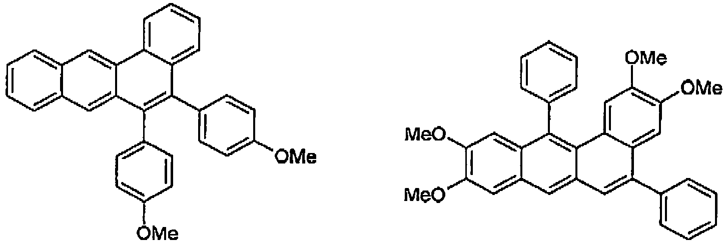

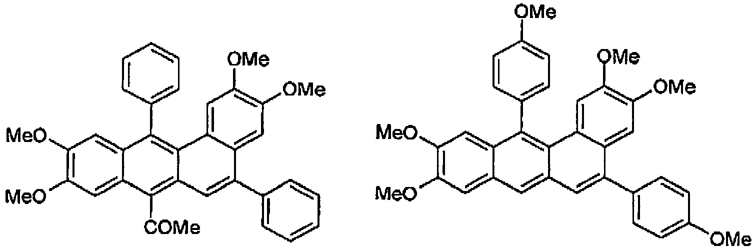

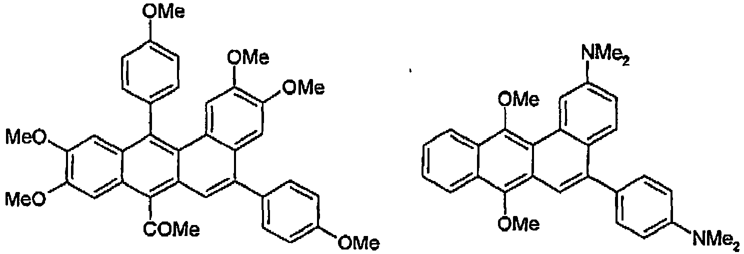

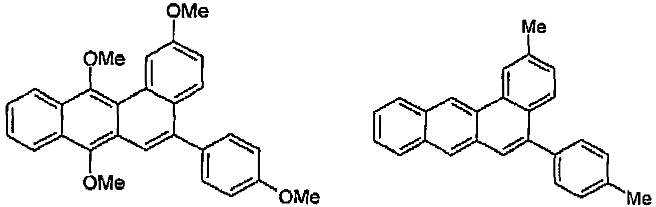

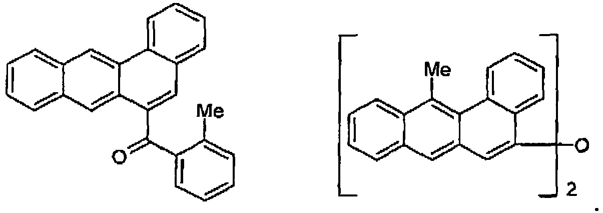

Bevorzugt weisen die Verbindungen gemäß Formel (1) eine Glasübergangstemperatur Tg von größer als 70°C auf, besonders bevorzugt größer als 100°C, ganz besonders bevorzugt größer als 130°C.

Eine Arylgruppe im Sinne dieser Erfindung enthält 6 bis 60 C-Atome; eine Heteroarylgruppe im Sinne dieser Erfindung enthält 2 bis 60 C-Atome und mindestens 1 Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Pyren, Chinolin, Isochinolin, Benzimidazol, Phenanthren, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 2 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (bevorzugt weniger als 10 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, Stilben, Benzophenon, etc. als aromatische Ringsysteme im Sinne dieser Erfindung verstanden werden. Ebenso werden unter einem aromatischen bzw. heteroaromatischen Ringsystem Systeme verstanden, in denen mehrere Aryl- bzw. Heteroarylgruppen durch Einfachbindungen miteinander verknüpft sind, beispielsweise Biphenyl, Terphenyl oder Bipyridin.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 40 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Naphthacen, Pentacen, Benzanthracen, Dibenzanthracen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthroimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Eine bevorzugte Ausführungsform der vorliegenden Erfindung sind Verbindungen gemäß Formel (2), 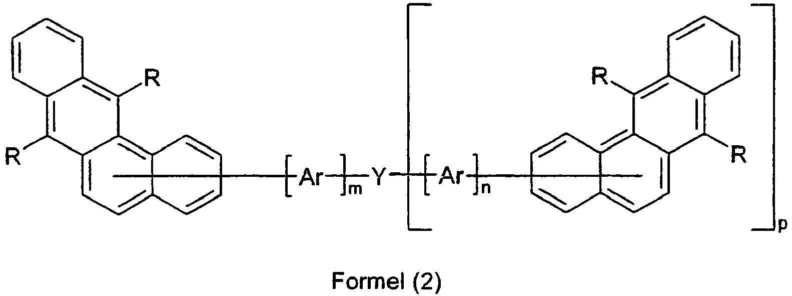 wobei die Gruppe Ar bzw. Y über eine der Positionen 2, 3, 4, 5 oder 6 des Benz[a]anthracens gebunden ist und wobei die Symbole und Indizes dieselbe Bedeutung haben, wie oben beschrieben. Wenn nur einer der beiden Substituenten R an einem Benz[a]anthracen in Formel (2) ungleich Wasserstoff ist, so ist dies bevorzugt der Substituent in 7-Position des Benz[a]anthracens.

Wie oben beschrieben ist die Gruppe Ar oder Y an das Benz[a]anthracen über die Position 2, 3, 4, 5 oder 6 gebunden. Wenn die Verbindung gemäß Formel (1) oder (2) mehrere Benz[a]anthraceneinheiten enthält, wenn also der Index p 1 oder größer ist, kann jede dieser Einheiten über dieselbe Position des Benz[a]anthracens oder über unterschiedliche Positionen des Benz[a]anthracens gebunden sein. Eine Bindung über dieselbe Position des Benz[a]anthracens hat den Vorteil, dass die Verbindungen synthetisch leichter zugänglich sind. Eine Bindung über unterschiedliche Positionen des Benz[a]anthracens führt zu unsymmetrischen Verbindungen, die im Allgemeinen den Vorteil aufweisen, dass sie besser löslich sind und eine höhere Glasübergangstemperatur aufweisen.

In einer bevorzugten Ausführungsform der Erfindung ist die Gruppe Ar bzw. Y in Formel (1) bzw. Formel (2) über die Position 2 oder 3 am Benz[a]anthracen gebunden, so dass das Benzanthracen keine Substituenten bzw. Protonen in peri-Stellung zu Ar bzw. Y aufweist. In diesen Fällen ist auch bei sterisch anspruchsvollen Gruppen Ar bzw. Y, wie z. B. Anthracen, keine Bildung von Atropisomeren um die Benz[a]-anthracen-Ar- bzw. die Benz[a]anthracen-Y-Bindung möglich.

In einer weiteren bevorzugten Ausführungsform der Erfindung ist die Gruppe Ar bzw. Y in Formel (1) bzw. Formel (2) über die Position 4, 5 oder 6 am Benz[a]anthracen gebunden, so dass das Benzanthracen entweder einen Substituenten oder ein Proton in peri-Stellung zu Ar bzw. Y aufweist. In diesen Fällen ist bei sterisch anspruchsvollen Gruppen Ar bzw. Y, wie z. B. Anthracen, die Bildung von Atropisomeren um die Benz[a]anthracen-Ar- bzw. die Benz[a]anthracen-Y-Bindung möglich.

Dabei soll die peri-Stellung am Benz[a]anthracen im Sinne dieser Erfindung analog zum Naphthalin definiert werden.

Besonders bevorzugte Ausführungsformen der Strukturen gemäß Formel (1) sind die Strukturen gemäß den Formeln (3) bis (24), 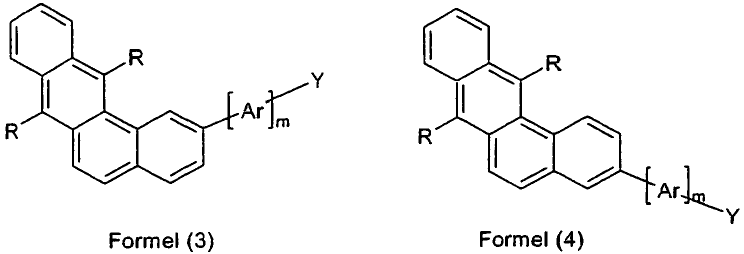 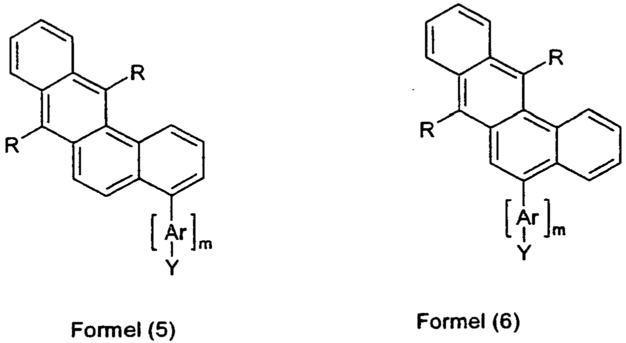 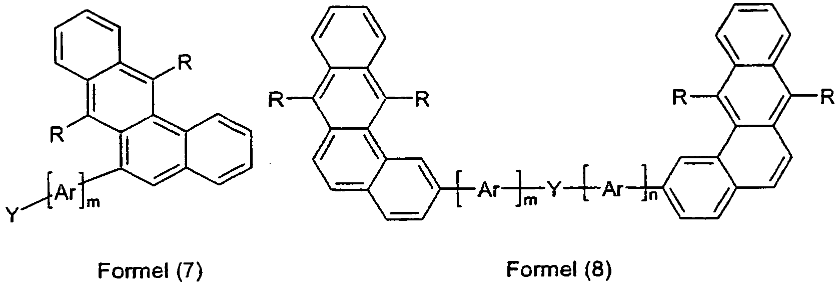 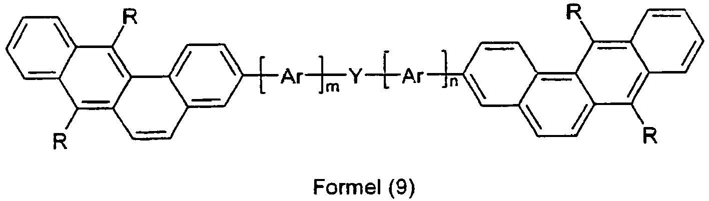 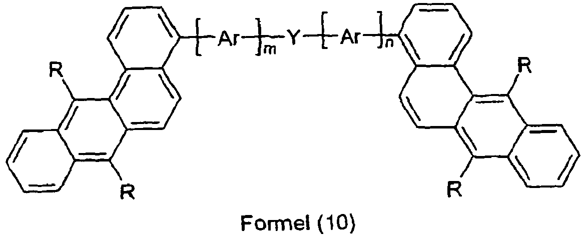 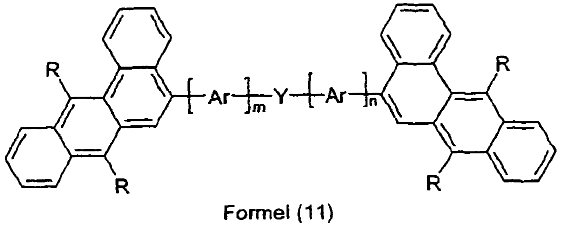 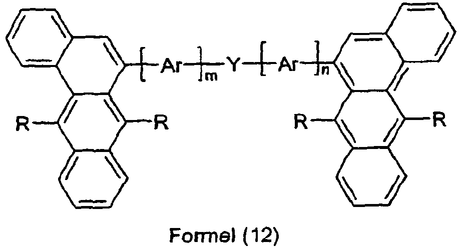 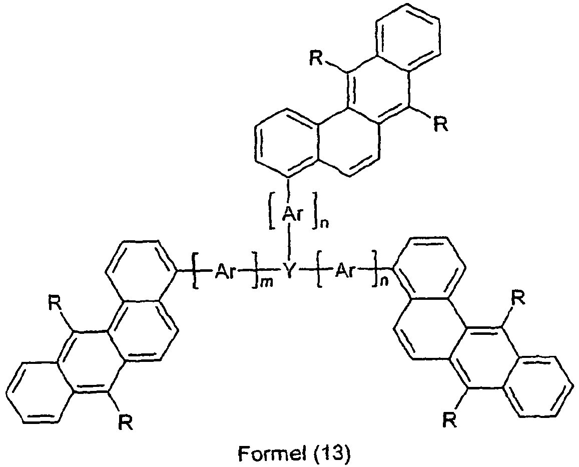 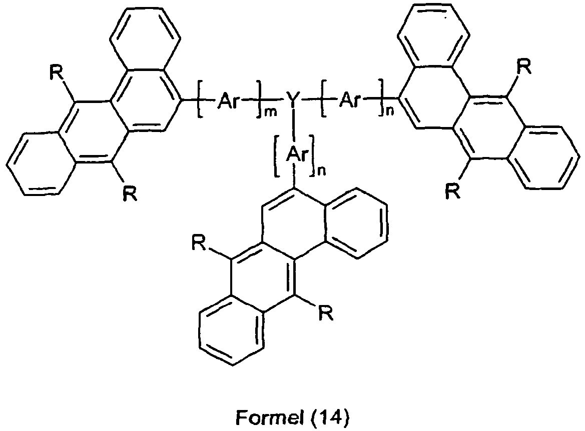 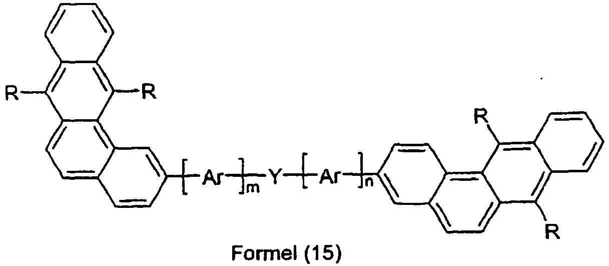 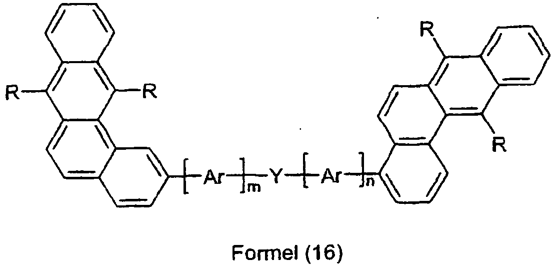 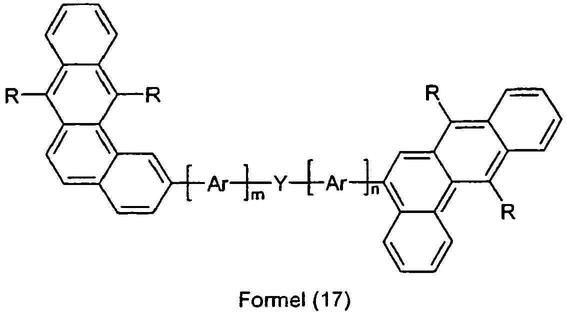 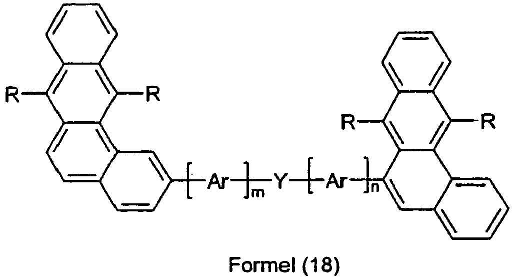 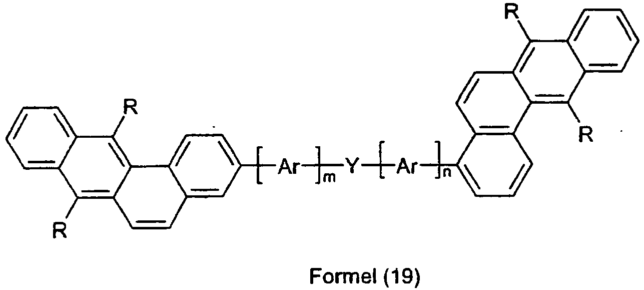 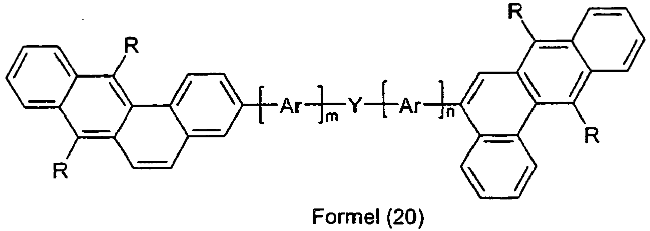 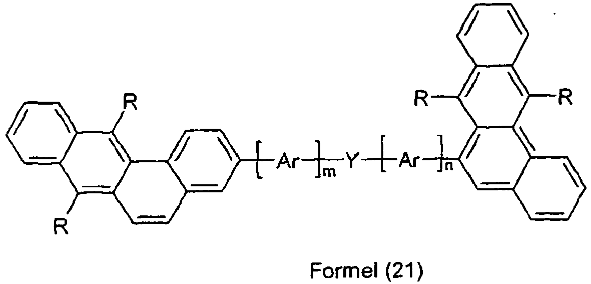 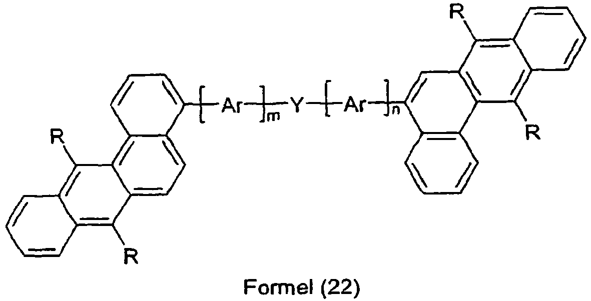 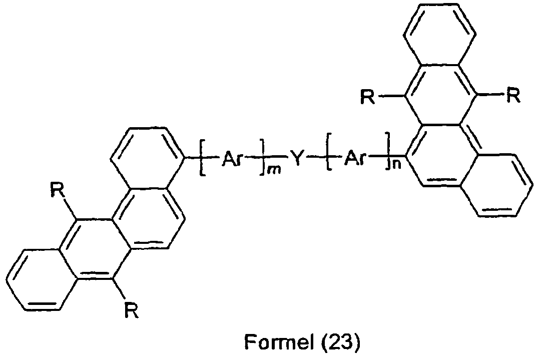 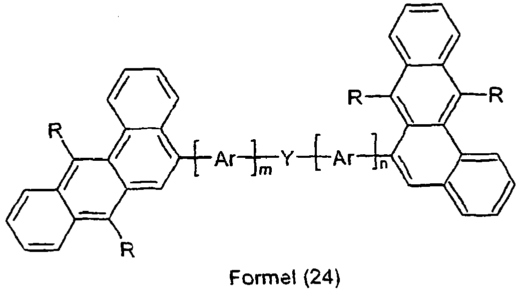 wobei die Symbole und Indizes dieselbe Bedeutung haben, wie oben beschrieben, wobei der Benz[a]anthracen-Grundkörper statt Wasserstoff auch Deuterium tragen kann und wobei die Gruppe Y in den Formeln (3) bis (7) für ein monovalentes aromatisches oder heteroaromatisches Ringsystem oder eine Gruppe N(Ar¹)₂ steht.

In den Strukturen gemäß Formel (3) bis (24) ist der Substituent R in 12-Position bevorzugt Wasserstoff oder Deuterium, insbesondere Wasserstoff. Besonders bevorzugt sind beide Substituenten R, also die Substituenten in 7- und in 12-Position, Wasserstoff oder Deuterium, insbesondere Wasserstoff.

Bevorzugt sind weiterhin Verbindungen gemäß Formel (1) bzw. gemäß Formel (3) bis (7), in denen das Symbol Y für ein rein aromatisches oder heteroaromatisches System steht, welches keine nicht-aromatischen Gruppen aufweist und welches kondensiert oder nicht-kondensiert sein kann, oder für eine aromatische Aminogruppe. Besonders bevorzugt ist die Gruppe Y aus den Aryl- bzw. Heteroarylgruppen Benzol, Naphthalin, Anthracen, Carbazol, Phenanthren, Benzanthracen, Chrysen, Pyren, Phenanthrolin, 1,3,5-Triazin, Benzimidazol und Phenanthroimidazot aufgebaut oder stellt, wenn p = 0 ist, eine Gruppe N(Ar¹)₂ dar. Ganz besonders bevorzugte Gruppen Y für p = 0 bzw. für Verbindungen der Formel (3) bis (7) sind die Gruppen der folgenden Formeln (25) bis (33), 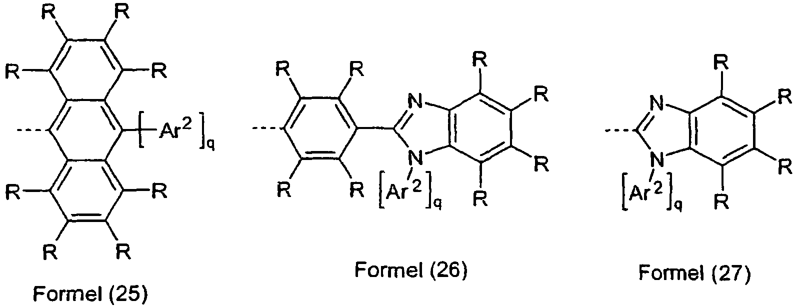 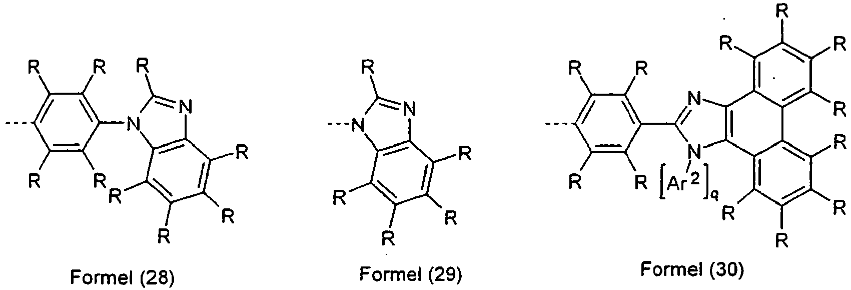 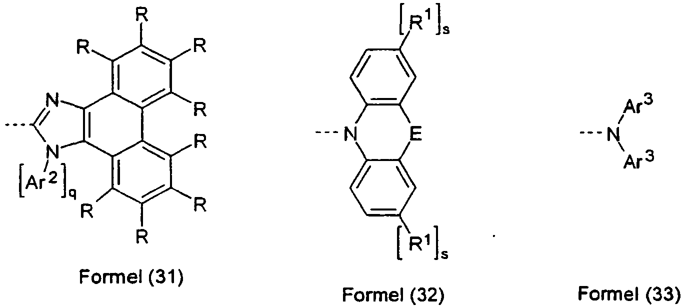 wobei R und R¹ die oben aufgeführte Bedeutung haben und weiterhin gilt:
- Ar²: ist eine Aryl- oder Heteroarylgruppe mit 5 bis 16 aromatischen Ringatomen, bevorzugt Phenyl, 1-Naphthyl, 2-Naphthyl, 9-Ahthryl, Chrysenyl, 1-Pyrenyl, 2-Pyrenyl, 2-Phenanthrenyl, 3-Phenanthrenyl, 9-Phenanthrenyl, 2-Benzimidazol oder Fluoranthenyl, welche jeweils mit einem oder mehreren Resten R¹ substituiert sein kann, oder in Formel (25) eine Gruppe der Formel (32) oder (33);
- Ar³: ist gleich oder verschieden bei jedem Auftreten eine Aryl- oder Heteroarylgruppe mit 5 bis 20 aromatischen Ringatomen oder eine Triarylamingruppe mit 15 bis 30 aromatischen Ringatomen, welche jeweils mit einem oder mehreren Resten R¹ substituiert sein kann, bevorzugt eine Aryl- oder Heteroarylgruppe mit 6 bis 14 aromatischen Ringatomen oder eine Triarylamingruppe mit 18 bis 30 aromatischen Ringatomen, bevorzugt mit 18 bis 22 aromatischen Ringatomen, welche jeweils mit einem oder mehreren Resten R¹ substituiert sein kann;
- E: steht für eine Einfachbindung, O, S, N(R¹) oder C(R¹)₂, wobei die beiden Reste R¹ durch Ringbildung auch ein Spirosystem aufspannen können;
- q: ist 1, 2 oder 3;
- s: ist bei jedem Auftreten gleich oder verschieden 0 oder 1.

Besonders bevorzugte Systeme für q = 2 sind ortho-Biphenyl, meta-Biphenyl, para-Biphenyl, Penylen-1-naphthyl, Phenylen-2-naphthyl, N-Phenyl-2-benzimidazol, 2-Fluorenyl und 2-Spirobifluorenyl.

Besonders bevorzugt steht Ar³ in Formel (33) gleich oder verschieden für Phenyl, 1-Naphthyl, 2-Naphthyl, 2-, 3- oder 4-Triphenylamin, 1- oder 2-Naphthyldiphenylamin, welches jeweils über die Naphthyl- oder die Phenylgruppe gebunden sein kann, oder 1- oder 2-Dinaphthylphenylamin, welches jeweils über die Naphthyl- oder die Phenylgruppe gebunden sein kann. Diese Gruppen können jeweils durch eine oder mehrere Alkylgruppen mit 1 bis 4 C-Atomen oder durch eine oder mehrere cyclische oder bicyclische Alkylgruppen mit 3 bis 8 C-Atomen oder durch Fluor substituiert sein.

Bevorzugt sind weiterhin Verbindungen gemäß Formel (1) bis (24), in denen das Symbol Ar, gleich oder verschieden bei jedem Auftreten, für ein bivalentes aromatisches oder heteroaromatisches Ringsystem steht, welche ausgewählt ist aus 1,2-Phenylen, 1,3-Phenylen, 1,4-Phenylen, 1,4-Naphthylen, 9,10-Anthrylen, 2,7-Phenanthrenylen, 3,6-Phenanthrenylen, 1,6-Pyrenylen, 2,7-Pyrenylen, 2,6-Pyridinylen, 2,5-Pyridinylen, 2,2'-Biphenyl, 3,3'-Biphenyl, 4,4'-Biphenyl, 2,7-Fluorenyl oder 2,7-Spirobifluorenyl. Dabei sei an dieser Stelle ausdrücklich betont, dass die Gruppen Ar in Formel (1) bzw. in Formel (2) bzw. in Formel (8) bis (24) gleich oder verschieden gewählt werden können.

Bevorzugt sind weiterhin Verbindungen gemäß Formel (1) und Formel (2) mit p = 1 bzw. gemäß Formel (8) bis (12) und (15) bis (24), in denen das Symbol Y für eine Einfachbindung oder eine bivalente Gruppe, ausgewählt aus C=O, O, NAr², POAr², O-B(Ar²)-O, einer bivalenten Alkylen- oder Alkylidengruppe mit 1 bis 6 C-Atomen oder einem bivalenten aromatischen oder heteroaromatischen Ringsystem mit 5 bis 14 aromatischen Ringatomen, steht. Bevorzugt sind weiterhin Verbindungen gemäß Formel (1) und Formel (2) mit p = 2 bzw. gemäß Formel (13) oder (14), in denen das Symbol Y für N oder ein trivalentes aromatisches oder heteroaromatisches Ringsystem mit 5 bis 14 aromatischen Ringatomen steht, insbesondere für 1,3,5-Benzol oder 1,3,5-Triazin. In Verbindungen gemäß Formel (1) und Formel (2) mit p > 2 steht Y bevorzugt für ein entsprechend höhervalentes aromatisches oder heteroaromatisches Ringsystem mit 5 bis 14 aromatischen Ringatomen.

In den Verbindungen gemäß Formel (1) oder (2) bzw. gemäß den Formeln (8) bis (24) können alle Indizes m und n gleich gewählt sein, was zu symmetrischen Verbindungen führt, oder sie können verschieden gewählt sein, was zu unsymmtrischen Verbindungen führt. Wie vorne bereits erwähnt, haben symmetrische Verbindungen den Vorteil der leichteren synthetischen Zugänglichkeit und unsymmetrische Verbindungen den Vorteil der besser geeigneten physikalischen Eigenschaften.

Bevorzugt sind weiterhin Verbindungen gemäß Formel (1) oder (2) bzw. gemäß den Formeln (3) bis (24), in denen die Indizes m und n für 0 oder 1 stehen.

Bevorzugt sind weiterhin Verbindungen gemäß Formel (1) oder (2) bzw. gemäß den Formeln (3) bis (24), in denen der Index p für 0, 1 oder 2 steht, besonders bevorzugt für 0 oder 1.

Wenn ein Rest R für eine Gruppe N(Ar¹)₂ steht, so ist diese Gruppe bevorzugt ausgewählt aus den oben abgebildeten Gruppen der Formel (32) oder der Formel (33),

Beispiele für bevorzugte Verbindungen gemäß der Formel (1) sind die im Folgenden abgebildeten Strukturen (1) bis (338).

| | | |
|---|---|---|
| 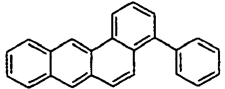 | 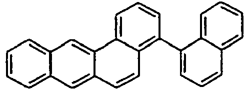 | |
| (1) | (2) | |
| 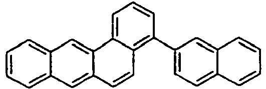 | 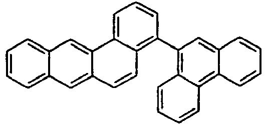 | |
| (3) | (4) | |
| 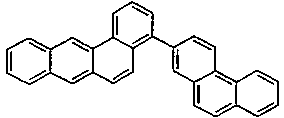 | 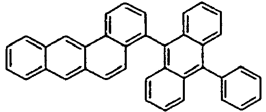 | |
| (5) | (6) | |
| 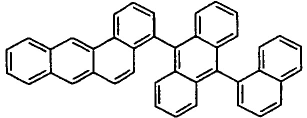 | 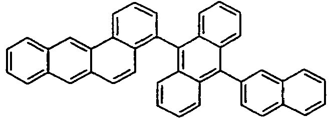 | |
| (7) | (8) | |
| 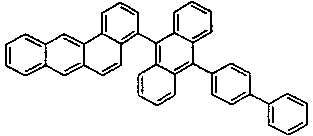 | 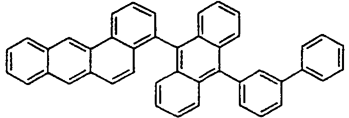 | |
| (9) | (10) | |
| 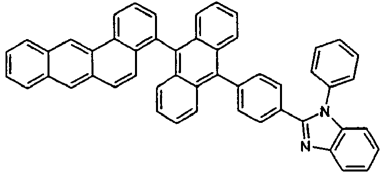 | 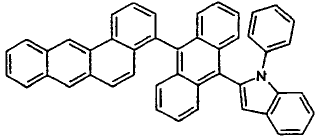 | |
| (11) | (12) | |
| 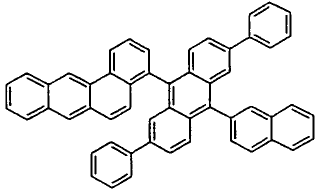 | 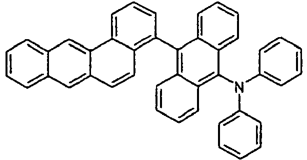 | |
| (13) | (14) | |
| 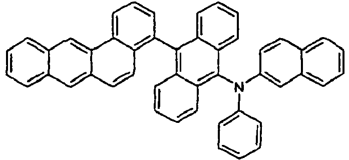 | 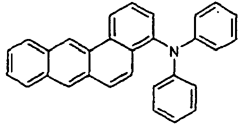 | |
| (15) | (16) | |
| 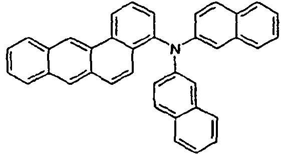 | 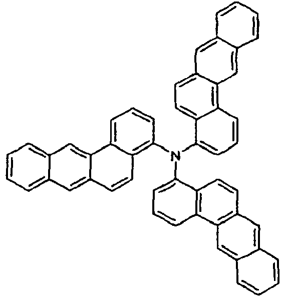 | |
| (17) | (18) | |
| 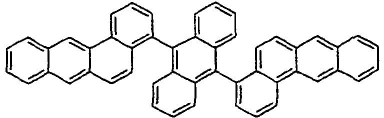 | 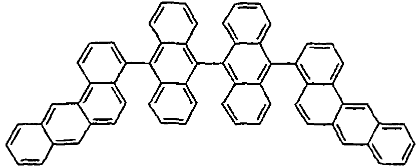 | |
| (19) | (20) | |
| 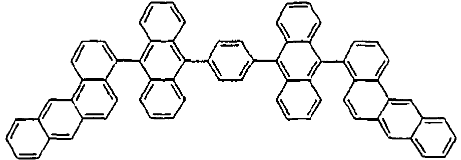 | 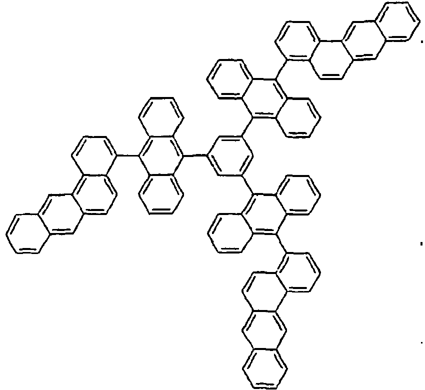 | |
| (21) | (22) | |
| 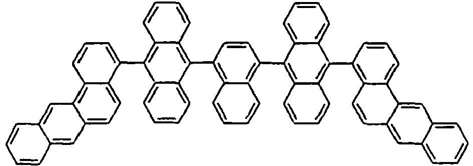 | 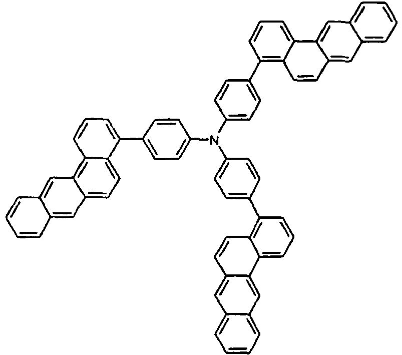 | |
| (23) | (24) | |
| 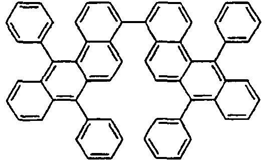 | 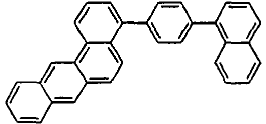 | |
| (25) | (26) | |
| 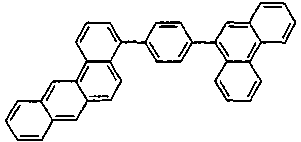 | 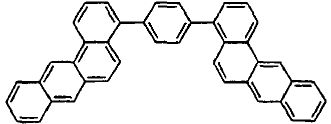 | |
| (27) | (28) | |
| 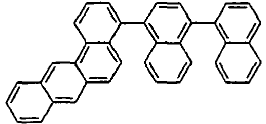 | 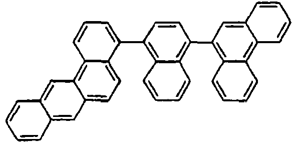 | |
| (29) | (30) | |
| 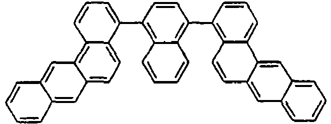 | 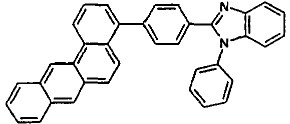 | |
| (31) | (32) | |
| 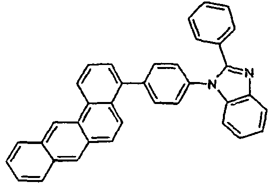 | 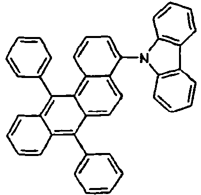 | |
| (33) | (34) | |
| 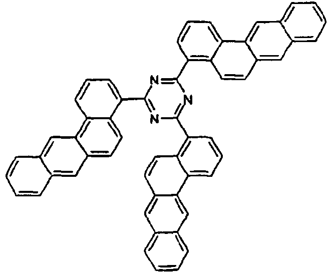 | 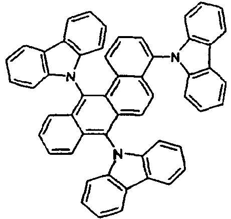 | |
| (35) | (36) | |
| 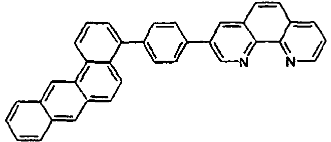 | 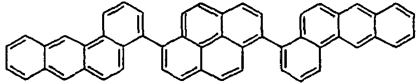 | |
| (37) | (38) | |
| 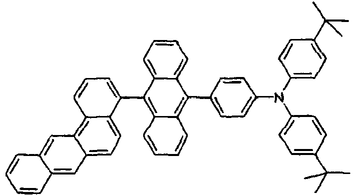 | 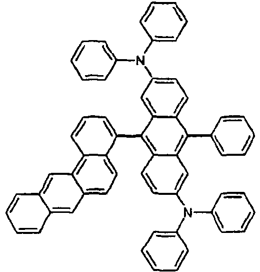 | |
| (39) | (40) | |
| 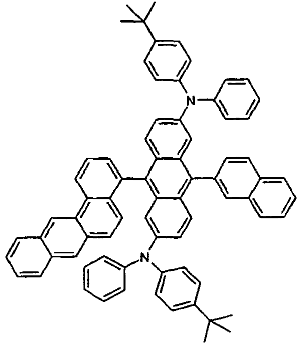 | 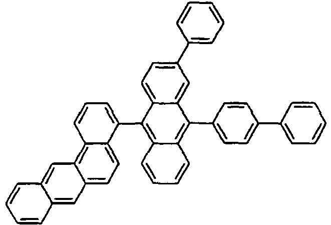 | |
| (41) | (42) | |
| 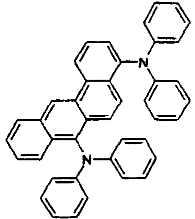 | 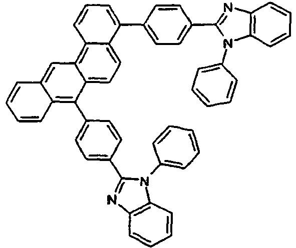 | |
| (43) | (44) | |
| 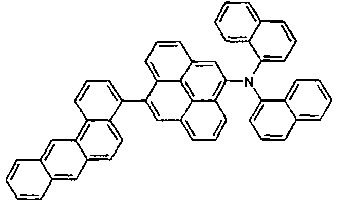 | 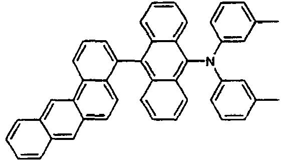 | |
| (45) | (46) | |
| 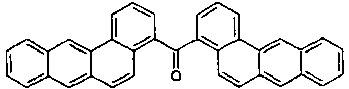 | 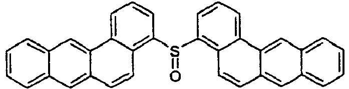 | |
| (47) | (48) | |
| 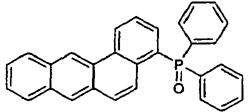 | 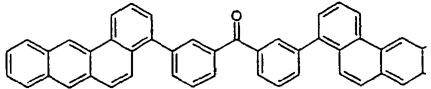 | |
| (49) | (50) | |
| 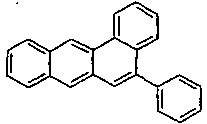 | 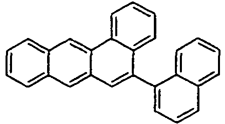 | |
| (51) | (52) | |
| 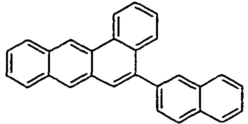 | 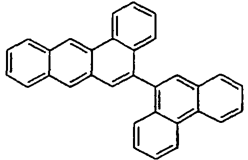 | |
| (53) | (54) | |
| 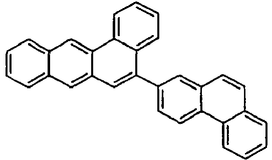 | 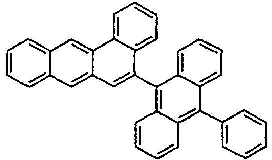 | |
| (55) | (56) | |
| 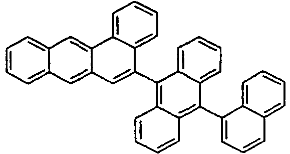 | 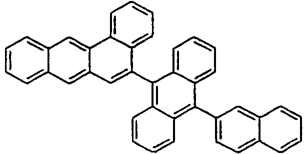 | |
| (57) | (58) | |
| 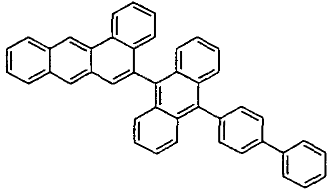 | 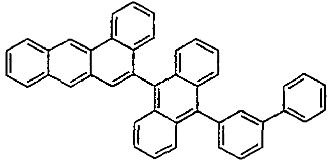 | |
| (59) | (60) | |
| 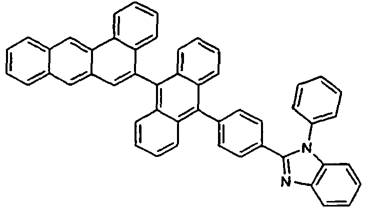 | 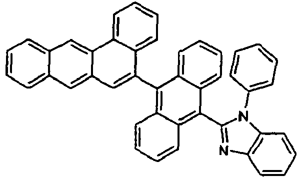 | |
| (61) | (62) | |
| 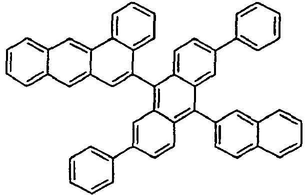 | 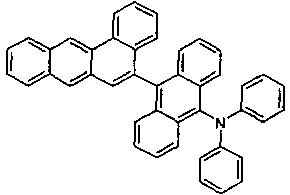 | |
| (63) | (64) | |
| 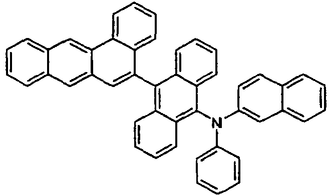 | 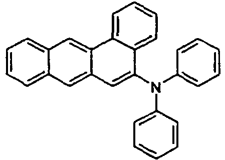 | |
| (65) | (66) | |
| 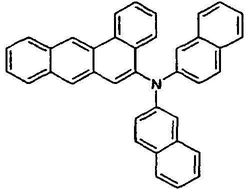 | 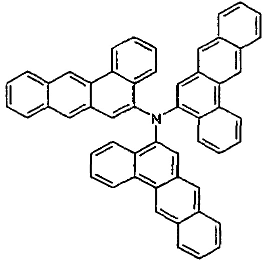 | |
| (67) | (68) | |
| 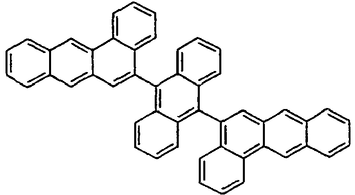 | 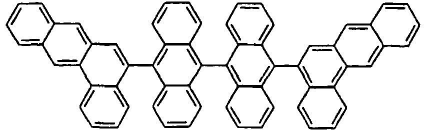 | |
| (69) | (70) | |
| 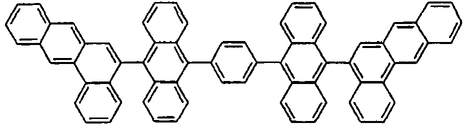 | 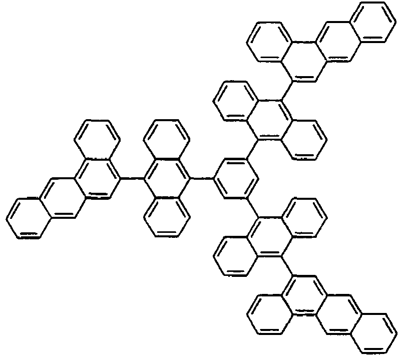 | |
| (71) | (72) | |
| 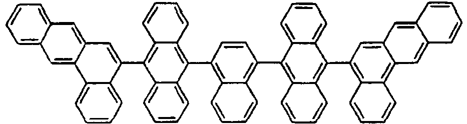 | 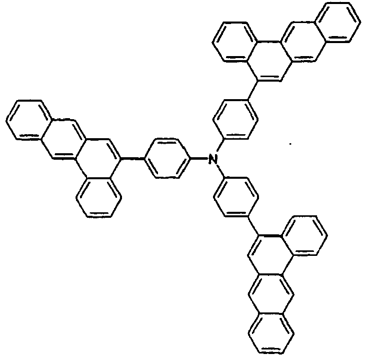 | |
| (73) | (74) | |
| 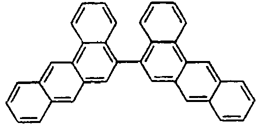 | 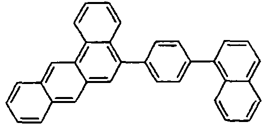 | |
| (75) | (76) | |
| 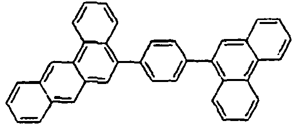 | 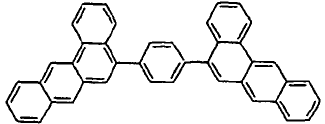 | |
| (77) | (78) | |
| 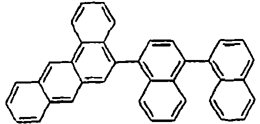 | 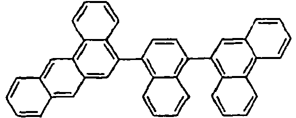 | |
| (79) | (80) | |
| 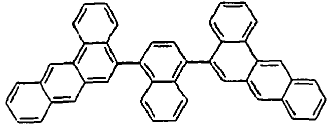 | 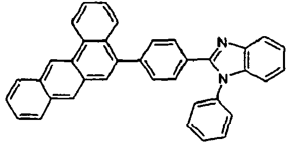 | |
| (81) | (82) | |
| 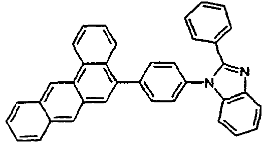 | 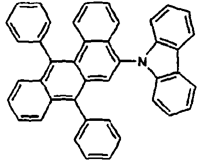 | |
| (83) | (84) | |
| 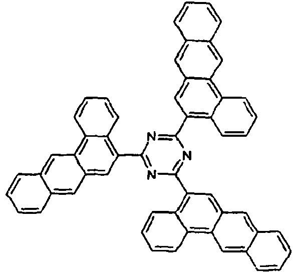 | 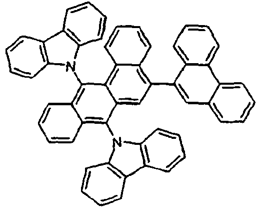 | |
| (85) | (86) | |
| 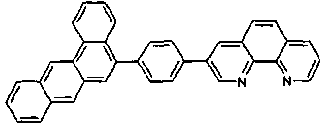 | 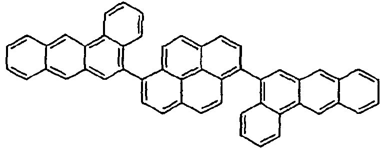 | |
| (87) | (88) | |
| 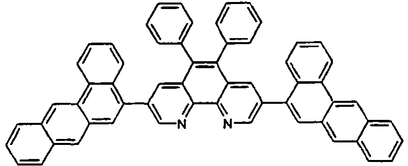 | 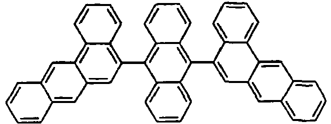 | |
| (89) | (90) | |
| 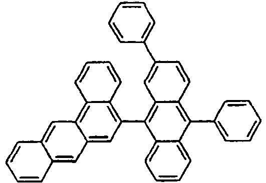 | 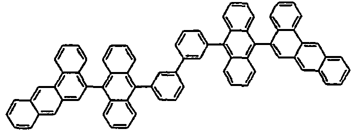 | |
| (91) | (92) | |
| 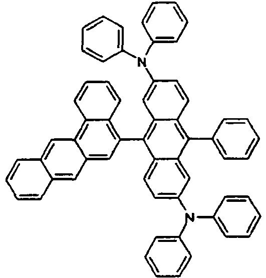 | 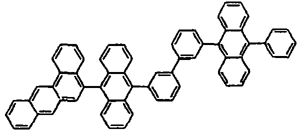 | |
| (93) | (94) | |
| 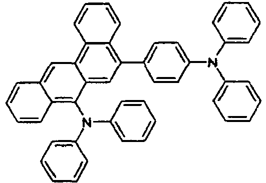 | 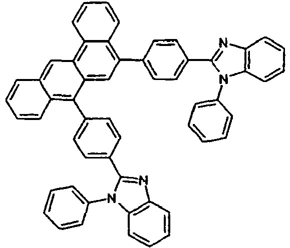 | |
| (95) | (96) | |
| 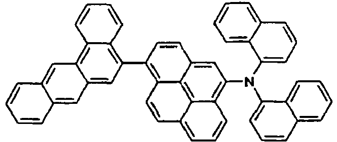 | 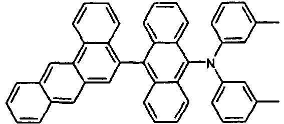 | |
| (97) | (98) | |
| 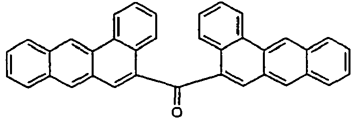 | 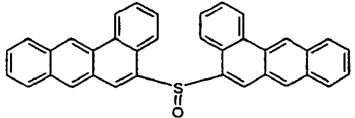 | |
| (99) | (100) | |
| 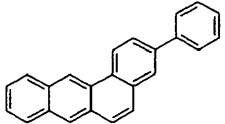 | 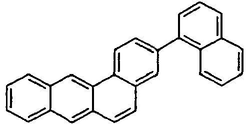 | |
| (101) | (102) | |
| 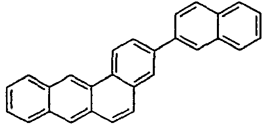 | 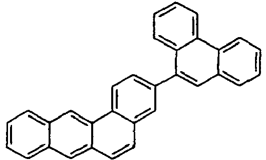 | |
| (103) | (104) | |
| 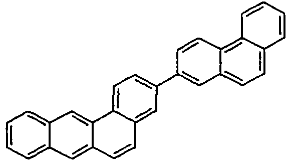 | 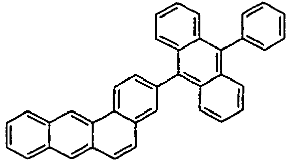 | |
| (105) | (106) | |
| 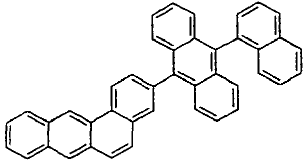 | 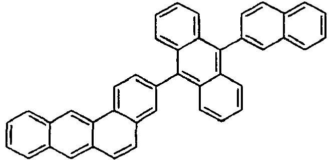 | |
| (107) | (108) | |
| 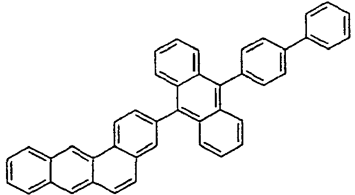 | 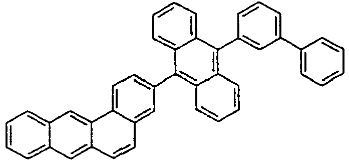 | |
| (109) | (110) | |
| 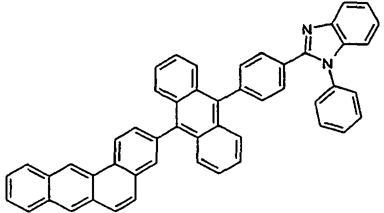 | 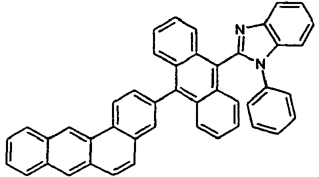 | |
| (111) | (112) | |
| 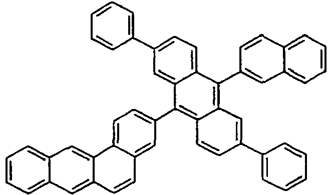 | 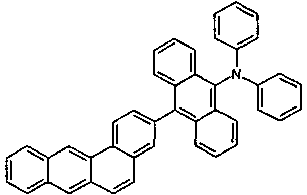 | |
| (113) | (114) | |
| 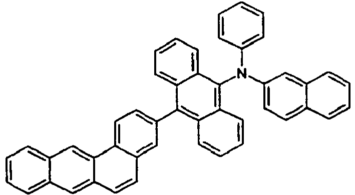 | 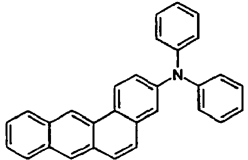 | |
| (115) | (116) | |
| 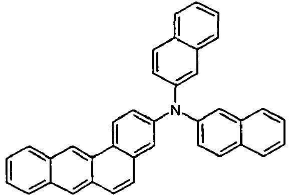 | 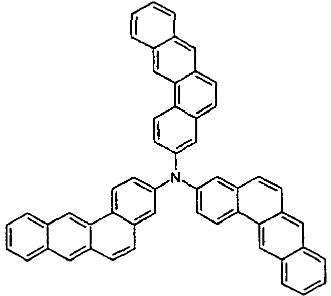 | |
| (117) | (118) | |
| 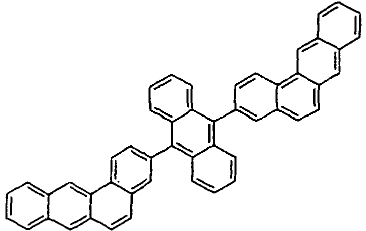 | 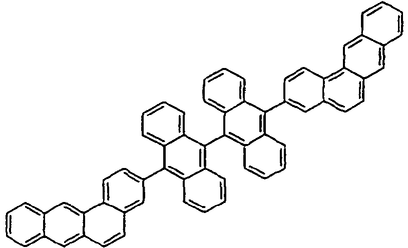 | |
| (119) | (120) | |
| 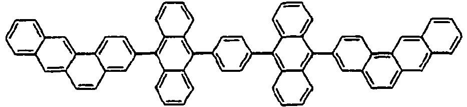 | 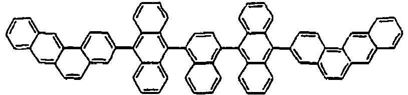 | |
| (121) | (122) | |
| 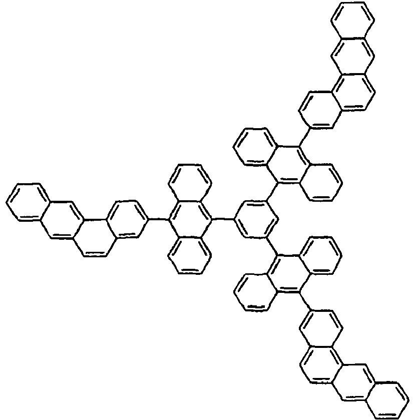 | 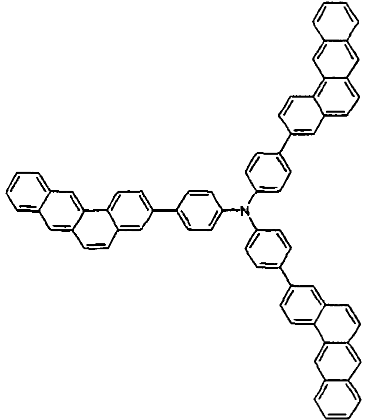 | |
| (123) | (124) | |
| 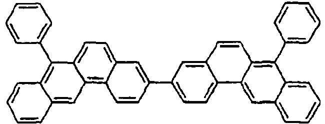 | 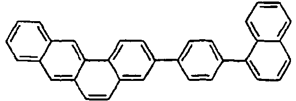 | |
| (125) | (126) | |
| 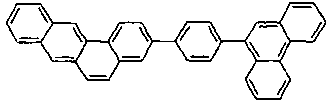 | 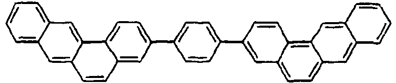 | |
| (127) | (128) | |
| 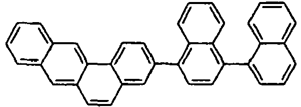 | 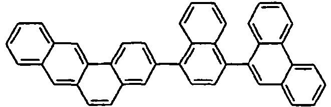 | |
| (129) | (130) | |
| 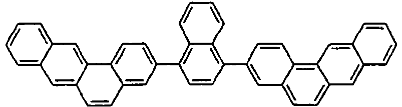 | 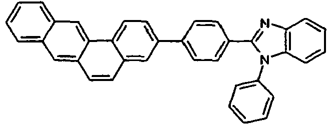 | |
| (131) | (132) | |
| 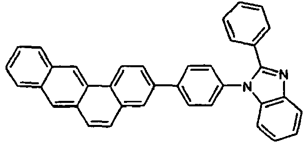 | 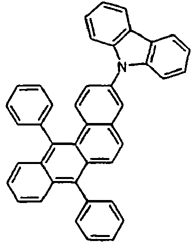 | |
| (133) | (134) | |
| 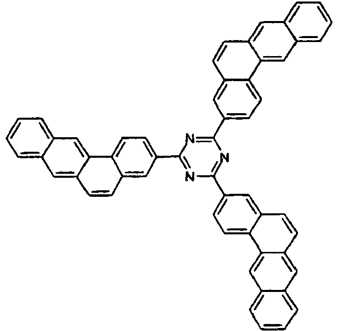 | 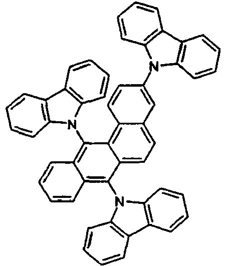 | |
| (135) | (136) | |
| 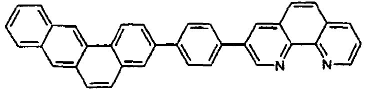 | 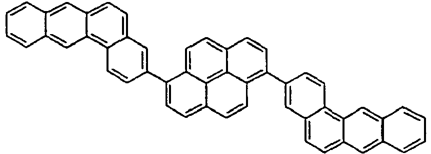 | |
| (137) | (138) | |
| 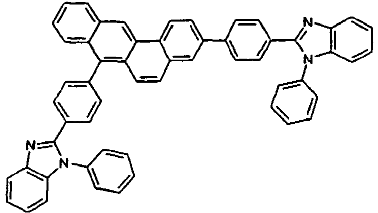 | 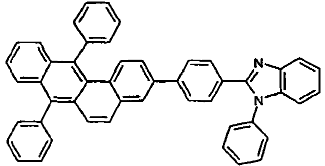 | |
| (139) | (140) | |
| 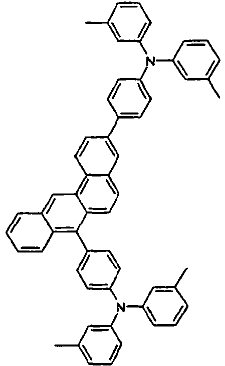 | 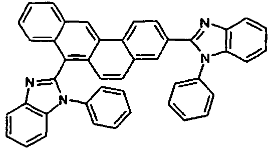 | |
| (141) | (142) | |
| 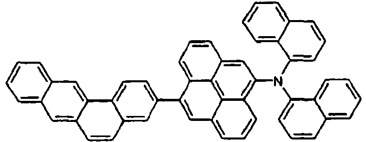 | 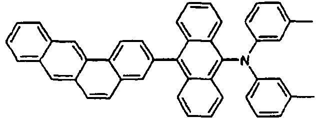 | |
| (143) | (144) | |
| 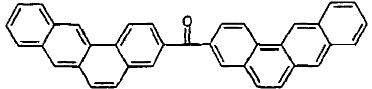 | 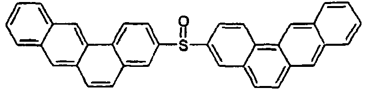 | |
| (145) | (146) | |
| 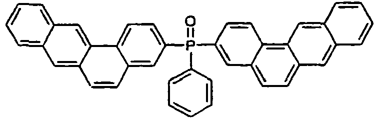 | 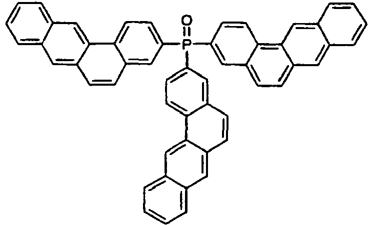 | |
| (147) | (148) | |
| 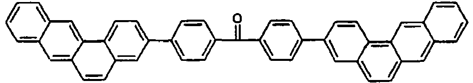 | 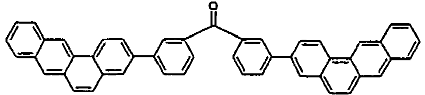 | |
| (149) | (150) | |
| 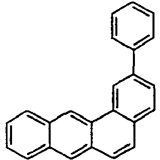 | 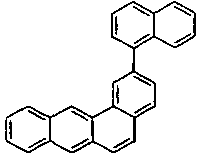 | |
| (151) | (152) | |
| 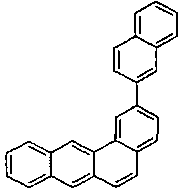 | 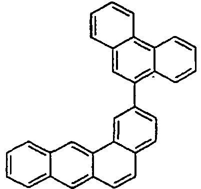 | |
| (153) | (154) | |
| 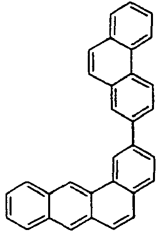 | 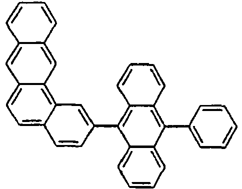 | |
| (155) | (156) | |
| 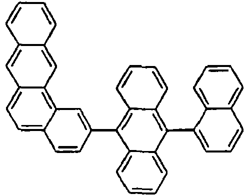 | 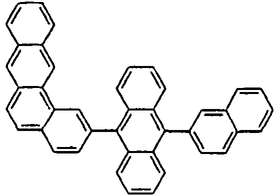 | |
| (157) | (158) | |
| 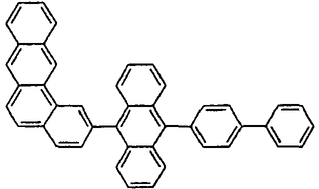 | 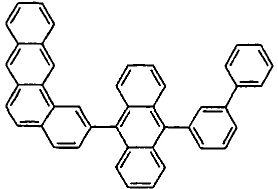 | |
| (159) | (160) | |
| 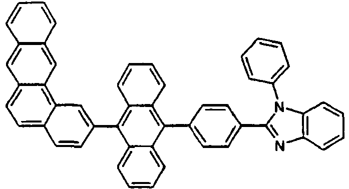 | 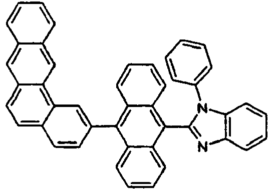 | |
| (161) | (162) | |
| 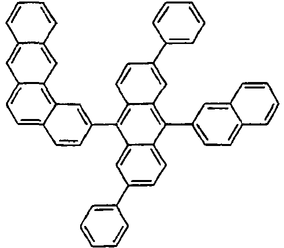 | 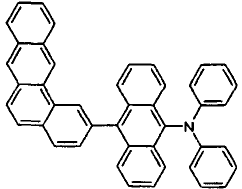 | |
| (163) | (164) | |
| 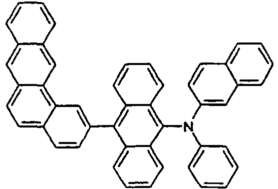 | 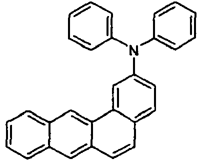 | |
| (165) | (166) | |
| 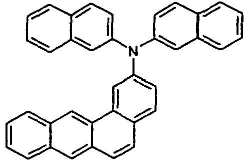 | 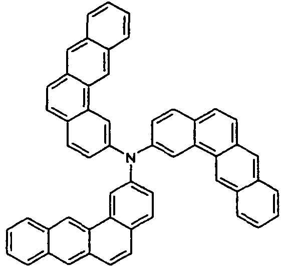 | |
| (167) | (168) | |
| 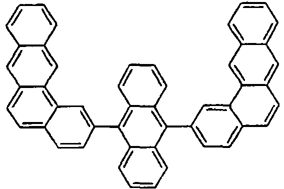 | 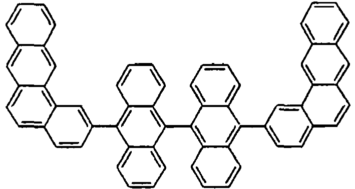 | |
| (169) | (170) | |
| 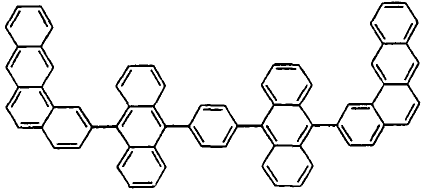 | 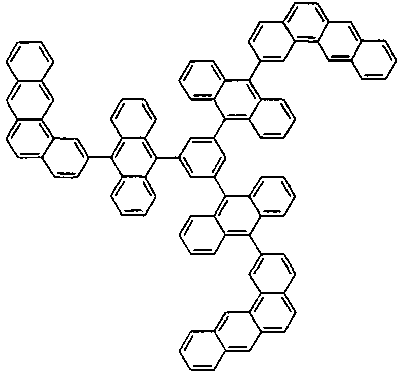 | |
| (171) | (172) | |
| 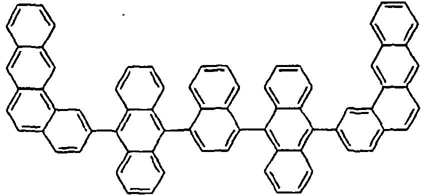 | 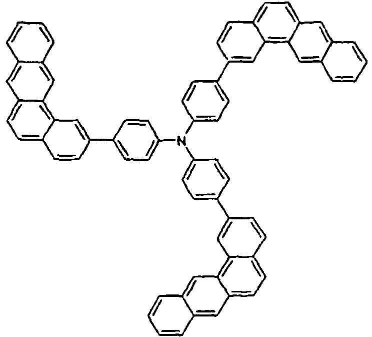 | |
| (173) | (174) | |
| 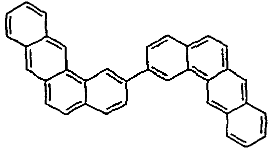 | 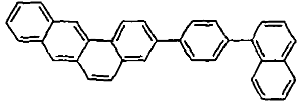 | |
| (175) | (176) | |
| 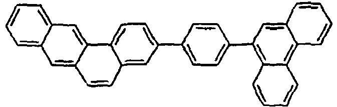 | 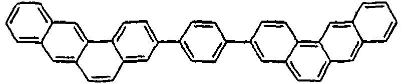 | |
| (177) | (178) | |
| 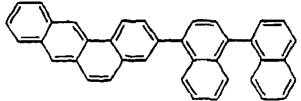 | 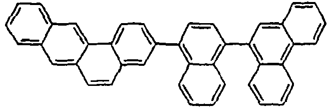 | |
| (179) | (180) | |
| 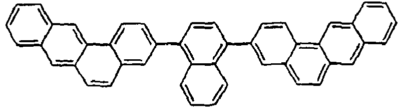 | 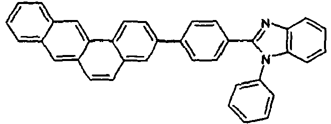 | |
| (181) | (182) | |
| 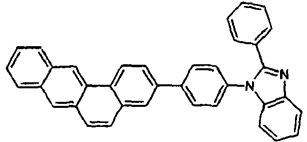 | 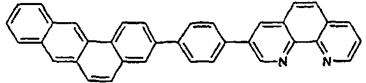 | |
| (183) | (184) | |
| 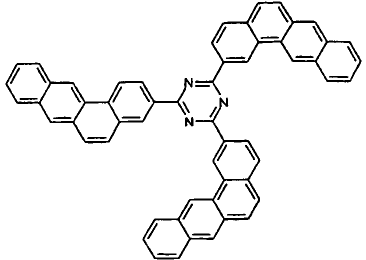 | 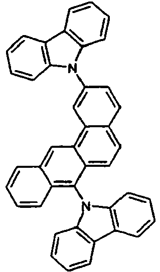 | |
| (185) | (186) | |
| 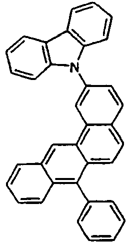 | 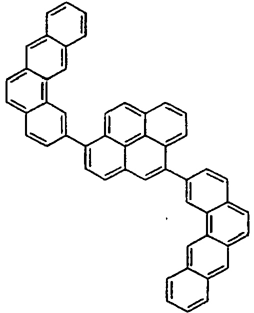 | |
| (187) | (188) | |
| 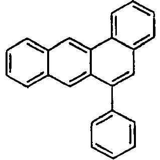 | 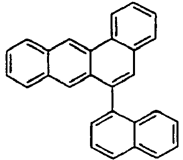 | |
| (189) | (190) | |
| 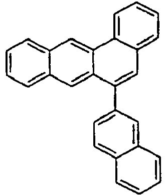 | 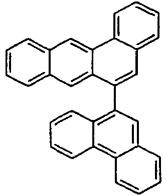 | |
| (191) | (192) | |
| 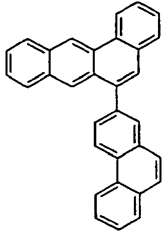 | 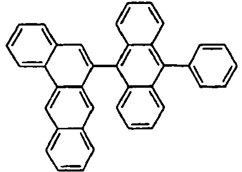 | |
| (193) | (194) | |
| 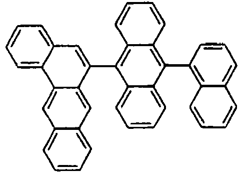 | 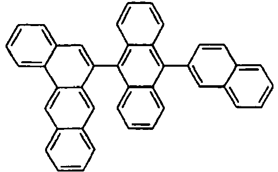 | |
| (195) | (196) | |
| 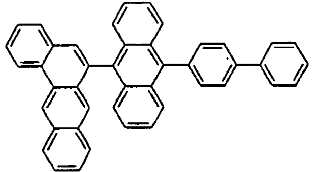 | 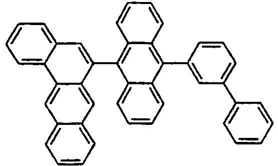 | |
| (197) | (198) | |
| 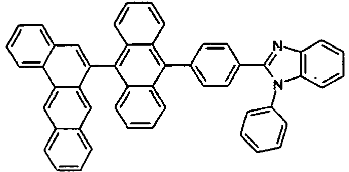 | 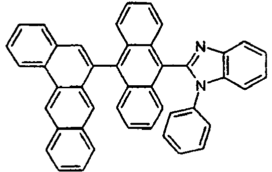 | |
| (199) | (200) | |
| 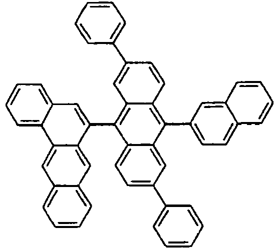 | 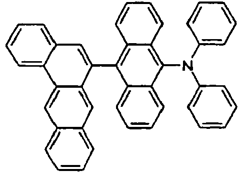 | |
| (201) | (202) | |
| 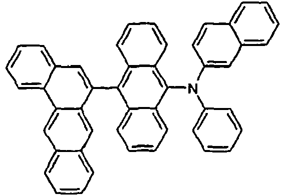 | 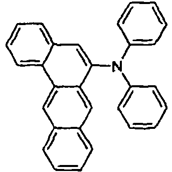 | |
| (203) | (204) | |
| 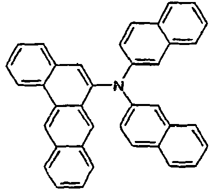 | 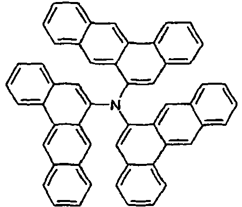 | |
| (205) | (206) | |
| 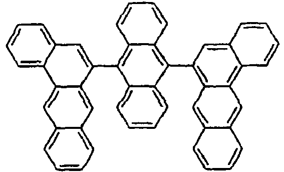 | 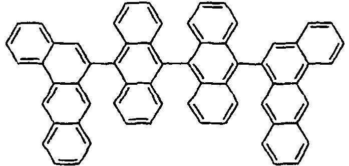 | |
| (207) | (208) | |
| 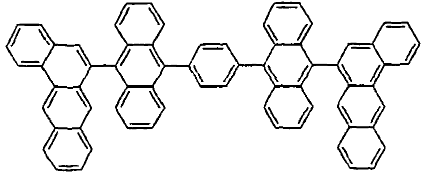 | 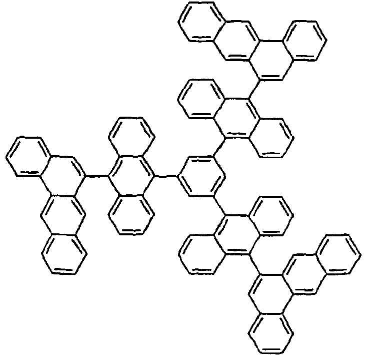 | |
| (209) | (210) | |
| 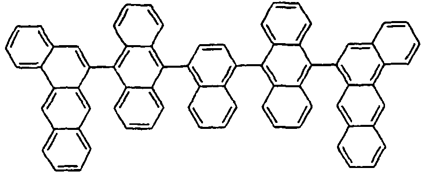 | 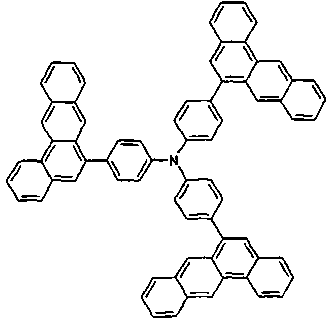 | |
| (211) | (212) | |
| 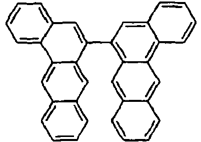 | 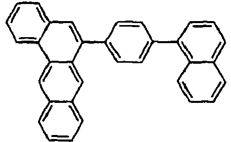 | |
| (213) | (214) | |
| 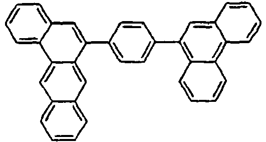 | 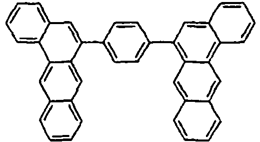 | |
| (215) | (216) | |
| 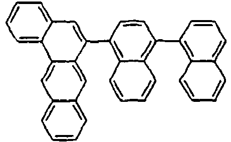 | 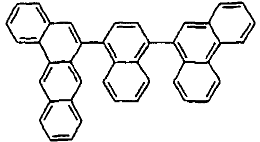 | |
| (217) | (218) | |
| 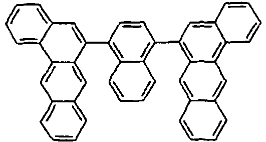 | 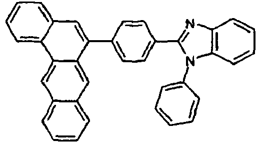 | |
| (219) | (220) | |
| 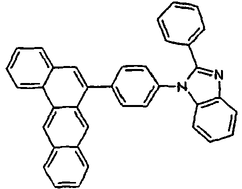 | 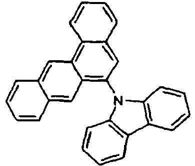 | |
| (221) | (222) | |
| 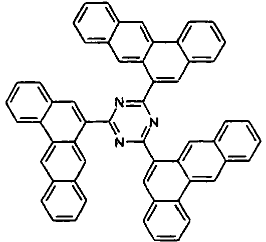 | 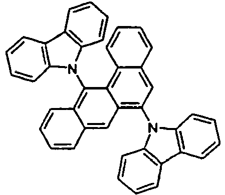 | |
| (223) | (224) | |
| 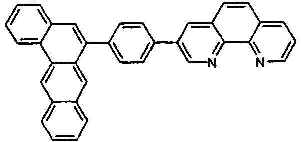 | 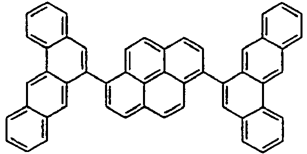 | |
| (225) | (226) | |
| 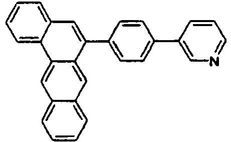 | 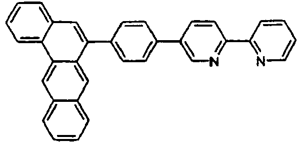 | |
| (227) | (228) | |
| 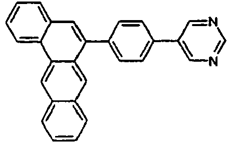 | 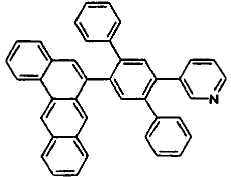 | |
| (229) | (230) | |
| 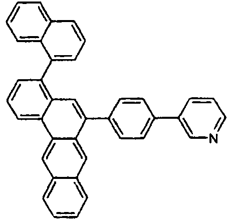 | 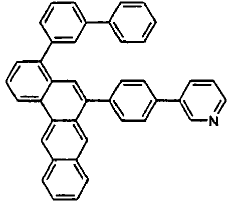 | |
| (231) | (232) | |
| 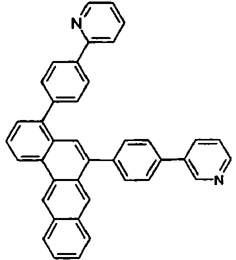 | 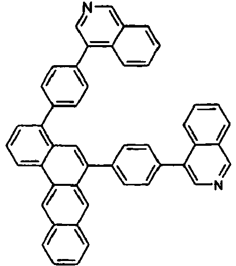 | |
| (233) | (234) | |
| 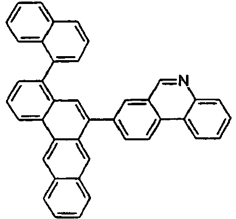 | 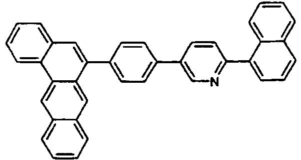 | |
| (235) | (236) | |
| 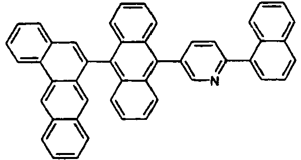 | 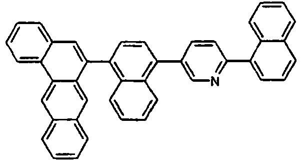 | |
| (237) | (238) | |
| 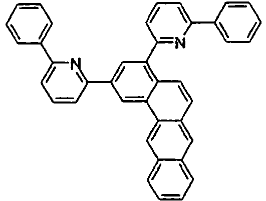 | 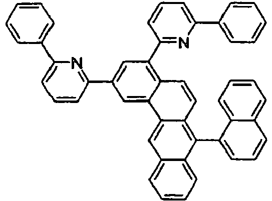 | |
| (239) | (240) | |
| 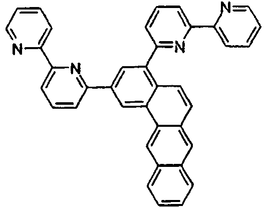 | 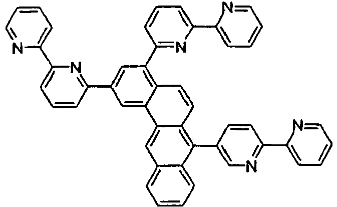 | |
| (241) | (242) | |
| 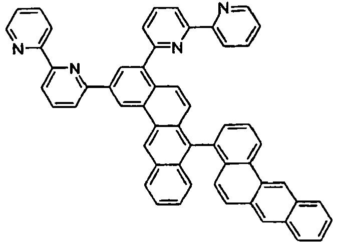 | 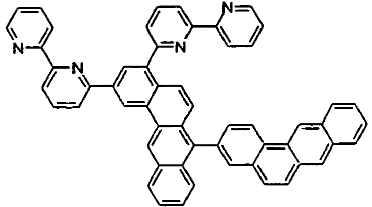 | |
| (243) | (244) | |
| 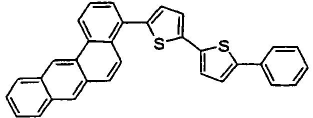 | 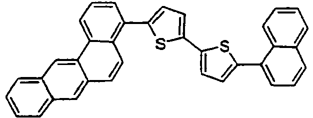 | |
| (245) | (246) | |
| 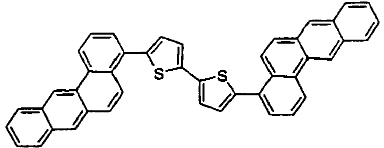 | 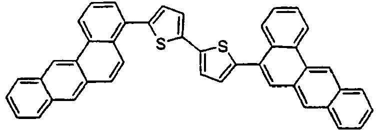 | |
| (247) | (248) | |
| 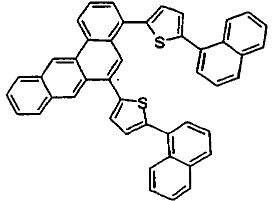 | 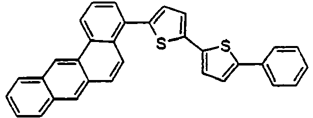 | |
| (249) | (250) | |
| 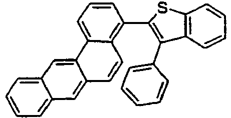 | 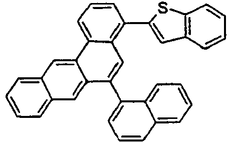 | |
| (251) | (252) | |
| 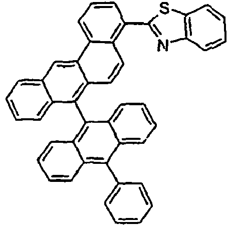 | 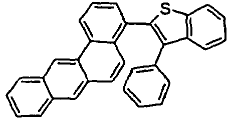 | |
| (253) | (254) | |
| 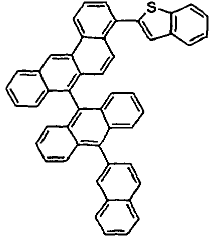 | 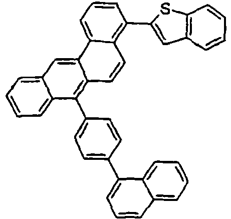 | |
| (255) | (256) | |
| 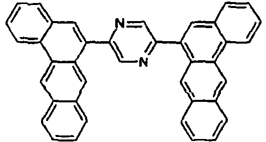 | 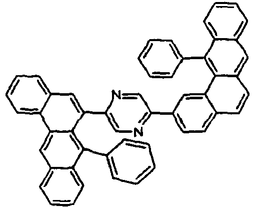 | |
| (257) | (258) | |
| 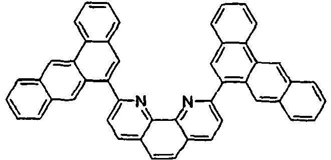 | 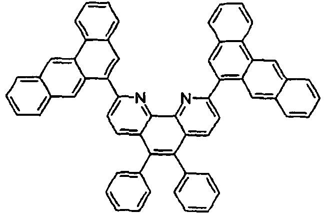 | |
| (259) | (260) | |
| 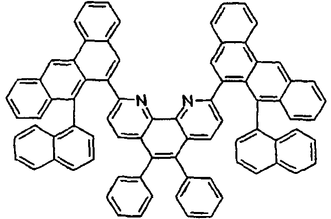 | 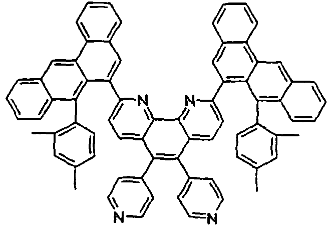 | |
| (261) | (262) | |
| 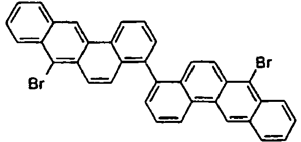 | 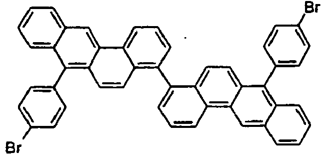 | |
| (263) | (264) | |
| 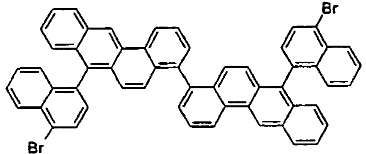 | 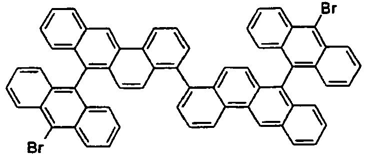 | |
| (265) | (266) | |
| 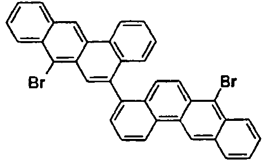 | 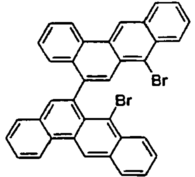 | |
| (267) | (268) | |
| 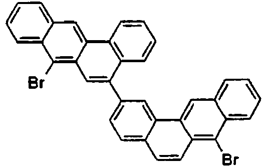 | 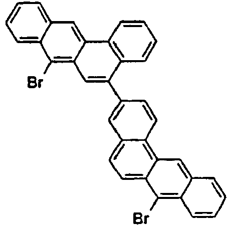 | |
| (269) | (270) | |
| 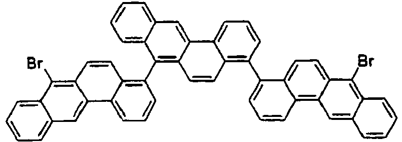 | 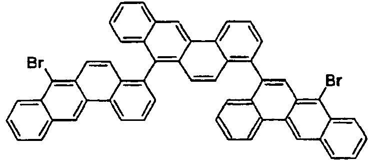 | |
| (271) | (272) | |
| 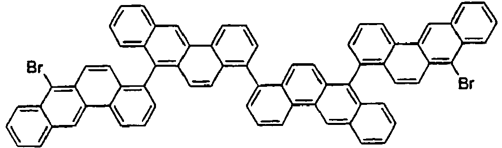 | | |
| (273) | | |
| 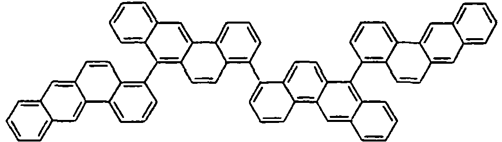 | | |
| (274) | | |
| 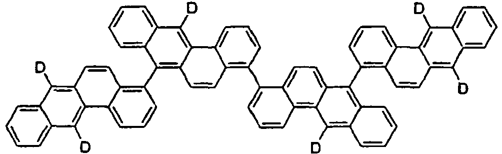 | | |
| (275) | | |
| 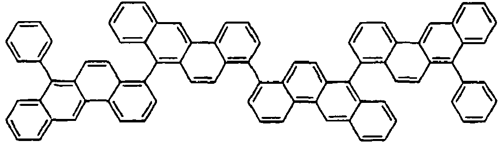 | | |
| (276) | | |
| 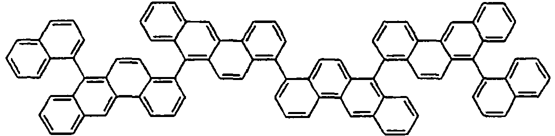 | | |
| (277) | | |
| 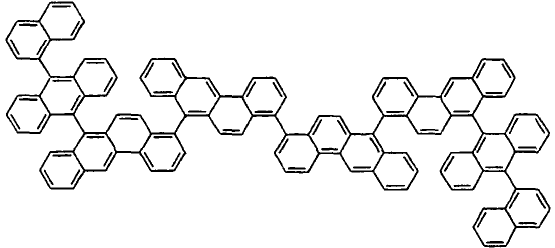 | | |
| (278) | | |
| 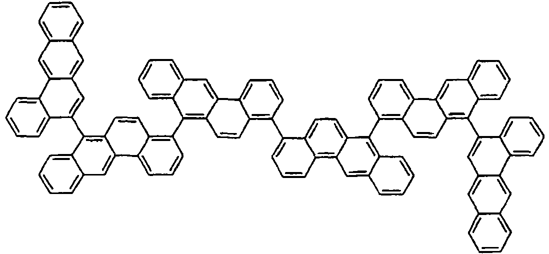 | | |
| (279) | | |
| 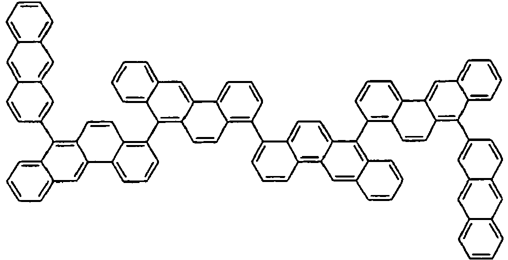 | | |
| (280) | | |
| 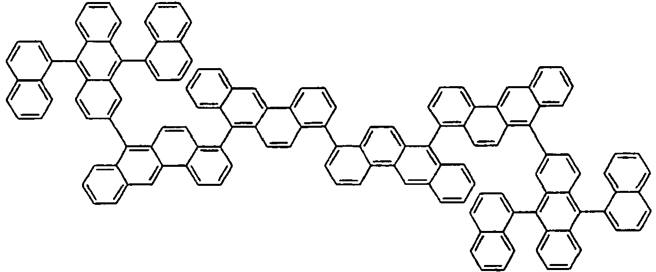 | | |
| (281) | | |
| 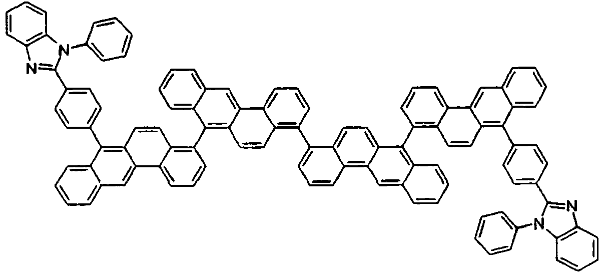 | | |
| (282) | | |
| 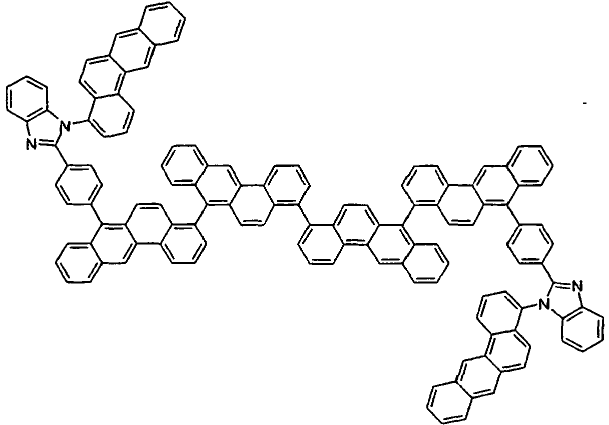 | | |
| (283) | | |
| 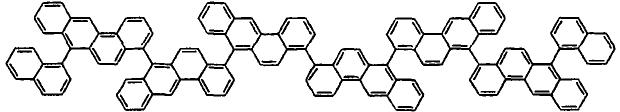 | | |
| (284) | | |
| 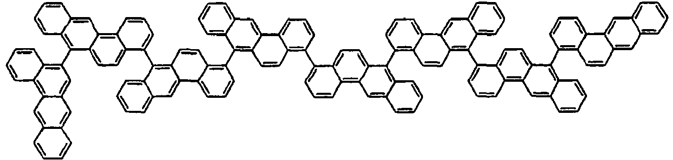 | | |
| (285) | | |
| 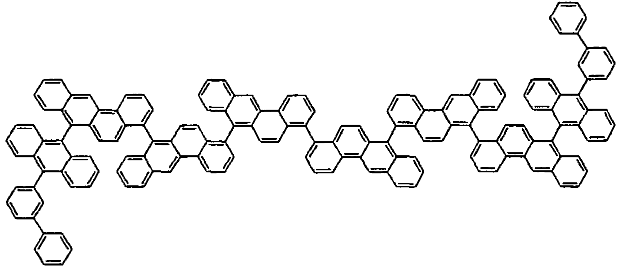 | | |
| (286) | | |
| 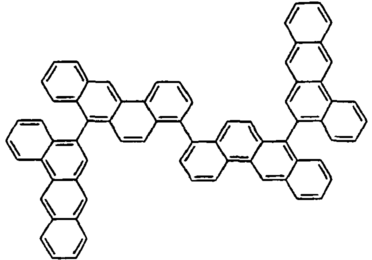 | | |
| (287) | | |
| 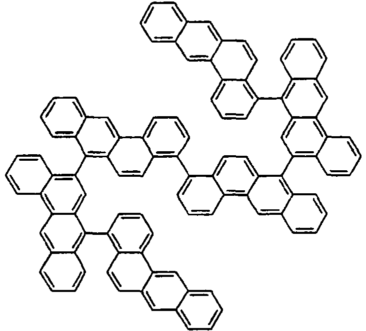 | | |
| (288) | | |
| 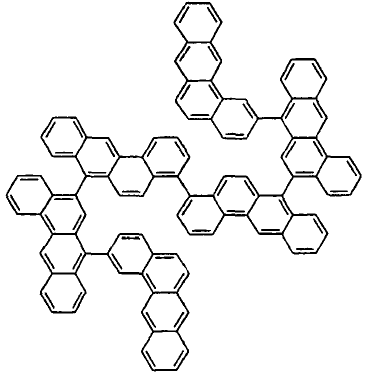 | | |
| (289) | | |
| 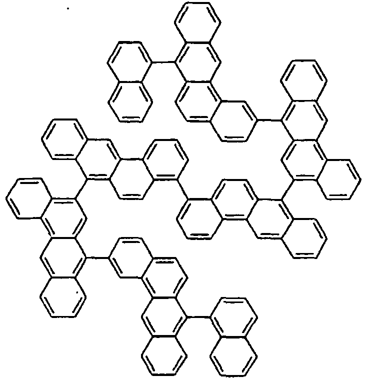 | | |
| (290) | | |
| 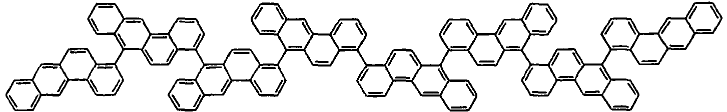 | | |
| (291) | | |
| 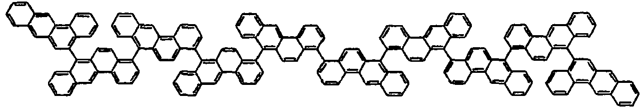 | | |
| (292) | | |
| 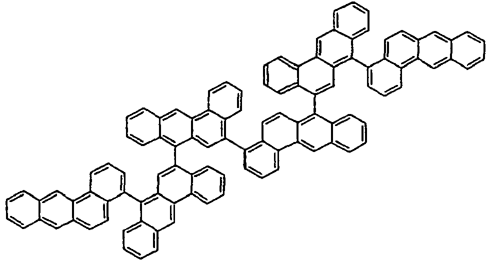 | | |
| (293) | | |
| 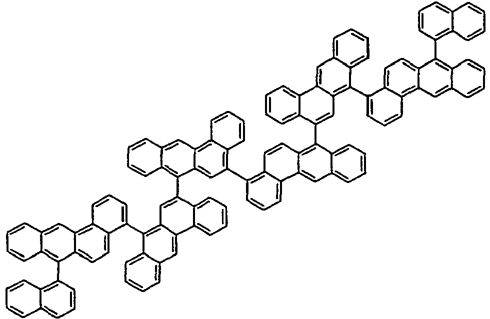 | | |
| (294) | | |
| 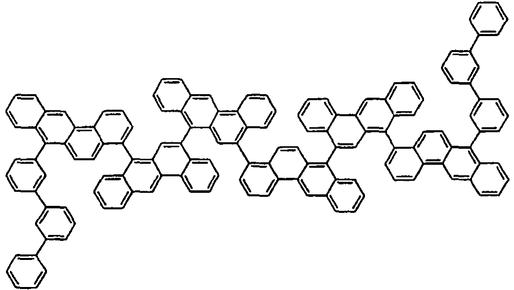 | | |
| (295) | | |
| 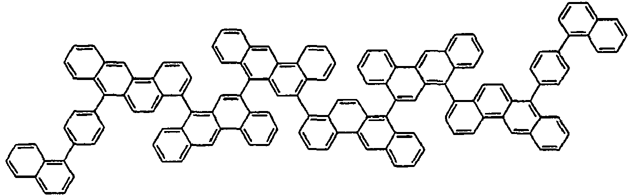 | | |
| (296) | | |
| 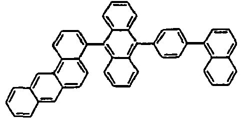 | 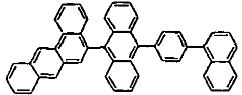 | 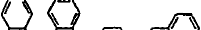 |
| (297) | (298) | (299) |
| 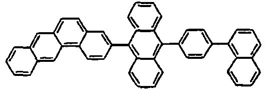 | 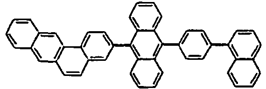 | 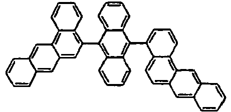 |
| (300) | (301) | (302) |
| 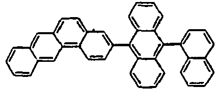 | 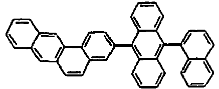 | 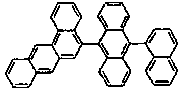 |
| (303) | (304) | (305) |
| 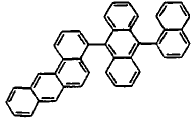 | 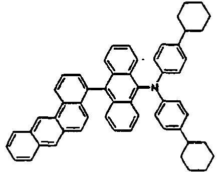 | 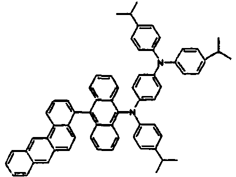 |
| (306) | (307) | (308) |
| 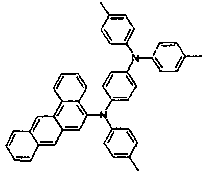 | 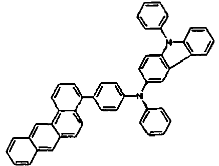 | 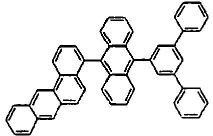 |
| (309) | (310) | (311) |
| 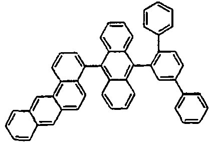 | 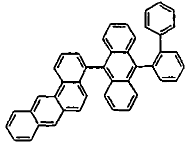 | 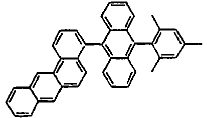 |
| (312) | (313) | (314) |
| 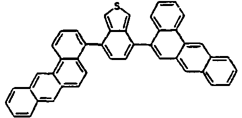 | 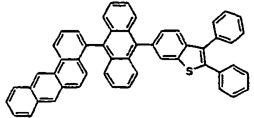 | 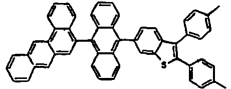 |
| (315) | (316) | (317) |
| 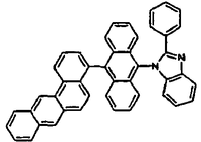 | 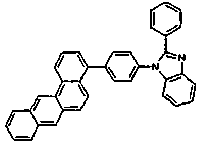 | 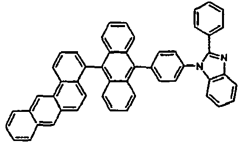 |
| (318) | (319) | (320) |
| 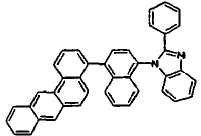 | 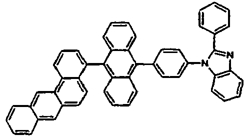 | 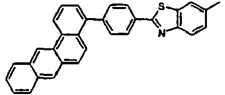 |
| (321) | (322) | (323) |
| 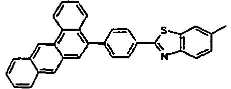 | 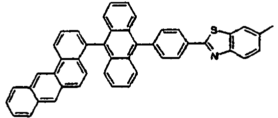 | 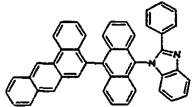 |
| (324) | (325) | (326) |
| 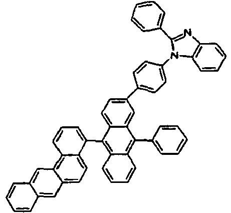 | 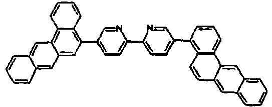 | 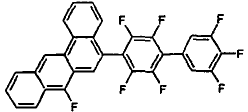 |
| (327) | (328) | (329) |
| 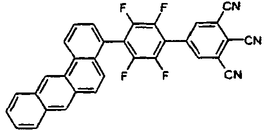 | 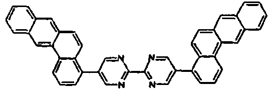 | 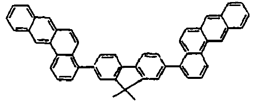 |
| (330) | (331) | (332) |
| 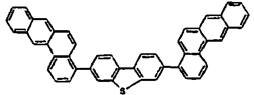 | 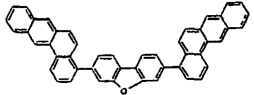 | 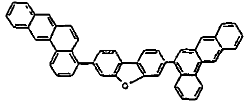 |
| (333) | (334) | (335) |
| 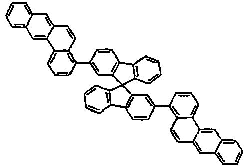 | 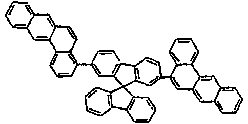 | 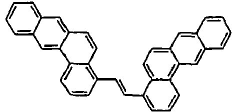 |
| (336) | (337) | (338) |

Die erfindungsgemäßen Verbindungen gemäß Formel (1) können nach dem Fachmann allgemein bekannten Syntheseschritten dargestellt werden. Als Ausgangsverbindung können z. B. die entsprechenden Brombenz[a]anthracene dienen, deren Synthese bekannt ist (2- und 3-Brombenz[a]anthracen: Hallmark et al., J. Lab. Comp. Radiopharm. 1981, 18(3), 331; 4-Brombenz[a]anthracen: Badgar et al., J. Chem. Soc. 1949, 799; 5-Brombenz[a]anthracen: Newman et al., J. Org. Chem. 1982, 47(15), 2837). Ebenso können die mit entsprechenden Abgangsgruppen wie Chlor, Iod, Triflat oder Tosylat substituierten Benz[a]anthracene als Ausgangsverbindungen dienen. Substituiertes oder unsubstituiertes 5-Brombenz[a]anthracen kann alternativ auch aus 2-Brombenzaldehyd und 1-Chlormethylnaphthalin gemäß Schema 1 erhalten werden. Dabei steht R in Schema 1 für einen oder mehrere Reste, wie für Formel (1) definiert. Statt Lithiierung kann im ersten Schritt auch die Umsetzung mit einem anderen reaktiven Metall, beispielsweise Magnesium, erfolgen. Die Suzuki-Kupplung im ersten Schritt findet unter Standardbedingungen statt, wie sie dem Fachmann der organischen Chemie bekannt sind, beispielsweise mit Pd(PPh₃)₄ in Toluol/Wasser unter Zusatz einer Base bei erhöhter Temperatur. Die Bromierung im zweiten Schritt kann beispielsweise mit elementarem Brom oder mit NBS erfolgen. Der Ringschluss im dritten Schritt kann beispielsweise durch Einwirkung von Polyphosphorsäure erfolgen.

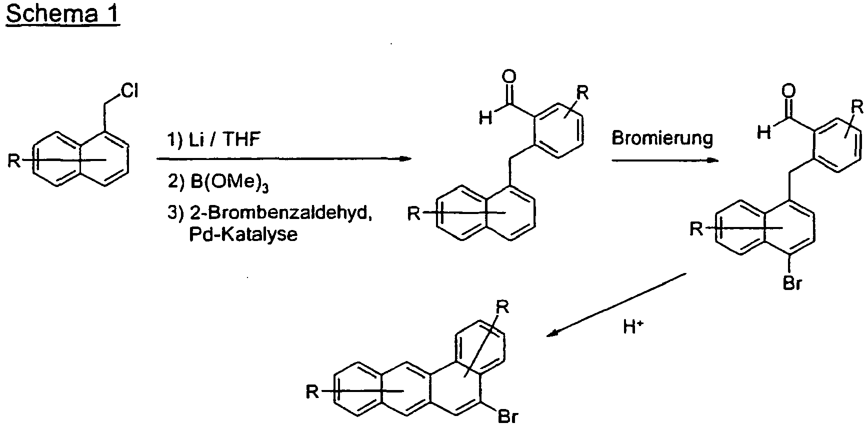

Ein weiterer Zugang zu substituiertem Benz[a]anthracen besteht in der Kupplung von Naphthalin-2-boronsäure mit 2-Bromphenylacetylen. Das so erhaltene Acetylen kann entweder direkt in einer Ringschlussreaktion umgesetzt werden, oder es kann nach Halogenierung cyclisiert werden, oder es kann in einer Sonogashira-Kupplung mit einem Aromaten umgesetzt und im Anschluss daran cyclisiert werden. Dabei erfolgt der Ringschluss des Acetylens jeweils unter Einsatz eines Elektrophils. Die Verbindungen in Schema 2 können auch durch einen oder mehrere Reste R substituiert sein, wobei R dieselbe Bedeutung hat, wie oben unter Formel (1) beschrieben. Ar bedeutet ein aromatisches oder heteroaromatisches Ringsystem. Die Suzuki-Kupplungen und die Sonogashira-Kupplung werden unter Standardbedingungen, wie sie dem Fachmann der organischen Synthese bekannt sind, durchgeführt. Bevorzugte Elektrophile für die Ringschlussreaktion sind starke Säuren wie CF₃COOH, Indiumhalogenide wie InCl₃ oder InBr₃, Platinhalogenide wie PtCl₂ oder Interhalogenverbindungen wie I-Cl.

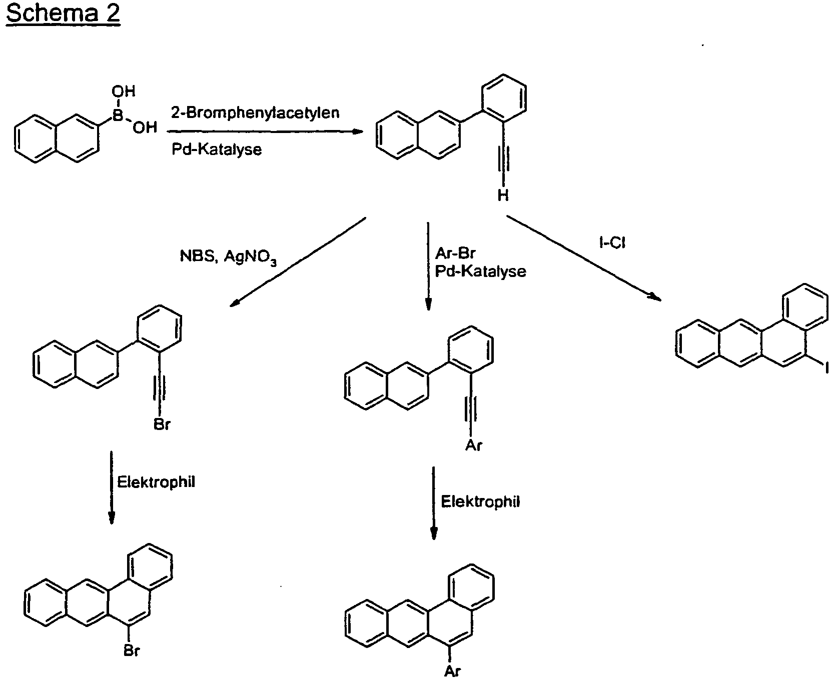

Ein weiterer Gegenstand der vorliegenden Erfindung ist ein Verfahren zur Herstellung von gegebenenfalls substituiertem 6-Arylbenz[a]anthracen durch Reaktion eines gegebenenfalls substituierten 2-(2'-Arylacetylen)-phenyl-naphthalin mit einem Elektrophil. Die gegebenenfalls vorhanden Substituenten sind analog dem oben definierten Rest R definiert.

Die von diesen Verbindungen abgeleiteten Boronsäuren bzw. Boronsäurederivate können durch Transmetallierung, beispielsweise mit n-Butyllithium in THF bei -78 °C, und anschließender Umsetzung des intermediär gebildeten Lithio-benz[a]anthracens mit Borsäuretrimethylester erhalten werden, wie in Schema 3 exemplarisch am Beispiel von 4-Brombenz[a]-anthracen gezeigt, gegebenenfalls gefolgt von einer Veresterung. Weiterhin können die lithiierten Verbindungen durch Reaktion mit Elektrophilen wie Benzonitril und anschließender saurer Hydrolyse zu Ketonen oder mit Chlordiarylphosphinen und anschließender Oxidation zu Phosphinoxiden umgesetzt werden. Auch die Reaktion der lithiierten Verbindung mit anderen Elektrophilen ist möglich.

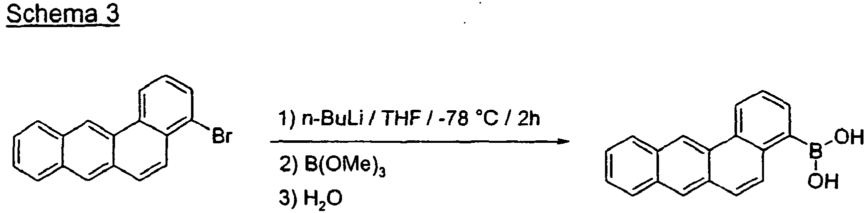

Die Verbindungen in Schema 3 können auch durch einen oder mehrere Reste R substituiert sein, wobei R dieselbe Bedeutung hat, wie oben unter Formel (1) beschrieben. Die Suzuki-Kupplung der Boronsäuren bzw. Boronsäurederivate mit Arylbromiden führt zu einer großen Klasse verschiedener aromatischer und heteroaromatischer Verbindungen. Dies ist exemplarisch in Schema 4 a) bis e), ausgehend von Benz[a]anthracen-4-boronsäure, gezeigt, gilt aber in gleicher Weise auch für die anderen Substitutionsmuster. Weiterhin können alle Strukturen auch durch einen oder mehrere Reste R substituiert sein, wobei R dieselbe Bedeutung hat, wie oben unter Formel (1) beschrieben.

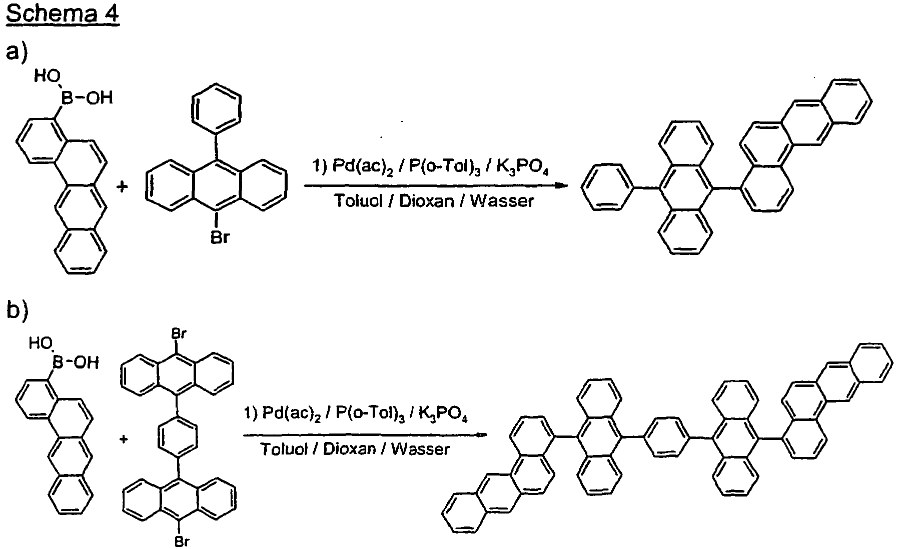

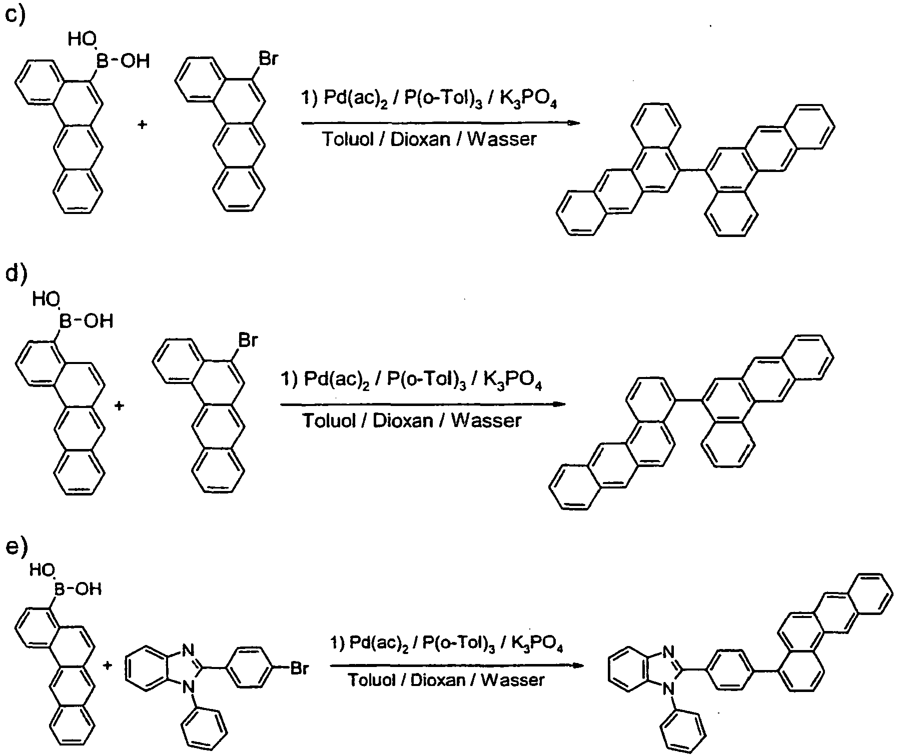

Alternativ können zunächst die Brombenz[a]anthrachinone (Synthese gemäß Newman et al., J. Org. Chem. 1983, 48, 2926-8; Cho et al., J. Org. Chem. 1987, 52, 2668-78; Becker et al. J. Phys. Chem. 1993, 97, 344-9) gekuppelt und dann zu den entsprechenden Kohlenwasserstoffen reduziert werden, wie exemplarisch am 5-Brombenz[a]anthrachinon in Schema 5 gezeigt. Statt einfacher Aromatisierung können hier auch durch Addition eines metallorganischen Reagenzes, beispielsweise einer Organolithiumverbindung oder einer Grignardverbindung, gefolgt von Aromatisierung die entsprechenden 7,12-substituierten Benz[a]anthracenderivate synthetisiert werden.

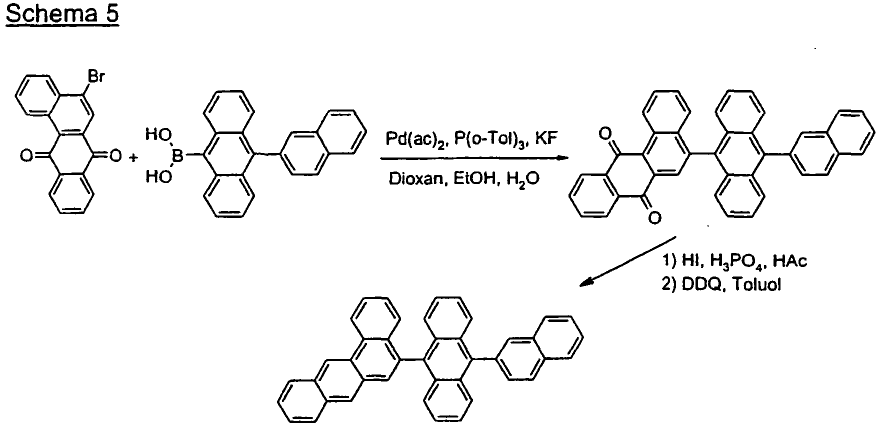

Die Verbindungen in Schema 5 können auch durch einen oder mehrere Reste R substituiert sein, wobei R dieselbe Bedeutung hat, wie oben unter Formel (1) beschrieben.

Die Palladium-vermittelte Aminierung der Bromide nach Hartwig-Buchwald führt zu dem entsprechenden aminierten Benz[a]anthracenen (Schema 6). Die Aminierung an den anderen Positionen des Benz[a]anthracens ist entsprechend zugänglich. Eine entsprechende Reaktion ist mit anderen Abgangsgruppen, wie Chlor, Iod, Triflat, Tosylat, etc. möglich.

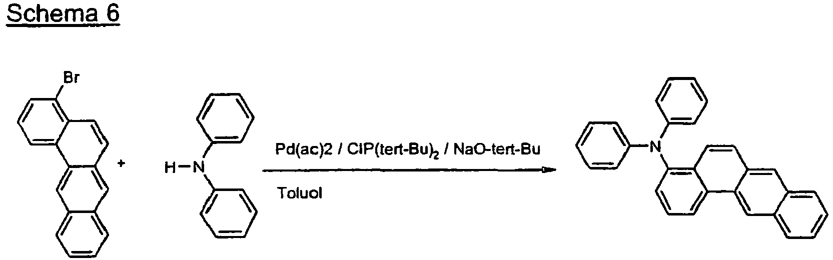

Die Verbindungen in Schema 6 können auch durch einen oder mehrere Reste R substituiert sein, wobei R dieselbe Bedeutung hat, wie oben unter Formel (1) beschrieben.

Nochmals ein weiterer Gegenstand der Erfindung ist ein Verfahren zur Herstellung von Verbindungen gemäß Formel (1) durch Kupplung eines Benz[a]anthracens, welches durch eine reaktive Abgangsgruppe, insbesondere Chlor, Brom, Iod, Triflat, Tosylat, Boronsäure oder Boronsäureester, substituiert ist, mit einem funktionalisierten Aromaten oder mit einem mono- oder disubstituierten Amin. Die reaktive Abgangsgruppe ist bevorzugt Brom. Als Kupplungsreaktion zwischen dem Grundgerüst gemäß Formel (1) und dem Arylsubstituenten eignen sich vor allem übergangsmetallkatalysierte Kupplungsreaktionen, insbesondere Suzuki-Kupplung unter Palladium-Katalyse, so dass sich hier insbesondere die Kupplung eines Boronsäurederivats mit einem Halogenderivat anbietet. Als Kupplungsreaktion mit einem mono- oder disubstituierten Amin eignet sich insbesondere die palladiumkatalysierte Kupplung nach Hartwig-Buchwald. Die Reaktionsbedingungen für derartige Reaktionen sind dem Fachmann der organischen Synthese generell bekannt.

Weiterhin können die Boronsäuren zu Boronsäureestern durch Umsetzung mit Diolen, Oligoolen und Polyolen bzw. zu Anhydriden durch kochen in Toluol am Wasserabscheider umgesetzt werden (Schema 7), wobei die Reaktion für die Positionen 2, 3, 5 und 6 am Benz[a]anthracen entsprechend abläuft.

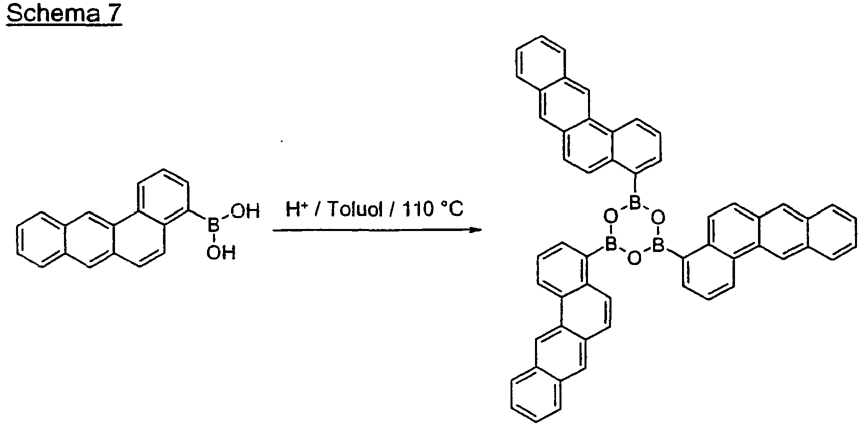

Die Verbindungen in Schema 7 können auch durch einen oder mehrere Reste R substituiert sein, wobei R dieselbe Bedeutung hat, wie oben unter Formel (1) beschrieben.

Ein weiterer Gegenstand der Erfindung sind Verbindungen der folgenden Formel (34), welche eine wesentliche Zwischenstufe für die Synthese von Verbindungen gemäß Formel (1) darstellen, 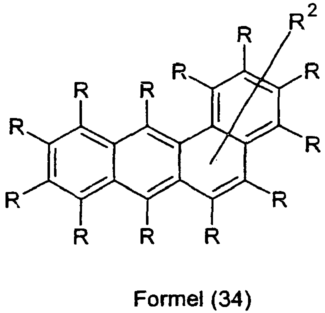 wobei R, R¹ und Ar¹ dieselbe Bedeutung haben, wie oben für Verbindungen der Formel (1) beschrieben, und R² in der Position 2, 3, 4, 5 oder 6 des Benz[a]anthracens gebunden ist und entsprechend an dieser Position keine Gruppe R gebunden ist, und weiterhin gilt:
- R²: steht für B(OR¹)2 oder B(OAr¹)₂.

Es ist weiterhin auch möglich, die Boronsäurederivate der Formel (20) direkt als aktive Verbindung in organischen elektronischen Vorrichtungen einzusetzen, wie in WO 06/117052 allgemein beschrieben.

Die oben beschriebenen erfindungsgemäßen Verbindungen, insbesondere Verbindungen, welche mit reaktiven Abgangsgruppen, wie Brom, Iod, Boronsäure oder Boronsäureester, substituiert sind, können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität. Weiterer Gegenstand der Erfindung sind daher Oligomere, Polymere oder Dendrimere enthaltend eine oder mehrere Verbindungen gemäß Formel (1), wobei ein oder mehrere Reste R bzw. Ar bzw. Y Bindungen der Verbindung gemäß Formel (1) zum Polymer, Oligomer oder Dendrimer darstellen. Je nach Verknüpfung der Verbindung gemäß Formel (1) bildet die Benz[a]anthraceneinheit daher eine Seitenkette des Oligomers oder Polymers oder ist in der Hauptkette verknüpft. Unter einem Oligomer im Sinne dieser Erfindung wird eine Verbindung verstanden, welche mindestens drei Benz[a]anthraceneinheiten aufweist. Die Polymere, Oligomere oder Dendrimere können konjugiert, teilkonjugiert oder nichtkonjugiert sein. Die Oligomere oder Polymere können linear, verzweigt oder dendritisch sein. In den linear verknüpften Strukturen können die Einheiten gemäß Formel (1) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe, miteinander verknüpft sein. In verzweigten und dendritischen Strukturen können beispielsweise drei oder mehrere Einheiten gemäß Formel (1) über eine trivalente oder höhervalente Gruppe, beispielsweise über eine trivalente oder höhervatente aromatische oder heteroaromatische Gruppe, zu einem verzweigten bzw. dendritischen Oligomer oder Polymer verknüpft sein. Eine bevorzugte Verknüpfung der Einheiten gemäß Formel (1) in das Oligomer, Dendrimer oder Polymer erfolgt über die Positionen 7 und 12 des Benz[a]anthracens. Eine weitere bevorzugte Verknüpfung erfolgt über zwei Positionen an der Gruppe R bzw. an der Gruppe Y.

Für die Wiederholeinheiten gemäß Formel (1) in Oligomeren, Dendrimeren und Polymeren gelten dieselben Bevorzugungen wie oben beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 00/22026), Spirobifluorenen (z. B. gemäß EP 707020, EP 894107 oder WO 06/061181), Paraphenylenen (z. B. gemäß WO 92/18552), Carbazolen (z. B. gemäß WO 04/070772 oder WO 04/113468), Thiophenen (z. B. gemäß EP 1028136), Dihydrophenanthrenen (z. B. gemäß WO 05/014689 oder WO 07/006383), cis- und trans-Indenofluorenen (z. B. gemäß WO 04/041901 oder WO 04/113412), Ketonen (z. B. gemäß WO 05/040302), Phenanthrenen (z. B. gemäß WO 05/104264 oder WO 07/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 07/068325) oder phosphoreszierende Metallkomplexe (z. B. gemäß WO 06/003000), und/oder Ladungstransporteinheiten, insbesondere solche basierend auf Triarylaminen.

Die Verbindungen gemäß Formel (1) und entsprechende Oligomere, Dendrimere und Polymere eignen sich für den Einsatz in elektronischen Vorrichtungen, insbesondere in organischen Elektrolumineszenzvorrichtungen (OLEDs, PLEDs). Abhängig von der Substitution werden die Verbindungen in unterschiedlichen Funktionen und Schichten eingesetzt.

Ein weiterer Gegenstand der Erfindung ist daher die Verwendung von Verbindungen gemäß Formel (1) und entsprechender Oligomere, Dendrimere oder Polymere in elektronischen Vorrichtungen, insbesondere in organischen Elektrolumineszenzvorrichtungen. Besonders eignen sich hierfür die oben aufgeführten bevorzugten Verbindungen der Formeln (2) bis (24).

Nochmals ein weiterer Gegenstand der Erfindung sind organische elektronische Vorrichtungen, enthaltend mindestens eine Verbindung gemäß Formel (1) bzw. mindestens ein entsprechendes Oligomer, Dendrimer oder Polymer, insbesondere organische Elektrolumineszenzvorrichtungen, enthaltend Anode, Kathode und mindestens eine emittierende Schicht, dadurch gekennzeichnet, dass mindestens eine organische Schicht, die eine emittierende Schicht oder eine andere Schicht sein kann, mindestens eine Verbindung gemäß Formel (1) bzw. mindestens ein entsprechendes Oligomer, Dendrimer oder Polymer enthält. Besonders eignen sich hierfür die oben aufgeführten bevorzugten Verbindungen der Formeln (2) bis (24).

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Elektronentransportschichten, Elektroneninjektionsschichten und/oder Ladungserzeugungsschichten (Charge-Generation Layers; IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer). Es sei aber darauf hingewiesen, dass nicht notwendigerweise jede dieser Schichten vorhanden sein muss.

Dem Fachmann der organischen Elektrolumineszenz ist bekannt, welche Materialien er für diese weiteren Schichten einsetzen kann. Generell eignen sich für die weiteren Schichten alle Materialien, wie sie gemäß dem Stand der Technik verwendet werden und der Fachmann kann diese Materialien ohne Ausübung von erfinderischer Tätigkeit mit den erfindungsgemäßen Materialien in einer organischen Elektrolumineszenzvorrichtung kombinieren. Beispiele für bevorzugte Lochtransportmaterialien, die in einer Lochtransport- oder Lochinjektionsschicht in der erfindungsgemäßen Elektrolumineszenzvorrichtung verwendet werden können, sind Indenofluorenamine und verwandte Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449) oder Dibenzoindenofluorenamine (z. B. gemäß WO 07/140847).

In einer weiteren bevorzugten Ausführungsform der Erfindung enthält die organische Elektrolumineszenzvorrichtung mehrere emittierende Schichten, wobei mindestens eine organische Schicht mindestens eine Verbindung gemäß Formel (1) enthält. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues und gelbes, orange oder rotes Licht emittieren. Dabei wird die Verbindung gemäß der Formel (1) bevorzugt in einer blau emittierenden Schicht verwendet. Insbesondere bevorzugt sind Dreischichtsysteme, also Systeme mit drei emittierenden Schichten, wobei mindestens eine dieser Schichten mindestens eine Verbindung gemäß Formel (1) enthält und wobei die drei Schichten blaue, grüne und orange oder rote Emission zeigen (für den prinzipiellen Aufbau siehe z. B. WO 05/011013). Ebenso eignen sich für weiße Emission Emitter, welche breitbandige Emission aufweisen und dadurch weiße Emission zeigen.

In einer Ausführungsform der Erfindung werden die Verbindungen gemäß Formel (1) bis (24) als Hostmaterial für fluoreszierende Dotanden, insbesondere für blau fluoreszierende Dotanden, eingesetzt. In diesem Fall sind bevorzugt ein oder mehrere Gruppen Ar und/oder Y in den Formeln (1) bzw. (3) bis (7) aus einfachen oder kondensierten Aryl- oder Heteroarylgruppen gewählt, insbesondere Phenylanthryl oder 1- oder 2-Naphthylanthryl. Weiterhin bevorzugt sind in den Formeln (1) bzw. (8) bis (24) ein oder mehrere Gruppen Ar und/oder Y aus kondensierten Arylengruppen gewählt, insbesondere 9,10-Anthracen.

Unter einem Hostmaterial wird in einem System aus Host und Dotand diejenige Komponente verstanden, die in dem System im höheren Anteil vorliegt. Bei einem System aus einem Host und mehreren Dotanden wird als Host diejenige Komponente verstanden, deren Anteil der höchste in der Mischung ist.

Der Anteil des Hostmaterials gemäß Formel (1) in der emittierenden Schicht beträgt zwischen 50.0 und 99.9 Gew.-%, bevorzugt zwischen 80.0 und 99.5 Gew.-%, besonders bevorzugt zwischen 90.0 und 99.0 Gew.-%. Entsprechend beträgt der Anteil des Dotanden zwischen 0.01 und 50.0 Gew.-%, bevorzugt zwischen 0.1 und 20.0 Gew.-%, besonders bevorzugt zwischen 0.5 und 15 Gew.-%, ganz besonders bevorzugt zwischen 1.0 und 10.0 Gew.-%.

Bevorzugte Dotanden sind ausgewählt aus der Klasse der Monostyrylamine, der Distyrylamine, der Tristyrylamine, der Tetrastyrylamine, der Styrylphosphine, der Styrylether und der Arylamine. Unter einem Monostyrylamin wird eine Verbindung verstanden, die eine substituierte oder unsubstituierte Styrylgruppe und mindestens ein, bevorzugt aromatisches, Amin enthält. Unter einem Distyrylamin wird eine Verbindung verstanden, die zwei substituierte oder unsubstituierte Styrylgruppen und mindestens ein, bevorzugt aromatisches, Amin enthält. Unter einem Tristyrylamin wird eine Verbindung verstanden, die drei substituierte oder unsubstituierte Styrylgruppen und mindestens ein, bevorzugt aromatisches, Amin enthält. Unter einem Tetrastyrylamin wird eine Verbindung verstanden, die vier substituierte oder unsubstituierte Styrylgruppen und mindestens ein, bevorzugt aromatisches, Amin enthält. Die Styrylgruppen sind besonders bevorzugt Stilbene, die auch noch weiter substituiert sein können. Entsprechende Phosphine und Ether sind in Analogie zu den Aminen definiert. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Dotanden sind gewählt aus Indenofluorenaminen bzw. -diaminen, beispielsweise gemäß WO 06/122630, Benzoindenofluorenaminen bzw. -diaminen, beispielsweise gemäß WO 08/006449, und Dibenzoindenofluorenaminen bzw. -diaminen, beispielsweise gemäß WO 07/140847. Beispiele für Dotanden aus der Klasse der Styrylamine sind substituierte oder unsubstituierte Tristilbenamine oder die Dotanden, die in WO 06/000388, WO 06/058737, WO 06/000389, WO 07/065549 und WO 07/115610 beschrieben sind. Nochmals weiterhin bevorzugt sind die unten beschriebenen erfindungsgemäßen Dotanden.

In einer weiteren Ausführungsform der Erfindung werden die Verbindungen gemäß Formel (1) als emittierende Materialien eingesetzt. Die Verbindungen sind insbesondere dann als emittierende Verbindungen geeignet, wenn mindestens eine Gruppe Ar und/oder Y in Verbindungen der Formel (1) bzw. (3) bis (7) mindestens eine Arylamino-Einheit enthält. Bevorzugte Arylaminoeinheiten sind die Gruppen der vorne abgebildeten Formeln (32) und (33). Weiterhin sind die Verbindungen dann als emittierende Verbindungen geeignet, wenn die Gruppe Y in Verbindungen der Formel (1) bzw. (8) bis (24) für N bzw. NAr¹ steht.

Der Anteil der Verbindung gemäß Formel (1) in der Mischung der emittierenden Schicht beträgt zwischen 0.1 und 50.0 Gew.-%, bevorzugt zwischen 0.5 und 20.0 Gew.-%, besonders bevorzugt zwischen 1.0 und 10.0 Gew.-%. Entsprechend beträgt der Anteil des Hostmaterials zwischen 50.0 und 99.9 Gew.-%, bevorzugt zwischen 80.0 und 99.5 Gew.-%, besonders bevorzugt zwischen 90.0 und 99.0 Gew.-%.

Als Hostmaterialien kommen hierfür Materialien verschiedener Stoffklassen in Frage. Bevorzugte Hostmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911), der elektronenleitenden Verbindungen, insbesondere Ketone, Phosphinoxide, Sulfoxide, etc. (z. B. gemäß WO 05/084081 und WO 05/084082), der Atropisomere (z. B. gemäß WO 06/048268) oder der Boronsäurederivate (z. B. gemäß WO 06/117052). Weiterhin kommen als Hostmaterialien auch die oben beschriebenen erfindungsgemäßen Benz[a]anthracen-Verbindungen in Frage. Besonders bevorzugte Hostmaterialien sind außer den erfindungsgemäßen Verbindungen ausgewählt aus den Klassen der Oligoarylene, enthaltend Naphthalin, Anthracen und/oder Pyren oder Atropisomere dieser Verbindungen, der Oligoarylenvinylene, der Ketone, der Phosphinoxide und der Sulfoxide. Ganz besonders bevorzugte Hostmaterialien sind außer den erfindungsgemäßen Benz[a]anthracen-Verbindungen ausgewählt aus den Klassen der Oligoarylene, enthaltend Anthracen und/oder Pyren oder Atropisomere dieser Verbindungen, der Phosphinoxide und der Sulfoxide. Unter einem Oligoarylen im Sinne dieser Erfindung soll eine Verbindung verstanden werden, in der mindestens drei Aryl- bzw. Arylengruppen aneinander gebunden sind.

In nochmals einer weiteren Ausführungsform der Erfindung werden die Verbindungen gemäß Formel (1) als Lochtransportmaterial bzw. als Lochinjektionsmaterial eingesetzt. Die Verbindungen sind dann bevorzugt mit mindestens einer Gruppe N(Ar¹)₂ substituiert; insbesondere bevorzugt stellt mindestens ein Rest R eine Gruppe N(Ar¹)₂ dar. Die Gruppen N(Ar¹)₂ sind bevorzugt ausgewählt aus den oben beschriebenen Formeln (32) oder (33). Weiterhin sind die Verbindungen dann bevorzugt, wenn die Gruppe Y in Verbindungen der Formel (1) bzw. (8) bis (24) für N bzw. NAr¹ steht. Die Verbindung wird bevorzugt in einer Lochtransport- bzw. in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen einer Lochinjektionsschicht und einer Emissionsschicht liegt. Wenn die Verbindungen gemäß Formel (1) als Lochtransport- bzw. als Lochinjektionsmaterial verwendet werden, kann es bevorzugt sein, wenn sie mit Elektronenakzeptor-Verbindungen dotiert werden, beispielsweise mit F₄-TCNQ oder mit Verbindungen, wie in EP 1476881 oder EP 1596445 beschrieben.

In nochmals einer weiteren Ausführungsform der Erfindung werden die Verbindungen gemäß Formel (1) als Elektronentransportmaterial eingesetzt. Hier ist es bevorzugt, wenn ein oder mehrere Substituenten R und/oder R¹ mindestens eine Einheit C=O, P(=O) und/oder SO₂ enthalten, welche bevorzugt direkt an das Benz[a]anthracen gebunden ist. Ebenso ist es hier bevorzugt, wenn ein oder mehrere Substituenten R und/oder R¹ einen elektronenarmen Heterocyclus enthalten, wie beispielsweise Imidazol, Pyrazol, Thiazol, Benzimidazol, Benzothiazol, Triazol, Oxadiazol, Benzothiadiazol, Phenanthrolin, etc. Ebenso bevorzugt ist es, wenn in Verbindungen der Formel (1) bzw. (8) bis (24) eine oder mehrere Gruppen Ar¹ für einen elektronenarmen Heterocyclus steht, wie beispielsweise Imidazol, Pyrazol, Thiazol, Benzimidazol, Benzothiazol, Triazol, Oxadiazol, Benzothiadiazol, Phenanthrolin, etc. und/oder wenn die Gruppe Y für einen solchen elektronenarmen Heterocyclus oder für C=O, POAr¹, SO oder SO₂ steht. Weiterhin kann es bevorzugt sein, wenn die Verbindung mit Elektronendonorverbindungen dotiert ist.

Auch in Polymeren können Wiederholeinheiten gemäß Formel (1) entweder als Polymergrundgerüst (Backbone), als emittierende Einheit, als lochtransportierende Einheit und/oder als elektronentransportierende Einheit eingesetzt werden. Dabei entsprechen die bevorzugten Substitutionsmuster den oben beschriebenen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig. Hohe Löslichkeit lässt sich durch geeignete Substitution der Verbindungen erreichen.

Die erfindungsgemäßen Verbindungen weisen bei Verwendung in organischen Elektrolumineszenzvorrichtungen eine höhere Effizienz und eine deutlich erhöhte Lebensdauer auf, wodurch die erfindungsgemäßen organischen Elektrolumineszenzvorrichtungen besser für die Anwendung in hochwertigen und langlebigen Displays geeignet sind als solche, welche Materialien gemäß dem Stand der Technik enthalten. Weiterhin weisen die erfindungsgemäßen Verbindungen eine hohe thermische Stabilität und eine hohe Glasübergangstemperatur auf und lassen sich unzersetzt sublimieren.

Im vorliegenden Anmeldetext wird auf die Verwendung der erfindungsgemäßen Verbindungen in Bezug auf OLEDs und PLEDs und die entsprechenden Displays abgezielt. Trotz dieser Beschränkung der Beschreibung ist es für den Fachmann ohne weiteres erfinderisches Zutun möglich, die erfindungsgemäßen Verbindungen auch in anderen elektronischen Vorrichtungen einzusetzen, z. B. in organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Laserdioden (O-Laser) oder organischen Photorezeptoren.

Die Verwendung der erfindungsgemäßen Verbindungen in den entsprechenden Vorrichtungen ebenso wie diese Vorrichtungen selbst sind ebenfalls ein Gegenstand der vorliegenden Erfindung.

### Beispiele

Die nachfolgenden Synthesen werden, sofern nicht anders angegeben, unter einer Schutzgasatmosphäre durchgeführt. Die Edukte können von den Firmen ALDRICH bzw. ABCR bezogen bzw. nach Literaturmethoden dargestellt werden. 2- und 3-Brombenz[a]anthracen: Hallmark *et al., J. Lab. Comp. Radiopharm.* **1981**, *18(3)*, 331; 4-Brombenz[a]anthracen: Badgar et al., J. Chem. Soc. 1949, 799; 5-Brombenz[a]anthracen: Newman et al., J. Org. Chem. 1982, 47(15), 2837. Es war nicht möglich, zu allen Verbindungen die Glasübergangstemperatur mittels DSC-Messung zu bestimmen.

### Beispiel 1: Synthese von 5-Brombenz[a]anthracen

### a) 2-(1-Formylphenyl)-1-naphthylmethan

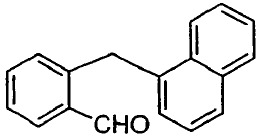

Aus 88.3 g (500 mmol) 1-Chlormethylnaphthalin und 12.2 g (500 mmol) Magnesium in 500 ml Ether wird die entsprechende Grignard-Verbindung hergestellt. Nach Abkühlen der Grignard-Lösung auf -78 °C versetzt man mit 59 ml (530 mmol) Trimethylborat, lässt dann auf Raumtemperatur erwärmen, entfernt das Lösemittel im Vakuum, versetzt den Rückstand mit 500 ml Toluol, 92.5 g (500 mmol) 2-Brombenzaldehyd, 2.9 g (2.5 mmol) Tetrakis(triphenylphosphino)palladium(O) und 300 ml 2 M Natriumcarbonatlösung und erhitzt die Reaktionsmischung 16 h unter Rückfluss. Nach Erkalten trennt man die organische Phase ab, wäscht zweimal mit je 500 ml Wasser, filtriert diese über-Kieselgel, engt dann zur Trockene ein und kristallisiert den Rückstand aus Toluol / Acetonitril um. Ausbeute: 93.1 g (378 mmol), 75.6 %, Reinheit ca. 97 % (NMR).

### b) 2-(1-Formylphenyl)-1-(4-bromnaphthyl)methan

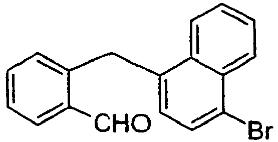

Eine auf 0 °C gekühlte Lösung von 86.2 g (350 mmol) 2-(1-Formylphenyl)-1-naphthylmethan in 1500 ml Dichlormethan wird unter Lichtausschluss tropfenweise mit einem Gemisch von 19.5 ml (380 mmol) Brom und 300 ml Dichlormethan versetzt. Nach 3 h Nachrühren gibt man 1000 ml 5 %ige Natriumsulfitlösung zu, rührt kurz nach, trennt die organische Phase ab, wäscht diese dreimal mit 500 ml Wasser, engt im Vakuum ein und nimmt den Rückstand in wenig Aceton auf. Nach 24 h stehen werden die Kristalle abgesaugt, mit Aceton : *n*-Hexan (1:1) gewaschen und im Vakuum getrocknet. Ausbeute: 90.7 g (279 mmol), 79.7 %, Reinheit ca. 98 % (NMR).

### c) 5-Brombenz[a]anthracen

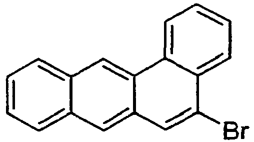

32.5 g (100 mmol) 2-(1-Formylphenyl)-1-(4-bromnaphthyl)methan werden bei 50 °C in 1000 g Polyphosphorsäure eingearbeitet. Die Masse wird 2 h auf 100 °C erhitzt und nach Erkalten in 5 1 Eiswasser aufgenommen. Der Feststoff wird abgesaugt, mit reichlich Wasser gewaschen, getrocknet und dann in Toluol über Aluminiumoxid säulenfiltriert. Ausbeute: 25.5 g (83 mmol), 83.0 %, Reinheit ca. 98 % (NMR).

### Beispiel 2: Synthese von Benz[a]anthracen-5-boronsäure

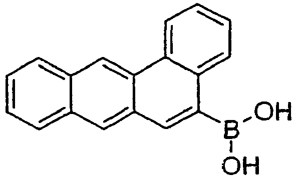

Eine Suspension von 30.7 g (100 mmol)) 5-Brombenz[a]anthracen in 1000 ml THF wird bei -78 °C unter gutem Rühren tropfenweise mit 52 ml (130 mmol) *n*-Buthyllithium (2.5 M in n-Hexan) versetzt und 2 h nachgerührt. Die rote Lösung wird unter gutem Rühren auf ein Mal mit 16.7 ml (150 mmol) Trimethylborat versetzt, 30 min. bei -78 °C nachgerührt, dann während 3 h auf Raumtemperatur erwärmt, mit 300 ml Wasser versetzt und 30 min. gerührt. Die organische Phase wird abgetrennt und im Vakuum zur Trockene eingeengt. Der Feststoff wird in 100 ml n-Hexan aufgenommen, abgesaugt, einmal mit 100 ml Hexan gewaschen und im Vakuum getrocknet. Ausbeute: 24.8 g (91 mmol), 91 %, Reinheit ca. 90 %ig (NMR) an Boronsäure, mit wechselnden Mengen an Boronsäureanhydrid und Borinsäure. Die Boronsäure kann ohne weitere Reinigung in dieser Form verwendet werden.

Analog zu Beispiel 2 werden aus den entsprechenden Bromiden die entsprechenden Boronsäuren erhalten (Beispiele 3 bis 6).

| Bsp. | Bromid | Boronsäure | Ausbeute |
|---|---|---|---|
| 3 | 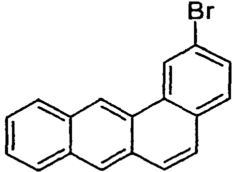 | 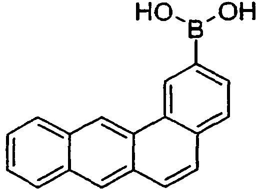 | 74.6 % |
| 4 | 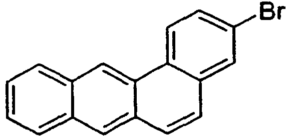 | 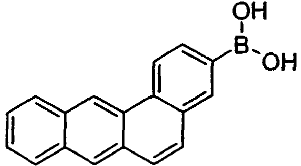 | 78.3% |
| 5 | 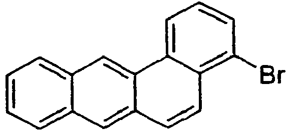 | 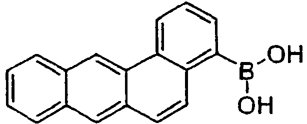 | 87.0% |
| 6 | 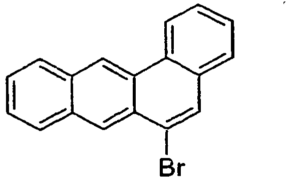 | 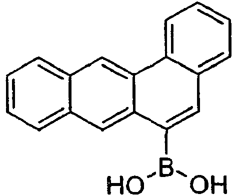 | 88.5% |

### Beispiel 7: Synthese von 9-(Naphth-2-yl)-10-(benz[a]anthracen-4-yl)-anthracen

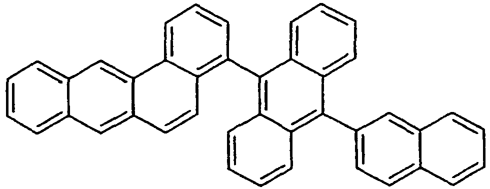

Eine gut gerührte Suspension von 19.2 g (50 mmol) 9-Brom-10-(2-naphthyl)anthracen, 15.0 g (55 mmol) Benz[a]anthracen-4-boronsäure, 25.5 g (120 mmol) Trikaliumphosphat in einem Gemisch aus 300 ml Toluol, 100 ml Dioxan und 400 ml Wasser wird mit 913 mg (3 mmol) Tri-o-tolylphosphin und dann 112 mg (0,5 mmol) Palladium(II)acetat versetzt und anschließend 16 h unter Rückfluss erhitzt. Nach Erkalten wird der ausgefallene Feststoff abgesaugt, dreimal mit 50 ml Toluol, dreimal mit 50 ml Ethanol : Wasser (1:1, v:v) und dreimal mit 100 ml Ethanol gewaschen, dreimal aus DMF (ca. 10 ml / g) umkristallisiert und anschließend zweimal sublimiert (p = 5 x 10⁻⁵ mbar, T = 330 °C). Ausbeute: 15.6 g (29 mmol), 58.8 %, Reinheit 99.9 % ig (HPLC), T_{g} = 168.9 °C.

Analog zu Beispiel 7 werden aus den entsprechenden Bromiden und Boronsäuren folgende erfindungsgemäße Verbindungen erhalten (Bsp. 8 bis 13).

| Bsp. | Boronsäure | Bromid | Produkt | Ausbeute |
|---|---|---|---|---|
| 8 | 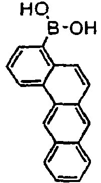 | 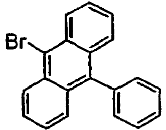 | 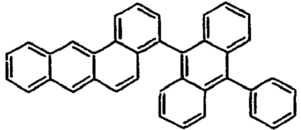 | 61.3 % |
| | | | | T_{g} = 147.9 °C |
| 9 | 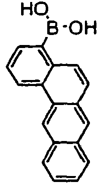 | 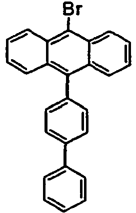 | 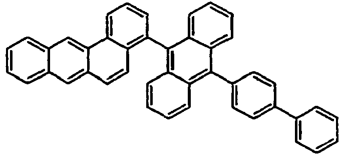 | 64.9 % |
| | | | | T_{g} = 161.4 °C |
| 10 | 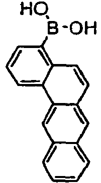 | 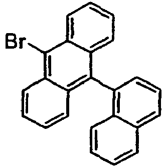 | 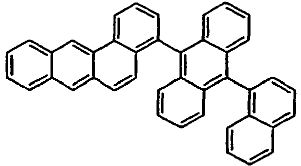 | 55.7 % |
| 11 | 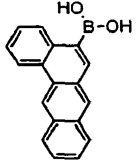 | 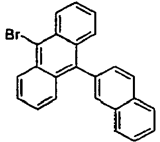 | 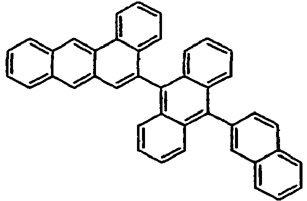 | 70.0% |
| | | | | T_{g} = 175.2 °C |
| 12 | 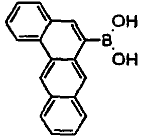 | 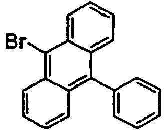 | 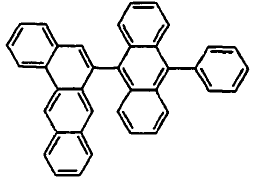 | 48.3 % |
| 13 | 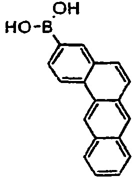 | 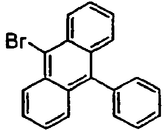 | 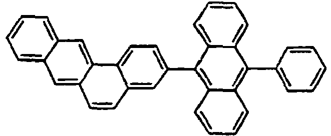 | 59.6 % |

### Beispiel 14: Synthese von 1,4-Bis(10-(benz[a]anthracen-4-yl)-anthracen-10-yl)benzol

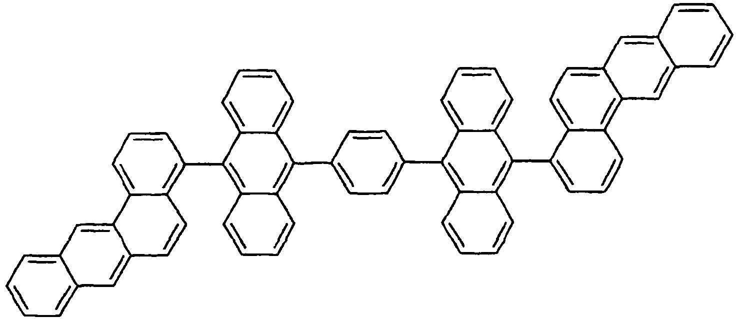

Darstellung analog Beispiel 7. Anstelle von 19.2 g (50 mmol) 9-Brom-10-(2-naphthyl)anthracen werden 14.7 g (25 mmol) 1,4-Bis(9-bromanthracen-10-yl)benzol eingesetzt. Umkristallisation dreimal aus NMP (ca. 10 ml/g); Sublimation (p = 5 × 10⁻⁵ mbar, T = 360 °C). Ausbeute: 14.2 g (16 mmol), 64.3 %, Reinheit 99.9 % ig (HPLC).

### Beispiel 15: Synthese von 1,3,5-Tris-(benz[a]anthracen-4-yl)benzol

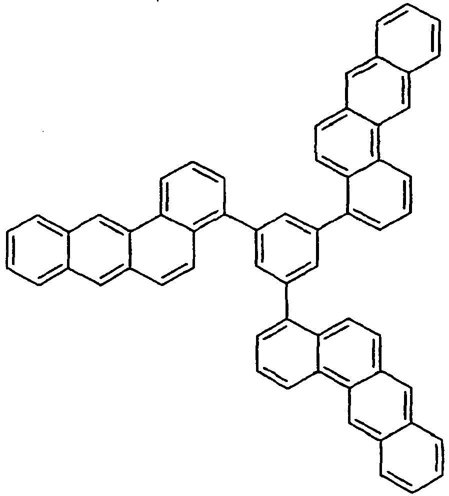

Darstellung analog Beispiel 7. Anstelle von 19.2 g (50 mmol) 9-Brom-10-(2-naphthyl)anthracen werden 5.0 g (16 mmol) 1,3,5-Tribrombenzol eingesetzt. Umkristallisation viermal aus o-Dichlorbenzol (ca. 25 ml/g); Sublimation (p = 5 × 10⁻⁵ mbar, T = 350 °C). Ausbeute: 8.4 g (11 mmol), 69.3 %, Reinheit 99.9 % ig (HPLC).

### Beispiel 16: Synthese von 5-(Benz[a]anthracen-5-yl)benz[a]anthracen

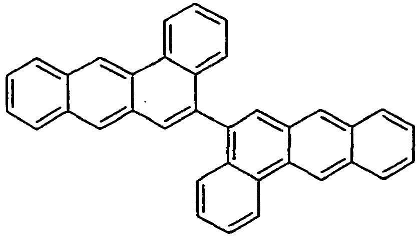

Darstellung analog Beispiel 7. Anstelle von 19.2 g (50 mmol) 9-Brom-10-(2-naphthyl)anthracen werden 15.4 g (50 mmol) 5-Brombenz[a]anthracen eingesetzt, und anstelle von Benz[a]anthracen-4-boronsäure wird Benz[a]-anthracen-5-boronsäure eingesetzt. Umkristallisation viermal aus o-Dichlorbenzol (ca. 15 ml/g); Sublimation (p = 5 x 10⁻⁵ mbar, T = 320 °C). Ausbeute: 15.0 g (33 mmol), 66.0 %, Reinheit 99.9 % ig (HPLC), T_{g} = 141.2 °C.

### Beispiel 17: Synthese von 4-(Benz[a]anthracen-5-yl)benz[a]anthracen

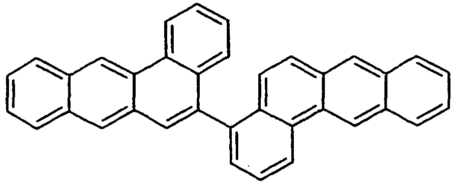

Darstellung analog Beispiel 7. Anstelle von 19.2 g (50 mmol) 9-Brom-10-(2-naphthyl)anthracen werden 15.4 g (50 mmol) 5-Brombenz[a]anthracen eingesetzt. Umkristallisation viermal aus o-Dichlorbenzol (ca. 15 ml/g); Sublimation (p = 5 x 10⁻⁵ mbar, T = 310 °C). Ausbeute: 16.4 g (36 mmol), 72.1 %, Reinheit 99.9 % ig (HPLC).

### Beispiel 18: Synthese von 1-Phenyl-2-(4-benz[a]anthracen-4-yl-phenyl)benzimidazol

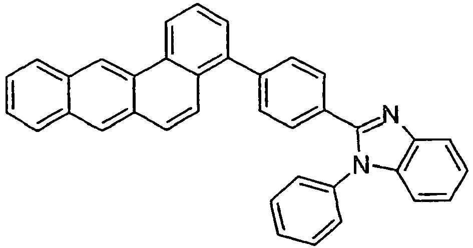

Darstellung analog Beispiel 7. Anstelle von 19.2 g (50 mmol) 9-Brom-10-(2-naphthyl)anthracen werden 17.5 g (50 mmol) 1-Phenyl-2-(4-bromphenyl)benzimidazol eingesetzt. Nach Erkalten der Reaktionsmischung wird die organische Phase abgetrennt, dreimal mit 300 ml Wasser gewaschen, über Kieselgel filtriert und zur Trocknen eingeengt. Der glasartige Rückstand wird in 50 ml siedendem Chloroform gelöst, und die Lösung wird mit 100 ml Ethanol versetzt. Nach 12 h stehen werden die farblosen Kristalle abgesaugt und anschließend an Kieselgel mit reinem Dichlormethan chromatographiert (Rf = 0.3). Abschließend wird noch einmal aus Chloroform / Ethanol umkristallisiert. Sublimation (p = 5 x 10⁻⁵ mbar, T = 310 °C). Ausbeute: 15.5 g (31 mmol), 62.4 %, Reinheit 99.9 % ig (HPLC), T_{g} = 110.9 °C.

### Beispiel 19: Synthese von 4-(Bis(3-methylphenyl)amino)benz[a]-anthracen

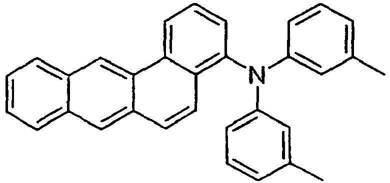

Eine Suspension von 15.4 g (50 mmol) 4-Brombenz[a]anthracen, 11.8 g (60 mmol) Bis(3-methylphenyl)amin und 7.7 g (80 mmol) Natrium-tert-butanolat in 500 ml Toluol wird mit 190 µl (1 mmol) Chor-di-tert-butylphosphin und dann mit 112 mg (0.5 mmol) Palladium(II)acetat versetzt und anschließend 5 h unter Rückfluss erhitzt. Nach Erkalten auf 60 °C wird mit 500 ml Wasser versetzt, die organische Phase wird abgetrennt, über Kieselgel filtriert, im Vakuum bei 80 °C fast bis zur Trockene eingeengt und dann mit 300 ml Ethanol versetzt. Nach Erkalten wird vom Feststoff abgesaugt. Umkristallisation fünfmal aus Dioxan (ca. 8 ml/g); Sublimation (p = 5 x 10⁻⁵ mbar, T = 280 °C). Ausbeute: 11.9 g (28 mmol), 56.1 %, Reinheit 99.9 ig (HPLC).

### Beispiel 20: Synthese von 9-(Phenyl)-10-(benz[a]anthracen-5-yl)-anthracen

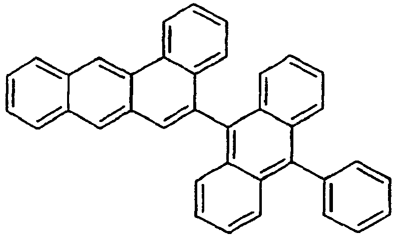

Eine gut gerührte Suspension von 16.7 g (50 mmol) 9-Brom-10-(phenyl)-anthracen, 15.0 g (55 mmol) Benz[a]anthracen-5-boronsäure, 25.5 g (120 mmol) Trikaliumphosphat in einem Gemisch aus 300 ml Toluol, 100 ml Dioxan und 400 ml Wasser wird mit 913 mg (3 mmol) Tri-o-tolylphosphin und dann mit 112 mg (0.5 mmol) Palladium(II)acetat versetzt und anschließend 16 h unter Rückfluss erhitzt. Nach Erkalten wird der ausgefallene Feststoff abgesaugt, dreimal mit 50 ml Toluol, dreimal mit 50 ml Ethanol : Wasser (1:1, v:v) und dreimal mit 100 ml Ethanol gewaschen, dreimal aus DMF (ca. 7 ml / g) umkristallisiert und anschließend zweimal sublimiert (p = 5 x 10⁻⁵ mbar, T = 320 °C). Ausbeute: 16.3 g (34 mmol), 67.8 %, Reinheit 99.9 % ig (HPLC), T_{g} = 150.0 °C.

Analog zu Beispiel 20 werden aus den entsprechenden Bromiden und Boronsäuren folgende erfindungsgemäße Verbindungen erhalten (Bsp. 21 bis 26).

| Bsp. | Boronsäure | Bromid | Produkt | Ausbeute |
|---|---|---|---|---|
| 21 | 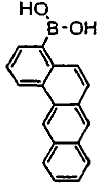 | 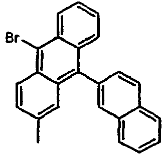 | 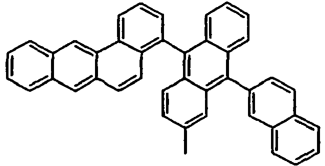 | 51.0 % |
| 22 | 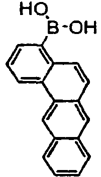 | 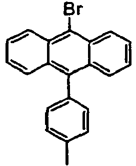 | 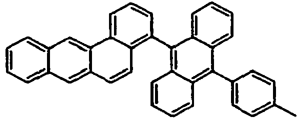 | 66.0 % |
| 23 | 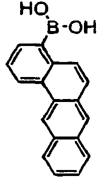 | 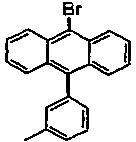 | 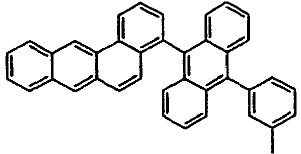 | 59.5 % |
| 24 | 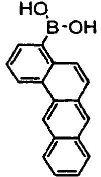 | 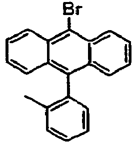 | 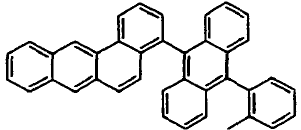 | 38.5 % |
| 25 | 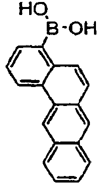 | 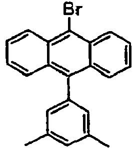 | 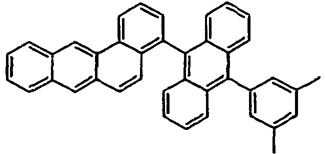 | 63.2 % |
| 26 | 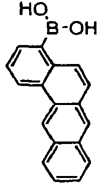 | 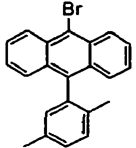 | 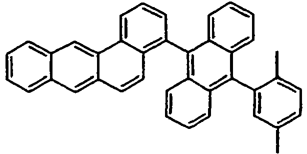 | 70.7 % |

### Beispiel 27: Synthese von 4-(Benz[a]anthracen-4-yl)benz[a]anthracen

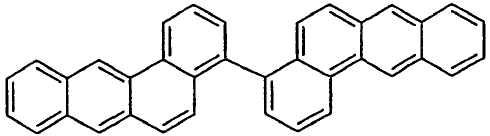

Darstellung analog Beispiel 7. Anstelle von 9-Brom-10-(2-naphthyl)-anthracen werden 15.4 g (50 mmol) 4-Brombenz[a]anthracen eingesetzt. Umkristallisation viermal aus o-Dichlorbenzol (ca. 15 ml/g); Sublimation (p = 5 x 10⁻⁵ mbar, T = 320 °C). Ausbeute: 16.8 g (37 mmol), 74.0 %, Reinheit 99.9 % ig (HPLC), T_{g} = 130.3 °C.

### Beispiel 28: Synthese von 1,4-Bis(benz[a]anthracen-4-yl))benzol

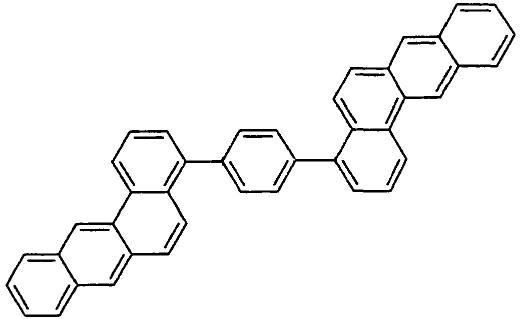

Darstellung analog Beispiel 7. Anstelle von 9-Brom-10-(2-naphthyl)-anthracen werden 4.1 g (25 mmol) Benzol-1,4-diboronsäure eingesetzt. Umkristallisation fünfmal aus o-Dichlorbenzol (ca. 20 ml/g); Sublimation (p = 5 x 10⁻⁵ mbar, T = 400 °C). Ausbeute: 11.1 g (21 mmol), 84.0 %, Reinheit 99.9 % ig (HPLC).

### Beispiel 29: Synthese von 4-(7-Phenyl-benz[a]anthracen-4-yl)-7-phenyl benz[a]anthracen

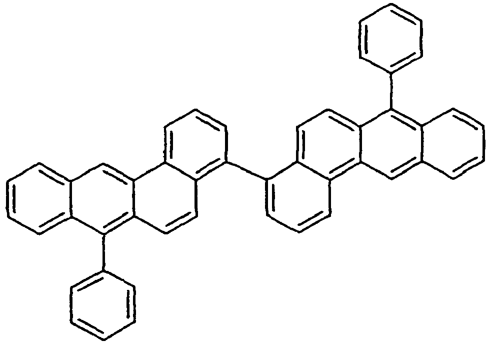

Eine Suspension von 22.7 g (50 mmol) 4-(Benz[a]anthracen-4-yl)benz[a]anthracen in 1000 ml DMF wird bei 100 °C unter gutem Rühren auf ein Mal mit 19.6 g (110 mmol) N-Bromsuccinimid versetzt. Die Suspension wird weitere 30 min. auf 100 °C gehalten und dann nach Erkalten mit 1000 ml Ethanol versetzt. Man saugt vom ausgefallenen Feststoff ab, wäscht diesen dreimal mit je 100 ml Ethanol und trocknet im Vakuum.
Der so erhaltene Feststoff wird analog Beispiel 7 mit 15.9 g (130 mmol) Benzolboronsäure umgesetzt. Umkristallisation fünfmal aus DMF (ca. 6 ml/g); Sublimation (p = 5 x 10⁻⁵ mbar, T = 360 °C). Ausbeute: 21.8 g (36 mmol), 72.0 %, Reinheit 99.9 % ig (HPLC), Tg = 181.4 °C.

Analog zu Beispiel 29 werden aus den entsprechenden Boronsäuren folgende erfindungsgemäße Verbindungen erhalten (Bsp. 30 bis 13).

| Bsp. | Boronsäure | Produkt | Ausbeute |
|---|---|---|---|
| 30 | 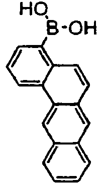 | 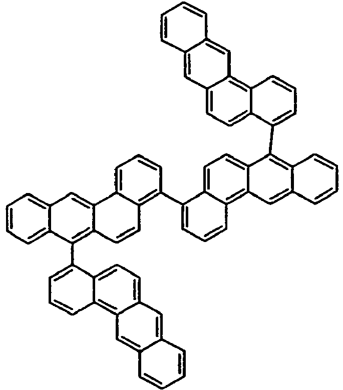 | 39.0 % |
| 31 | 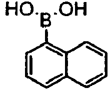 | 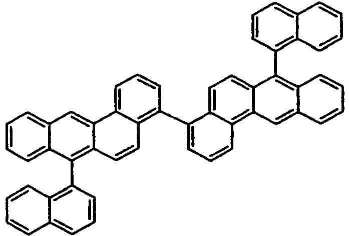 | 61.1 % |
| 32 | 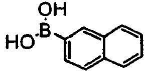 | 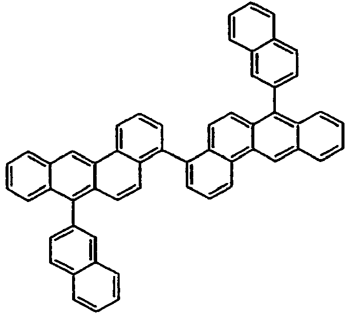 | 55.6% |
| 33 | 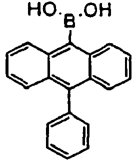 | 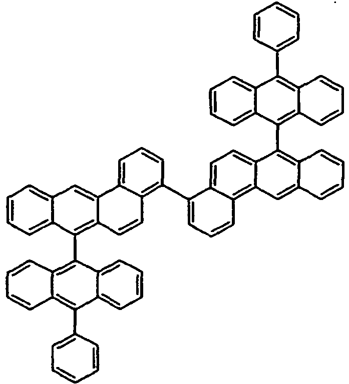 | 21.3 % |
| 34 | 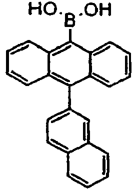 | 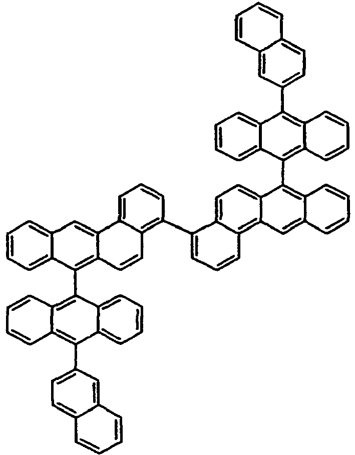 | 19.8 % |

### Beispiel 35: Synthese von 4,7-Bis(naphth-1-yl)benz[a]anthracen

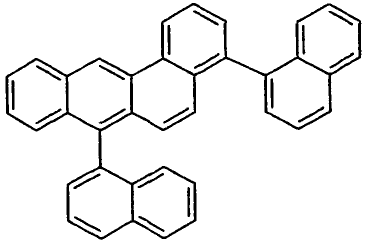

Eine Suspension von 15.4 g (50 mmol) 4-Brombenz[a]anthracen in 300 ml DMF wird bei 100 °C unter gutem Rühren auf ein Mal mit 19.6 g (110 mmol) N-Bromsuccinimid versetzt. Die Suspension wird weitere 30 min. auf 100 °C gehalten und nach Erkalten mit 1000 ml Ethanol versetzt. Man saugt vom ausgefallenen Feststoff ab, wäscht diesen dreimal mit je 100 ml Ethanol und trocknet im Vakuum. Der so erhaltene Feststoff wird analog Beispiel 7 mit 22.4 g (130 mmol) 1-Naphthalinboronsäure umgesetzt. Umkristallisation fünfmal aus DMF (ca. 4 ml/g); Sublimation (p = 5 x 10⁻⁵ mbar, T = 355 °C). Ausbeute: 12.5 g (26 mmol), 52.0 %, Reinheit 99.9 % ig (HPLC), Tg = 137.1 °C.

Analog zu Beispiel 35 werden aus den entsprechenden Boronsäuren folgende erfindungsgemäße Verbindungen erhalten (Bsp. 36 bis 40).

| Bsp. | Boronsäure | Produkt | Ausbeute |
|---|---|---|---|
| 36 | 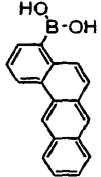 | 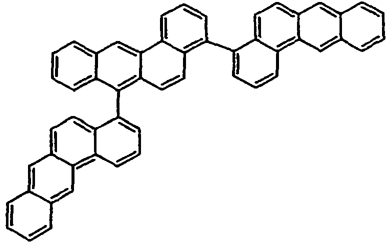 | 44.4 % |
| 37 | 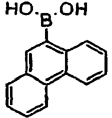 | 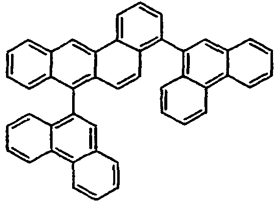 | 71.6% |
| 38 | 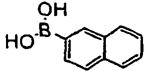 | 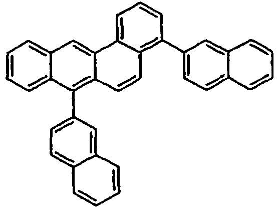 | 53.6 % |
| 39 | 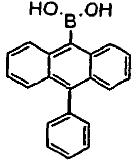 | 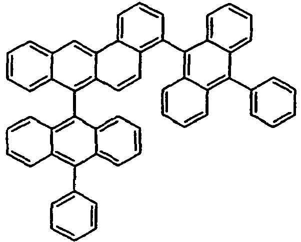 | 50.2% |
| 40 | 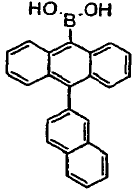 | 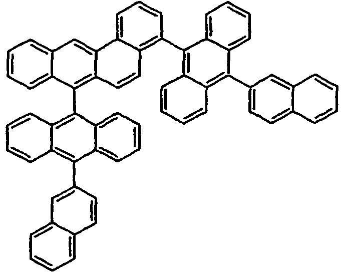 | 57.5 % |

### Beispiel 41: Synthese von 9-(Phenyl)-10-(7-phenyl-benz[a]anthracen-4-yl)anthracen

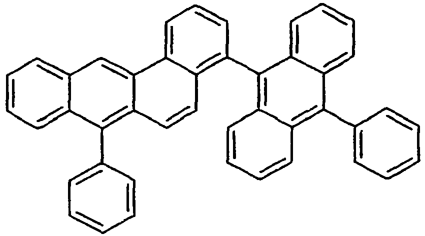

Eine gut gerührte Suspension von 12.9 g (50 mmol) 9-Bromanthracen, 15.0 g (55 mmol) Benz[a]anthracen-4-boronsäure, 25.5 g (120 mmol) Trikaliumphosphat in einem Gemisch aus 300 ml Toluol, 100 ml Dioxan und 400 ml Wasser wird mit 913 mg (3 mmol) Tri-o-tolylphosphin und dann mit 112 mg (0.5 mmol) Palladium(II)acetat versetzt und anschließend 16 h unter Rückfluss erhitzt. Nach Erkalten wird der ausgefallene Feststoff abgesaugt, dreimal mit 50 ml Toluol, dreimal mit 50 ml Ethanol : Wasser (1:1, v:v), dreimal mit 100 ml Ethanol gewaschen und abschließend getrocknet.

Eine Suspension von 18.2 g (45 mmol) des so erhaltenen 9-Benz[a]-anthracen-4-yl-anthracens in 500 ml DMF wird bei 100 °C auf ein Mal mit 16.9 g (95 mmol) N-Bromsuccinimid versetzt. Nach 3 h wird bei Raumtemperatur mit 500 ml Ethanol versetzt, der Feststoff wird abgesaugt, mit Ethanol gewaschen und getrocknet.
Eine gut gerührte Suspension von 22.5 g (40 mmol) des so erhaltenen 9-Brom-10-(7-Brom-benz[a]anthracen-4-yl)anthracen, 12.2 g (100 mmol) Benzolsäure, 25.5 g (120 mmol) Trikaliumphosphat in einem Gemisch aus 300 ml Toluol, 100 ml Dioxan und 400 ml Wasser wird mit 913 mg (3 mmol) Tri-o-tolylphosphin und dann 112 mg (0.5 mmol) Palladium(II)-acetat versetzt und anschließend 16 h unter Rückfluss erhitzt. Nach Erkalten wird der ausgefallene Feststoff abgesaugt, dreimal mit 50 ml Toluol, dreimal mit 50 ml Ethanol : Wasser (1:1, v:v), dreimal mit 100 ml Ethanol gewaschen und abschließend getrocknet. Umkristallisation fünfmal aus DMF (ca. 3 ml / g); Sublimation (p = 5 x 10⁻⁵ mbar, T = 350 °C). Ausbeute: 12.6 g (23 mmol), 46.0 %, Reinheit 99.9 % ig (HPLC), Tg = 171.8 °C.

Analog zu Beispiel 41 werden aus den entsprechenden Boronsäuren folgende erfindungsgemäße Verbindungen erhalten (Bsp. 42 bis 46).

| Bsp. | Boronsäure | Produkt | Ausbeute |
|---|---|---|---|
| 42 | 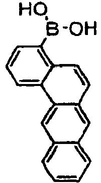 | 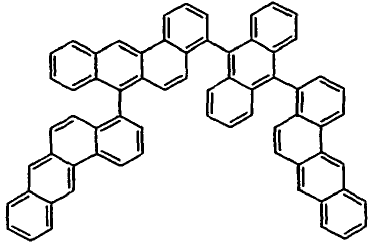 | 68.5 % |
| 43 | 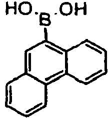 | 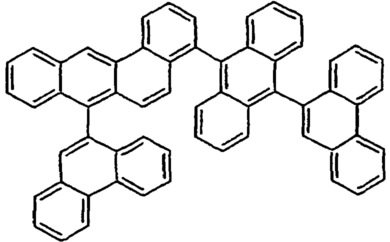 | 71.0 % |
| 44 | 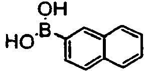 | 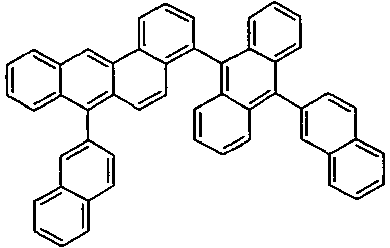 | 56.4 % |
| 45 | 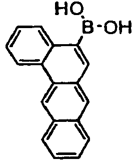 | 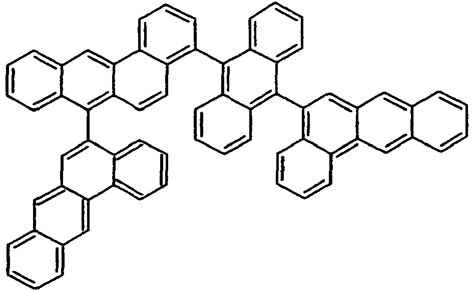 | 35.2 % |
| 46 | 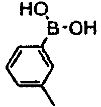 | 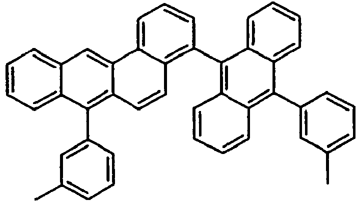 | 47.1 % |

### Beispiel 48: Synthese von 1,4-Bis(7-phenyl-(benz[a]anthracen-4-yl))benzol

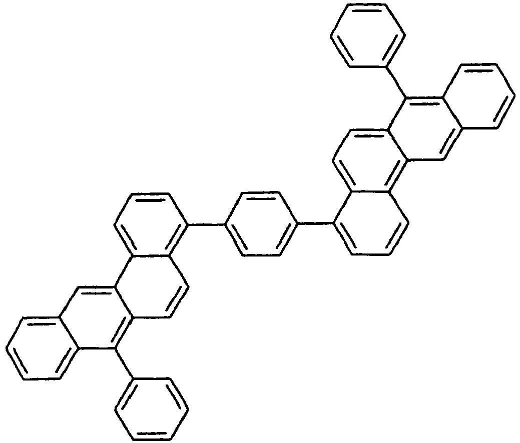

Eine Suspension von 26.5 g (50 mmol) 1,4-Bis-(benz[a]anthracen-4-yl)benzol und 19.6 g (110 mmol) N-Bromsuccinimid in 500 ml o-Dichlorbenzol wird unter gutem Rühren langsam zum Sieden erhitzt. Anschließend kocht man 2 h unter Rückfluss, lässt erkalten, saugt vom Niederschlag ab, wäscht diesen dreimal mit je 100 ml Ethanol und trocknet im Vakuum.
Eine gut gerührte Suspension von 21.2 g (40 mmol) des so erhaltenen 1,4-Bis(7-brom-(benz[a]anthracen-4-yl))benzol, 12.2 g (100 mmol) Benzolboronsäure, 25.5 g (120 mmol) Trikaliumphosphat in einem Gemisch aus 300 ml Toluol, 100 ml Dioxan und 400 ml Wasser wird mit 913 mg (3 mmol) Tri-o-tolylphosphin und dann 112 mg (0.5 mmol) Palladium(II)-acetat versetzt und anschließend 16 h unter Rückfluss erhitzt. Nach Erkalten wird der ausgefallene Feststoff abgesaugt, dreimal mit 50 ml Toluol, dreimal mit 50 ml Ethanol : Wasser (1:1, v:v), dreimal mit 100 ml Ethanol gewaschen und abschließend getrocknet. Umkristallisation fünfmal aus o-Dichlorbenzol (ca. 7 ml / g); Sublimation (p = 5 x 10⁻⁵ mbar, T = 400 °C). Ausbeute: 16.4 g (24 mmol), 48.0 %, Reinheit 99.9 % ig (HPLC), Tg = 176.7 °C.

Analog zu Beispiel 48 werden aus den entsprechenden Boronsäuren folgende erfindungsgemäße Verbindungen erhalten (Bsp. 49 bis 53).

| Bsp. | Boronsäure | Produkt | Ausbeute |
|---|---|---|---|
| 49 | 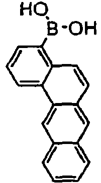 | 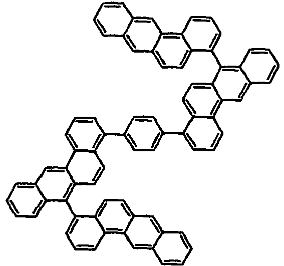 | 46.9 % |
| 50 | 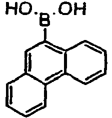 | 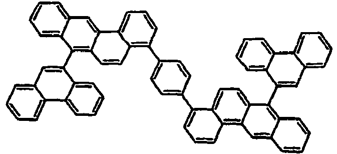 | 46.4 % |
| 51 | 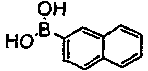 | 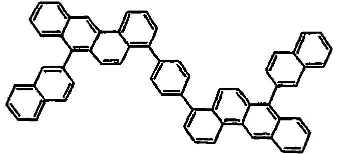 | 76.2 % |
| 52 | 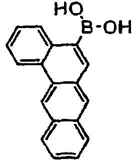 | 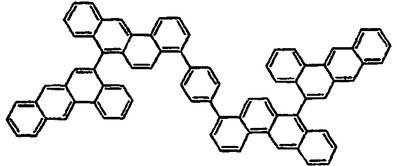 | 65.8 % |
| 53 | 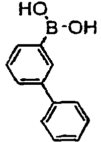 | 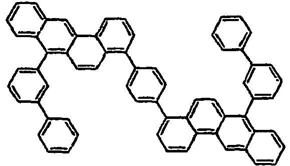 | 67.8 % |

### Beispiel 54: Synthese von 4-(Diphenyl)amino)benz[a]anthracen

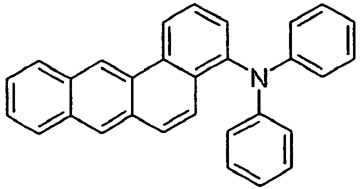

Eine Suspension von 15.4 g (50 mmol) 4-Brombenz[a]anthracen, 10.2 g (60 mmol) Diphenylamin und 7.7 g (80 mmol) Natrium-tert-butanolat in 500 ml Toluol wird mit 190 µl (1 mmol) Chor-di-tert-butylphosphin und dann mit 112 mg (0.5 mmol) Palladium(II)acetat versetzt und anschließend 5 h unter Rückfluss erhitzt. Nach Abkühlen auf 60 °C wird mit 500 ml Wasser versetzt, die organische Phase wird abgetrennt, über Kieselgel filtriert, im Vakuum bei 80 °C fast bis zur Trockene eingeengt und dann mit 300 ml Ethanol versetzt. Nach Erkalten wird vom Feststoff abgesaugt. Umkristallisation fünfmal aus Dioxan (ca. 8 ml/g); Sublimation (p = 5 x 10⁻⁵ mbar, T = 280 °C). Ausbeute: 12.7 g (32 mmol), 64.1 %, Reinheit 99.9 ig (HPLC), Tg = 74.7 °C.

Analog zu Beispiel 54 werden aus den entsprechenden Aminen folgende erfindungsgemäße Verbindungen erhalten (Bsp. 55 bis 59).

| Bsp. | Amin | Produkt | Ausbeute |
|---|---|---|---|
| 55 | 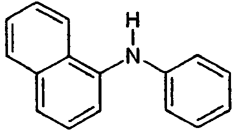 | 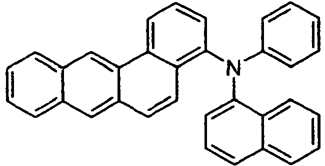 | 66.3 % |
| 56 | 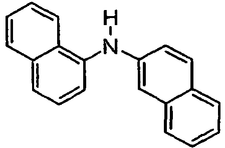 | 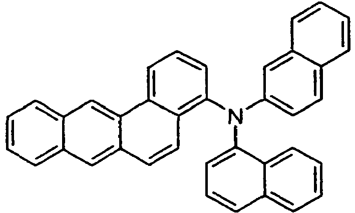 | 44.0% |
| 57 | 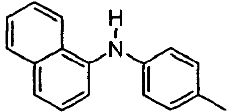 | 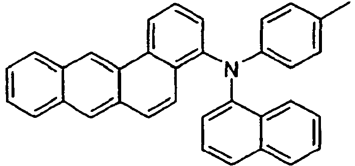 | 67.3 % |
| 58 | 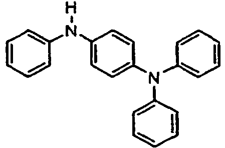 | 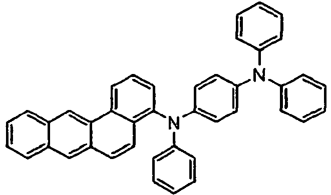 | 62.1 % |
| 59 | 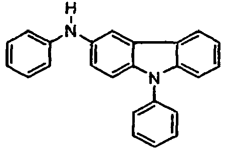 | 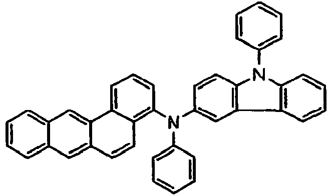 | 71.0% |

### Beispiel 60: Synthese von 4,7-Bis(diphenyl)amino)benz[a]anthracen

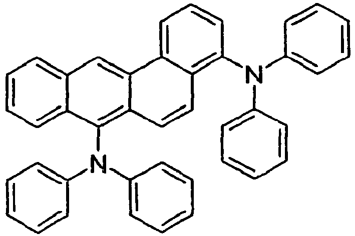

Eine Suspension von 19.3 g (50 mmol) 4,7-Dibrombenz[a]anthracen (Darstellung gemäß Bsp. 35), 20.4 g (120 mmol) Diphenylamin und 15.4 g (160 mmol) Natrium-tert-butanolat in 500 ml Toluol wird mit 380 µl (2 mmol) Chor-di-tert-butylphosphin und dann mit 224 mg (1 mmol) Palladium(II)acetat versetzt und anschließend 5 h unter Rückfluss erhitzt. Nach Abkühlen auf 60 °C wird mit 500 ml Wasser versetzt, die organische Phase wird abgetrennt, über Kieselgel filtriert, im Vakuum bei 80 °C fast bis zur Trockene eingeengt und dann mit 300 ml Ethanol versetzt. Nach Erkalten wird vom Feststoff abgesaugt. Umkristallisation fünfmal aus Dioxan (ca. 8 ml/g); Sublimation (p = 5 x 10⁻⁵ mbar, T = 300 °C). Ausbeute: 16.9 g (30 mmol), 60.2 %, Reinheit 99.9 ig (HPLC), Tg = 134.9 °C.

| Bsp. | Amin | Produkt | Ausbeute |
|---|---|---|---|
| 61 | 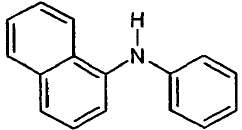 | 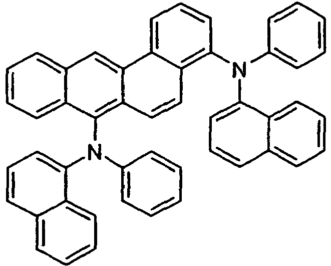 | 37.8 % |
| 62 | 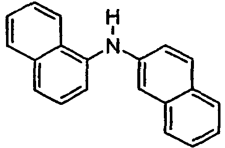 | 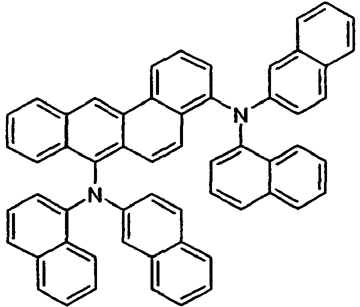 | 42.0 % |
| 63 | 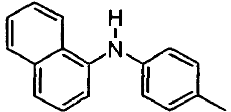 | 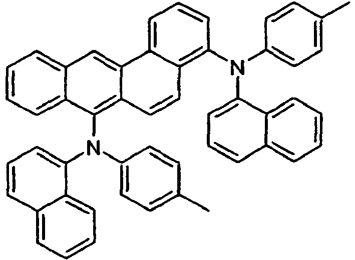 | 33.9 % |
| 64 | 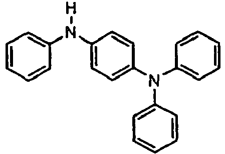 | 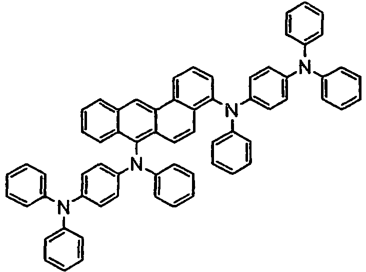 | 72.8 % |
| 65 | 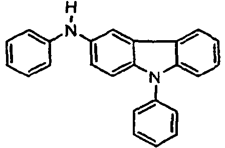 | 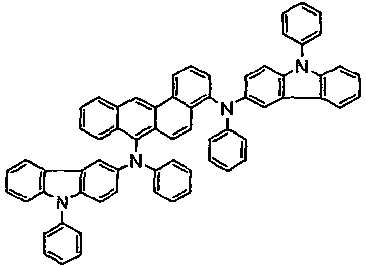 | 61.3 % |

### Beispiel 66: Synthese von 2-, 3-, 4-, 5- und 6-Brom(7,12-dideuteriumbenz[a]anthracen

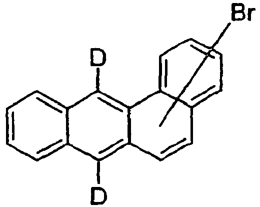

Eines Suspension von je 1.7 g (5 mmol) des entsprechenden 2-, 3-, 4-, 5- oder 6-Brombenz[a]anthracenchinons (2-Brom, 3-Brom: s. J. Org. Chem. 1983, 48(17), 2926; 4-Brom: s. J. Org. Chem. 1987, 52(26), 5668; 5-Brom: s. Bull. Chem. Soc. Jpn. 1976, 49(12), 3713) in einem Gemisch aus 4 ml 50 %iger D₃PO₂ in D₂O, 8 ml 57%iger DI in D₂O und 18 ml CD₃COOD wird 15 h unter Rückfluss erhitzt. Nach Erkalten wird der Feststoff abgesaugt, gut mit Wasser gewaschen, einmal in 50 ml EtOH ausgekocht und abschließend getrocknet. Der Deuterierungsgrad liegt bezogen auf den Deuterierungsgrad der Edukte D₃PO₂, DI und CD₃COOD bei > 99 %.

| Bsp. | Chinon | Produkt | Ausbeute |
|---|---|---|---|
| 67 | 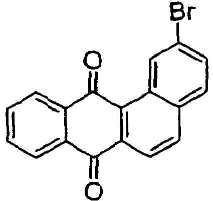 | 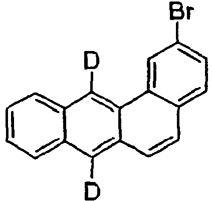 94.5 % | 94.5 % |
| 68 | 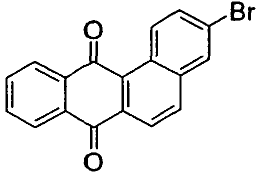 | 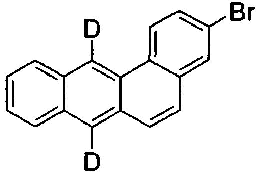 | 88.6 % |
| 69 | 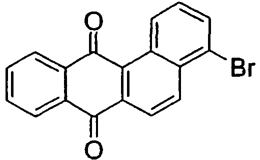 | 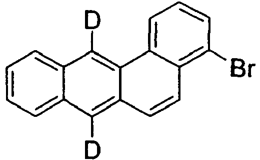 | 89.1 % |
| 70 | 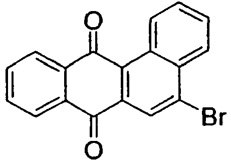 | 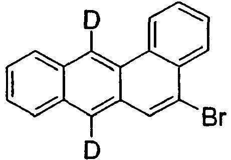 | 14.3 % |
| 71 | 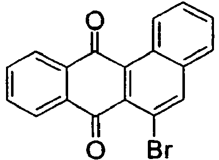 | 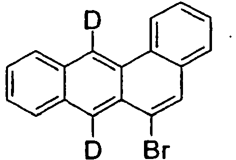 | 67.5 % |

Die so erhaltenen 2-, 3-, 4-, 5- und 6-Brom-(7,12-dideuterium-benz[a]-anthracene können analog zu den Beispielen 1 bis 60 zu den analogen 7- und/oder 12-deuterierten erfindungsgemäßen Verbindungen 1 bis 60 umgesetzt weden.

### Beispiel 72: Synthese von 4,7-Bis(4-phenyl-1H-benzimidazolyl)-benz[a]anthracen

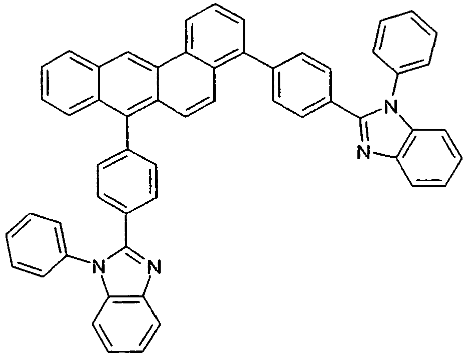

Eine gut gerührte Suspension von 11 g (31.5 mmol) 1-Phenyl-2-(4-bromphenyl)-benzimidazol, 4.4 g (14 mmol) Benz[a]anthracen-4,7-diboronsäure und 13 g (65 mmol) Trikaliumphosphat in einem Gemisch aus 300 ml Toluol, 100 ml Dioxan und 400 ml Wasser wird mit 913 mg (3 mmol) Tri-o-tolylphosphin und dann mit 112 mg (0.5 mmol) Palladium(II)acetat versetzt und anschließend 16 h unter Rückfluss erhitzt. Nach Erkalten wird der ausgefallene Feststoff abgesaugt, dreimal mit 50 ml Toluol, dreimal mit 50 ml Ethanol : Wasser (1:1, vv) und dreimal mit 100 ml Ethanol gewaschen, dreimal aus DMF (ca. 10 ml / g) umkristallisiert und anschließend zweimal sublimiert (p = 5 x 10⁻⁵ mbar, T = 308 °C). Ausbeute 17.5 g (23 mmol), 41 %, Reinheit 99.9 % ig (HPLC), T_{g} = 111.4 °C.

### Beispiel 73: Synthese von 4-Phenyl-7-(4-phenyl-1H-benzimidazolyl)-benz[a]anthracen

### a) 4-Phenyl-benz[a]anthracen

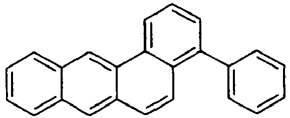

Eine gut gerührte Suspension von 7.8 g (50 mmol) Brombenzol, 15.0 g (55 mmol) Benz[a]anthracen-4-boronsäure und 25.5 g (120 mmol) Trikaliumphosphat in einem Gemisch aus 300 ml Toluol, 100 ml Dioxan und 400 ml Wasser wird mit 913 mg (3 mmol) Tri-o-tolylphosphin und dann mit 112 mg (0.5 mmol) Palladium(II)acetat versetzt und anschließend 16 h unter Rückfluss erhitzt. Nach Erkalten wird der ausgefallene Feststoff abgesaugt, dreimal mit 50 ml Toluol, dreimal mit 50 ml Ethanol : Wasser (1:1, v:v), dreimal mit 100 ml Ethanol gewaschen und abschließend getrocknet.

### b) 7-Brom-4-phenyl-benz[a]anthracen

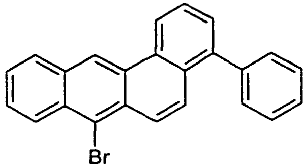

Eine Suspension von 18.2 g (45 mmol) 4-Phenylbenz[a]anthracen in 500 ml DMF wird bei 100 °C mit 16.9 g (95 mmol) N-Bromsuccinimid versetzt. Nach 3 h wird bei Raumtemperatur mit 500 ml Ethanol versetzt, der Feststoff wird abgesaugt, mit Ethanol gewaschen und getrocknet.

### c) 4-(4-Phenyl-benz[a]anthracen-7-yl)-benzaldehyd

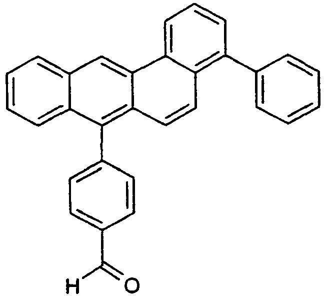

Eine gut gerührte Suspension von 15.3 g (40 mmol) 7-Brom-4-phenyl-benz[a]anthracen, 7.5 g (50 mmol) 4-Formylphenylboronsäure und 25.5 g (120 mmol) Trikaliumphosphat in einem Gemisch aus 300 ml Toluol, 100 ml Dioxan und 400 ml Wasser wird mit 913 mg (3 mmol) Tri-o-tolylphosphin und dann mit 112 mg (0,5 mmol) Palladium(II)acetat versetzt und anschließend 16 h unter Rückfluss erhitzt. Nach Erkalten wird der ausgefallene Feststoff abgesaugt, dreimal mit 50 ml Toluol, dreimal mit 50 ml Ethanol : Wasser (1:1, v:v), dreimal mit 100 ml Ethanol gewaschen und abschließend getrocknet. Umkristallisation aus DMF. Ausbeute: 14.3 g (35 mmol), 88 %.

### d) 7-(4-phenyl-1H-benzimidazol-yl)-4-phenyl-benzo[a]anthracen

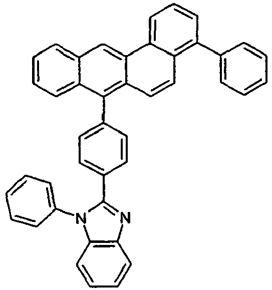

In einem gut ausgeheizten Kolben werden 18 g (45 mmol) 4-(4-Phenylbenz[a]anthracen-7-yl)-benzaldehyd und 15.3 g (81 mmol) N-phenyl-o-phenylendiamin in 900 ml DMF gelöst, tropfenweise mit 104 g (169 mmol) Kaliumhydrogenmonopersulfat versetzt, anschließend 1 h nachgerührt und dann 1 h auf auf 60 °C erhitzt. Nach Erkalten wird der ausgefallene Feststoff abgesaugt, dreimal mit 50 ml Ethanol : Wasser (1:1, v:v) und dreimal mit 100 ml Ethanol gewaschen und abschließend getrocknet. Umkristallisation fünfmal aus DMF (ca. 4 ml / g); Sublimation (p = 5 x 10⁻⁵ mbar, T = 320 °C). Ausbeute: 6 g (11 mmol), 26 %, Reinheit 99.9 % ig (HPLC), Tg = 130.4 °C.

### Beispiel 74: Synthese von 7-Naphth-1-yl-4-(4-phenyl-1H-benzimidazol)-benz[a]anthracen

### a) 4-Benz[a]anthracen-4-yl-benzaldehyd

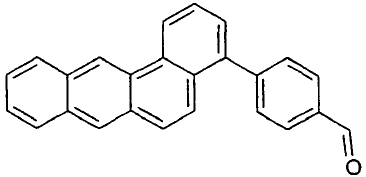

Eine gut gerührte Suspension von 50 g (333 mmol) 4-Formylphenylboronsäure, 81 g (266 mmol) 4-Brom-benz[a]anthracen und 118 g (558 mmol) Trikaliumphosphat in einem Gemisch aus 500 ml Toluol, 500 ml Dioxan und 400 ml Wasser wird mit 2.4 g (7.9 mmol) Tri-o-tolylphosphin und dann mit 300 mg (1.3 mmol) Palladium(II)acetat versetzt und anschließend 16 h unter Rückfluss erhitzt. Nach Erkalten wird der ausgefallene Feststoff abgesaugt, dreimal mit 50 ml Toluol, dreimal mit 50 ml Ethanol : Wasser (1:1, v:v), dreimal mit 100 ml Ethanol gewaschen und abschließend getrocknet. Ausbeute: 49.7 g (149 mmol), 56 %.

### b) 4-(7-Brom-benz[a]anthracen-4-yl)-benzaldehyd

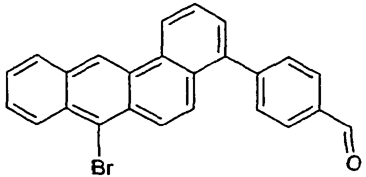

Eine Suspension von 49 g (150 mmol) 4-Benz[a]anthracen-4-yl-benzaldehyd in 800 ml DMF wird bei 100 °C mit 29.4 g (165 mmol) N-Bromsuccinimid versetzt. Nach 3 h wird bei Raumtemperatur mit 500 ml Ethanol versetzt, der Feststoff wird abgesaugt, mit Ethanol gewaschen und getrocknet. Ausbeute: 49.3 g (119 mmol), 79 %.

### c) 4-(7-Naphth-1-yl-benz(a)anthracen-4-yl)-benzaldebyd

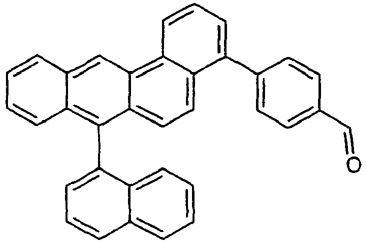

Eine gut gerührte Suspension von 49 g (115 mmol) Napthyl-1-boronsäure, 21 g (126 mmol) 4-(7-Brom-benz[a]anthracen-4-yl)-benzaldehyd, 36.6 g (172 mmol) Trikaliumphosphat in einem Gemisch aus 500 ml Toluol, 500 ml Dioxan und 400 ml Wasser wird mit 850 mg (2.8 mmol) Tri-o-tolylphosphin und dann 108 mg (0.48 mmol) Palladium(II)acetat versetzt und anschließend 16 h unter Rückfluss erhitzt. Nach Erkalten wird der ausgefallene Feststoff abgesaugt, dreimal mit 50 ml Toluol, dreimal mit 50 ml Ethanol : Wasser (1:1, v:v), dreimal mit 100 ml Ethanol gewaschen und abschließend getrocknet. Ausbeute: 76 g (161 mmol), 54 %.

### d) 7-Naphth-1-yl-4-(4-phenyl-1H-benzimidazol)-benz[a]anthracen

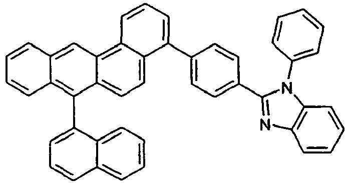

In einem gut ausgeheizten Kolben werden 76 g (116 mmol) 4-(7-Naphth-1-yl-benz[a]anthracen-4-yl)-benzaldehyd und 112 g (598 mmol) N-Phenyl-o-phenylendiamin in 900 ml DMF gelöst, tropfenweise mit 240 g (359 mmol) Kaliumhydrogenmonopersulfat versetzt, anschließend 1 h nachgerührt und dann 1 h auf auf 60 °C erhitzt. Nach Erkalten wird der ausgefallene Feststoff abgesaugt, dreimal mit 50 ml Ethanol : Wasser (1:1, v:v), dreimal mit 100 ml Ethanol gewaschen und abschließend getrocknet. Umkristallisation fünfmal aus DMF (ca. 4 ml / g); Sublimation (p = 5 x 10⁻⁵ mbar, T = 320 °C). Ausbeute: 60 g (96 mmol), 83 %, Reinheit 99.9 % ig (HPLC), Tg = 161.3 °C.

### Beispiel 75: Synthese von 7-(4-Phenyl-1H-benzimidazol)-4-(9-phenylanthracen-10-yl)-benz[a]anthracen

### a) 4-(9-Phenyl-anthracen-10-yl)-benz[a]anthracen

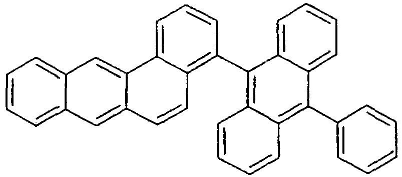

Eine gut gerührte Suspension von 20 g (60 mmol) 9-Brom-10-phenylanthracen, 19.8 g (73 mmol) 4-Brom-benz[a]anthracen und 19 g (89 mmol) Trikaliumphosphat in einem Gemisch aus 250 ml Toluol, 50 ml Dioxan und 250 ml Wasser wird mit 3 g (9.8 mmol) Tri-o-tolylphosphin und dann mit 391 mg (1.7 mmol) Palladium(II)acetat versetzt und anschließend 16 h unter Rückfluss erhitzt. Nach Erkalten wird der ausgefallene Feststoff abgesaugt, dreimal mit 50 ml Toluol, dreimal mit 50 ml Ethanol : Wasser (1:1, v:v), dreimal mit 100 ml Ethanol gewaschen und abschließend getrocknet. Ausbeute: 34 g (71 mmol), 97 %.

### b) 7-Brom-4-(9-phenyl-anthracen-10-yl)-benz[a]anthracen

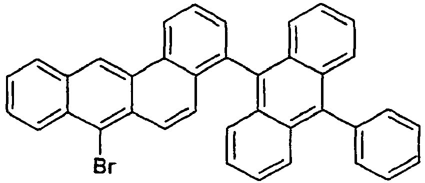

Eine Suspension von 55 g (116 mmol) 4-(9-Phenyl-anthracen-10-yl)-benz[a]anthracen in 500 ml DMF wird bei 100 °C mit 20 g (116 mmol) N-Bromsuccinimid versetzt. Nach 3 h wird bei Raumtemperatur mit 500 ml Ethanol versetzt, der Feststoff wird abgesaugt, mit Ethanol gewaschen und getrocknet. Ausbeute: 63 g (113 mmol), 65 %.

### c) 7-(p-Benzaldehyd)- 4-(9-phenyl-anthra-cen-10-yl)-benz[a]anthracen

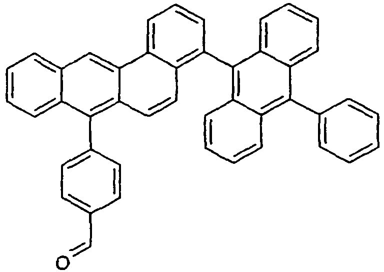

Eine gut gerührte Suspension von 21 g (145 mmol) 4-Formylphenylboronsäure, 66 g (119 mmol) 7-Brom-4-(9-phenyl-anthracen-10-yl)-benz[a]-anthracen und 49 g (234 mmol) Trikaliumphosphat in einem Gemisch aus 500 ml Toluol, 500 ml Dioxan und 400 ml Wasser wird mit 1.05 (3.6 mmol) Tri-o-tolylphosphin und dann mit 130 mg (0.58 mmol) Palladium(II)acetat versetzt und anschließend 16 h unter Rückfluss erhitzt. Nach Erkalten wird der ausgefallene Feststoff abgesaugt, dreimal mit 50 ml Toluol, dreimal mit 50 ml Ethanol : Wasser (1:1, v:v), dreimal mit 100 ml Ethanol gewaschen und abschließend getrocknet. Ausbeute: 44 g (76 mmol), 62 %.

### d) 7-(4-Phenyl-1H-benzimidazol)-4-(9-phenyl-anthracen-10-yl)-benz[a]anthracen

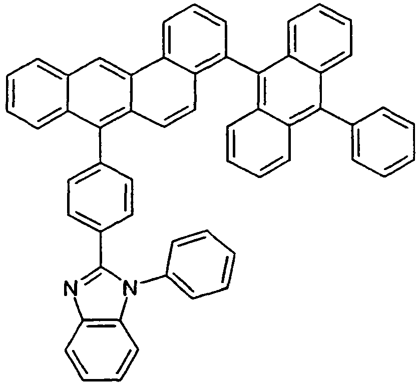

In einem gut ausgeheizten Kolben werden 20 g (34.2 mmol) 7-(p-Benzaldehyd)-4-(9-phenyl-anthracen-10-yl)-benz[a]anthracen und 23 g (123 mmol) N-Phenyl-o-phenylendiamin in 900 ml DMF gelöst, tropfenweise mit 157.7 g (256 mmol) Kaliumhydrogenmonopersulfat versetzt, anschließend 1 h nachgerührt und dann 1 h auf auf 60 °C erhitzt. Nach Erkalten wird der ausgefallene Feststoff abgesaugt, dreimal mit 50 ml Ethanol : Wasser (1:1, v:v), dreimal mit 100 ml Ethanol gewaschen und abschließend getrocknet. Umkristallisation fünfmal aus DMF (ca. 4 ml / g); Sublimation (p = 5 x 10⁻⁵ mbar, T = 320 °C). Ausbeute: 10 g (13 mmol), 38 %, Reinheit 99.9 %ig (HPLC).

### Beispiel 76: Herstellung der OLEDs

Die Herstellung von OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen 21 bis 34 werden die Ergebnisse verschiedener OLEDs vorgestellt. Glasplatten, die mit strukturiertem ITO (Indium Zinn Oxid) beschichtet sind, bilden die Substrate der OLEDs. Zur verbesserten Prozessierung wird 20 nm PEDOT (aus Wasser aufgeschleudert; bezogen von H. C. Starck, Goslar, Deutschland; Poly(3,4-ethylendioxy-2,5-thiophen)) auf das Substrat aufgebracht. Die OLEDs bestehen aus folgender Schichtenfolge: Substrat / PEDOT / Lochtransportschicht (HTM) 40 nm / Emissionschicht (EML) 30 nm / Elektronentransportschicht (ETM) 20 nm und abschließend eine Kathode. Die Materialien bis auf PEDOT werden in einer Vakuumkammer thermisch aufgedampft. Dabei besteht die Emissionsschicht immer aus einem Matrixmaterial (Host) und einem Dotierstoff (Dotand), der durch Coverdampfung dem Host beigemischt wird. Die Kathode wird durch eine 1 nm dünne LiF-Schicht und eine darauf abgeschiedene 100 nm Al-Schicht gebildet. Die Tabelle 1 zeigt die chemischen Strukturen der zum Aufbau der OLEDs verwendeten Materialien.

Diese OLEDs werden standardmäßig charakterisiert; hierfür werden die Elektrolumineszenzspektren, die Effizienz (gemessen in cd/A), die Leistungseffizienz (gemessen in Im/W) in Abhängigkeit der Helligkeit, berechnet aus Strom-Spannungs-Helligkeit-Kennlinien (IUL-Kennlinien), und die Lebensdauer bestimmt. Als Lebensdauer wird die Zeit definiert, nach der die Anfangshelligkeit von 6000 cd/m² auf die Hälfte gesunken ist.

In Tabelle 2 sind die Ergebnisse einiger OLEDs (Beispiele 71 bis 82) zusammengefasst. Als erfindungsgemäße Host-Materialien werden die Verbindungen der Beispiele 7, 8 und 11 verwendet. Als Vergleich wird der Host H1 gemäß dem Stand der Technik verwendet.

In Tabelle 3 sind die Ergebnisse von OLEDs (Beispiele 83 und 84) zusammengefasst, welche als erfindungsgemäßes Elektronentransportmaterial die Verbindung aus Beispiel 18 enthalten. Als Vergleich wird als Elektronentransportmaterial AlQ₃ gemäß dem Stand der Technik verwendet.

**Tabelle 1**

| | |
|---|---|
| 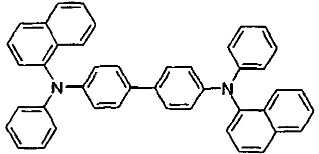 | 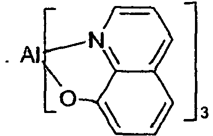 |
| HTM1 | AlQ₃ |
| 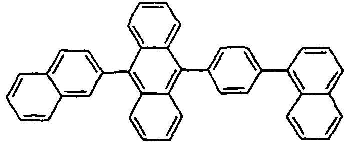 | 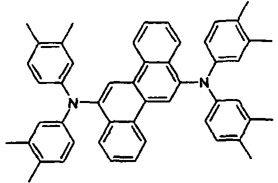 |
| H1 | D1 |
| 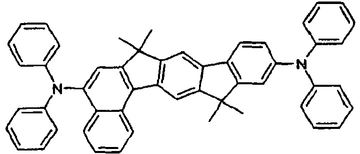 | 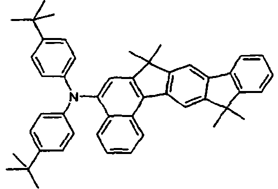 |
| D2 | D3 |
| 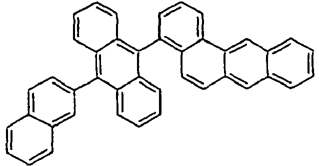 | 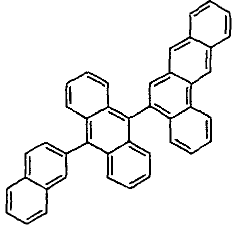 |
| Bsp. 7 | Bsp. 11 |
| 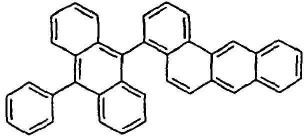 | 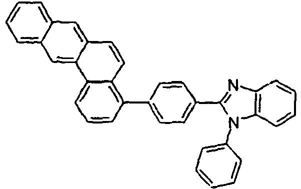 |
| Bsp. 8 | Bsp. 18 |

**Tabelle 2**

| **Beispiel** | **EML** | **Max. Effizienz (cd/A)** | **Spannung (V) bei 1000 cd/m²** | **CIE** | **Lebensdauer (h) bei 6000 cd/m²** |
|---|---|---|---|---|---|
| 77 (Vergleich) | H1 + 5% D1 | 6.8 | 5.8 | x=0.14/ y=0.19 | 210 |
| 78 (Vergleich) | H1 + 5% D2 | 7.0 | 5.9 | x=0.14/ y=0.18 | 240 |
| 79 (Vergleich) | H1 + 5% D3 | 6.9 | 5.7 | x=0.14/ y=0.21 | 280 |
| 80 | Bsp. 7 + 5% D1 | 7.1 | 5.9 | x=0.14/ y=0.20 | 270 |
| 81 | Bsp. 7 + 5% D2 | 7.4 | 5.6 | x=0.14/ y=0.17 | 280 |
| 82 | Bsp. 7 + 5% D3 | 7.3 | 6.1 | x=0.14/ y=0.22 | 340 |
| 83 | Bsp. 11 + 5% D1 | 7.2 | 5.9 | x=0.14/ y=0.19 | 270 |
| 84 | Bsp. 11 + 5% D2 | 7.5 | 5.5 | x=0.14/ y=0.17 | 310 |
| 85 | Bsp. 11 + 5% D3 | 7.6 | 5.9 | x=0.14/ y=0.21 | 400 |
| 86 | Bp. 8+ 5% D1 | 7.0 | 5.8 | x=0.14/ y=0.19 | 260 |
| 87 | Bsp. 8 + 5%D2 | 7.3 | 5.4 | x=0.14/ y=0.17 | 300 |
| 88 | Bsp. 8 + 5% D3 | 7.1 | 5.4 | x=0.14/ y=0.20 | 430 |

**Tabelle 3**

| **Beispiel** | **EML** | **ETM** | **Max. Effizienz (cd/A)** | **Spannung (V) bei 1000 cd/m²** | **CIE** |
|---|---|---|---|---|---|
| 89 (Vergleich) | Bsp. 7 + 5% D2 | AlQ₃ | 7.4 | 5.6 | x=0.14/ y=0.17 |
| 90 | Bsp. 7 + 5% D2 | Bsp. 18 | 7.6 | 5.2 | x=0.14_{/} y=0.17 |

Wie aus den oben aufgeführten Beispielen hervorgeht, weisen die erfindungsgemäßen organischen Elektrolumineszenzvorrichtungen, welche die erfindungsgemäßen Verbindungen als Hostmaterialien enthalten, höhere Effizienzen und Lebensdauern auf als Vorrichtungen gemäß dem Stand der Technik. Wird zusätzlich statt AlQ₃ ein erfindungsgemäßes Material als Elektronentransportmaterial verwendet, wie im obigen Beispiel 84, so lässt sich die Effizienz der Vorrichtung nochmals steigern und die Spannung verringern.

### Beispiel 91: Herstellung der OLEDs in modifizierten Schichtaufbau

In den folgenden Beispielen 86 bis 105 werden die Ergebnisse verschiedener OLEDs vorgestellt. Dabei bestehen die OLEDs aus folgender Schichtenfolge: Substrat / Lochinjektionsschicht (HIL1) 5 nm / Lochtransportschicht (HTM x) 60 nm / Lochtransportschicht (HTM3) 20 nm / Emissionschicht (EML) 40 nm / Elektronentransportschicht (ETM) 20 nm und abschließend eine Kathode. Die Materialien werden prozessiert, wie in Beispiel 70 aufgeführt. Die Kathode wird durch eine 1 nm dünne LiF-Schicht und eine darauf abgeschiedene 150 nm Al-Schicht gebildet. Die Tabelle 4 zeigt die chemischen Strukturen der zum Aufbau der OLEDs verwendeten Materialien.

Diese OLEDs werden standardmäßig charakterisiert; hierfür werden die Elektrolumineszenzspektren, die Effizienz (gemessen in cd/A), die Leistungseffizienz (gemessen in Im/W) in Abhängigkeit der Helligkeit, berechnet aus Strom-Spannungs-Helligkeit-Kennlinien (IUL-Kennlinien), und die Lebensdauer bestimmt. Als Lebensdauer wird die Zeit definiert, nach der die Anfangshelligkeit von 6000 cd/m² (im Fall von Blau) oder 25000 cd/m² (im Fall von Grün) auf die Hälfte gesunken ist.

In Tabelle 5 bis 7 sind die Ergebnisse einiger OLEDs (Beispiele 86 bis 105) zusammengefasst. Als erfindungsgemäße Dotanden bzw. Hostmaterialien bzw. Elektrontransportmaterialien wurden die in Tabelle 4 aufgeführten Verbindungen verwendet. In den Vergleichsbeispielen werden der Dotand D1 und das Hostmaterial H1 gemäß dem Stand der Technik verwendet.

**Tabelle 4**

| | | |
|---|---|---|
| 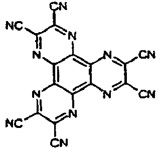 | 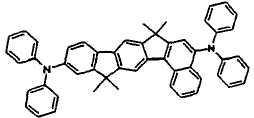 | 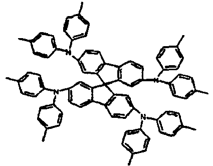 |
| HIL1 | HTM 1 | HTM 2 |
| 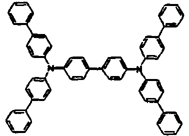 | 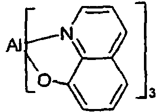 | 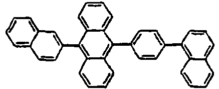 |
| HTM 3 | ETM1 | H1 |
| 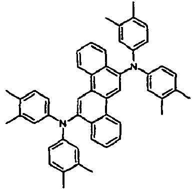 | 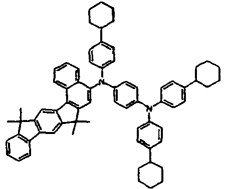 | 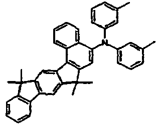 |
| D1 | D2 | D3 |
| 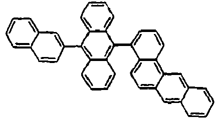 | 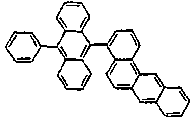 | 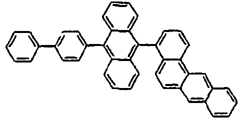 |
| Bsp. 7 | Bsp. 8 | Bsp. 9 |
| 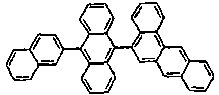 | 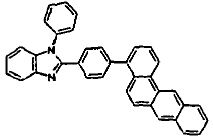 | 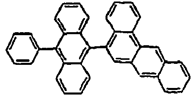 |
| Bsp. 11 | Bsp. 18 | Bsp. 20 |
| 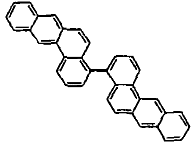 | 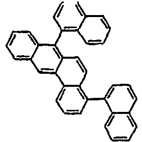 | 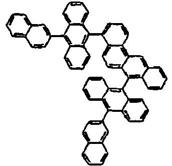 |
| Bsp. 27 | Bsp. 35 | Bsp. 41 |

**Tabelle 5**

| **Beispiel** | **HTM** | **EML** | **ETM** | **Farbe** | **Max. Effizienz (cd/A)** | **Spannung (V) bei 1000 cd/m²** | **CIE** | **Lebensdauer bei 6000 cd/m² (h)** |
|---|---|---|---|---|---|---|---|---|
| 92 (Vergleich) | HTM2 | H1 + 5% D1 | ETM1 | blau | 6.5 | 5.5 | x=0.14/ y=0.18 | 450 |
| 93 | HTM2 | Bsp. 7 + 5% D1 | ETM1 | blau | 6.9 | 5.6 | x=0.141 y=0.19 | 530 |
| 94 | HTM2 | Bsp. 8 + 5% D1 | ETM1 | blau | 7.0 | 5.5 | x=0.14/ y=0.20 | 580 |
| 95 | HTM2 | Bsp. 20 + 5% D1 | ETM1 | blau | 7.2 | 5.6 | x=0.14/ y=0.19 | 460 |
| 96 | HTM2 | Bsp. 27 + 5% D1 | ETM1 | blau | 6.5 | 5.3 | x=0.14/ y=0.18 | 430 |
| 97 | HTM2 | Bsp. 8 + 5% D1 | Bsp. 18 | blau | 8.5 | 5.1 | x=0.14/ y=0.18 | 560 |
| 98 | HTM2 | Bsp. 20 + 5% D1 | Bsp. 18 | blau | 8.7 | 5.2 | x=0.14/ y=0.17 | 580 |

**Tabelle 6**

| **Beispiel** | **HTM** | **EML** | **ETM** | **Farbe** | **Max Effizienz (cd/A)** | **Spannung (V) bei 1000cd/m²** | **CIE** | **Lebensdauer bei 25000cd/m² (h)** |
|---|---|---|---|---|---|---|---|---|
| 99 (Vergleich) | HTM1 | H2 + 5% D2 | ETM1 | Grün | 18.5 | 5.1 | x=0.29/ y=0.60 | 380 |
| 100 | HTM1 | H2 + 5% D2 | Bsp. 18 | Grün | 24.5 | 4.8 | x=0.29/ y=0.62 | 430 |
| 101 | HTM1 | Bsp. 8 + 5% D2 | ETM1 | Grün | 21.5 | 5.0 | x=0.29/ y=0.60 | 550 |
| 102 | HTM1 | Bsp. 8 + 5% D2 | Bsp. 18 | Grün | 26.0 | 4.7 | x=0.29/ y=0.63 | 540 |
| 103 | HTM1 | Bsp. 8 + 5% D2 | ETM1 | Grün | 21.5 | 5.0 | x=0.29/ y=0.60 | 550 |
| 104 | HTM1 | Bsp. 9 + 5% D2 | ETM1 | Grün | 22.5 | 4.9 | x=0.29/ y=0.60 | 530 |
| 105 | HTM1 | Bsp. 11 + 5% D2 | ETM1 | Grün | 23.0 | 4.8 | x=0.29/ y=0.63 | 500 |

**Tabelle 7**

| **Beispiel** | **HTM** | **EML** | **ETM** | **Farbe** | **Max Effizienz (cd/A)** | **Spannung (V) bei 1000cd/m²** | **CIE** | **Lebensdauer bei 6000cd/m² (h)** |
|---|---|---|---|---|---|---|---|---|
| 106 (Vergleich) | HTM2 | H1 + 5% D3 | ETM1 | blau | 4.5 | 5.8 | x=0.14/ y=0.12 | 150 |
| 107 | HTM2 | Bsp. 27 + 5% D3 | ETM1 | blau | 4.8 | 5.9 | x=0.14/ y=0.11 | 110 |
| 108 | HTM2 | Bsp. 35 + 5% D3 | ETM1 | blau | 4.9 | 5.8 | x=0.14/ y=0.10 | 130 |
| 109 | HTM2 | Bsp. 8 + 5% D3 | Bsp. 18 | blau | 4.8 | 5.6 | x=0.14/ y=0.12 | 230 |
| 110 | HTM2 | Bsp. 8 + 5% D3 | ETM1 | blau | 4.5 | 5.8 | x=0.14/ y=0.12 | 250 |
| 111 | HTM2 | Bsp. 35 + 5% D3 | ETM1 | blau | 3.9 | 5.6 | x=0.14/ y=0.09 | 90 |

## Patentansprüche

1. Ungeladene Verbindungen der Formel (1), 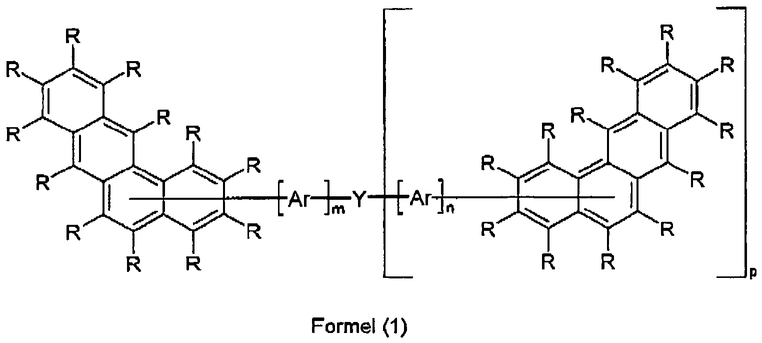 wobei die Gruppe Ar bzw. Y über eine der Positionen 2, 3, 4, 5 oder 6 des Benz[a]anthracens gebunden ist und entsprechend an dieser Position kein Rest R gebunden ist und wobei für die Symbole und Indizes gilt:
Ar ist bei jedem Auftreten gleich oder verschieden ein bivalentes aromatisches oder heteroaromatisches Ringsystem mit 5 bis 40 aromatischen Ringatomen, welches mit einem oder mehreren Resten R substituiert sein kann;
Y ist, abhängig vom Index p, ein mono-, bi-, tri- tetra-, penta- oder hexavalentes aromatisches oder heteroaromatisches Ringsystem mit 5 bis 40 aromatischen Ringatomen, welches durch einen oder mehrere Reste R substituiert sein kann; oder Y ist, wenn p = 0 ist, eine Gruppe N(Ar¹)₂, C(=O)Ar¹ oder P(=O)(Ar¹)₂; oder Y ist, wenn p = 1 ist, eine Einfachbindung oder C=O, 0, S, SO, Sol, NR¹, NAr¹, PAr¹, P(=O)Ar¹, P(=S)Ar¹, O-B(Ar¹)-O, O-BR¹-O, -CR¹=CR¹-, -C≡C-, eine Alkylen- oder Alkylidengruppe mit 1 bis 20 C-Atomen, die jeweils auch mit einem oder mehreren Resten R¹ substituiert sein kann, wobei eine oder mehrere nicht benachbarte CH₂-Gruppen durch R¹C=CR¹, C≡C, Si(R¹)₂, Ge(R¹)₂, Sn(R¹)₂, C=O, C=S, C=Se, C=NR¹, P(=O)(R¹), SO, SO₂, NR¹, O, S oder CONR¹ ersetzt sein können und wobei ein oder mehrere H-Atome durch F, Cl, Br, I, CN oder NO₂ ersetzt sein können; oder Y ist, wenn p = 2 ist, gleich B, B₃O₃, CR¹, CAr¹, N, P, P=O oder P=S;
R ist gleich oder verschieden bei jedem Auftreten H, D, F, Cl, Br, I, CHO, N(Ar¹)₂, N(R¹)₂, C(=O)Ar¹, P(Ar¹)₂, P(=O)(Ar¹)₂, S(=O)Ar¹, S(=O)₂Ar¹, CR¹=CR¹Ar¹, CN, NO₂, Si(R¹)₃, B(OAr¹)₂, B(OR¹)₂, OSO₂R¹, OH, eine geradkettige Alkyl- oder Thioalkoxygruppe mit 1 bis 40 C-Atomen oder eine geradkettige Alkoxygruppe mit 2 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R¹ substituiert sein können, wobei eine oder mehrere nicht benachbarte CH₂-Gruppen durch R¹C=CR¹, C=C, Si(R¹)₂, Ge(R¹)₂, Sn(R¹)₂, C=O, C=S, C=Se, C=NR¹, P(=O)(R¹), SO, SO₂, NR¹, O, S oder CONR¹ ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere nicht-aromatische Reste R substituiert sein kann, oder eine Aryloxy- oder Heteroaryloxygruppe mit 5 bis 60 aromatischen Ringatomen, die durch einen oder mehrere nicht-aromatische Reste R substituiert sein kann, oder eine Kombination dieser Systeme; dabei können zwei oder mehrere benachbarte Substituenten R auch miteinander ein mono- oder polycyclisches aliphatisches Ringsystem bilden;
Ar¹ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R substituiert sein kann; dabei können auch zwei Reste Ar¹, weiche an dasselbe Stickstoff- oder Phosphoratom binden, durch eine Einfachbindung oder eine Brücke, ausgewählt aus B(R¹), C(R¹)₂, Si(R¹)₂, C=O, C=NR¹, C=C(R¹)₂, O, S, S=O, SO₂, N(R¹), P(R¹) und P(=O)R¹, miteinander verknüpft sein;
R¹ ist bei jedem Auftreten gleich oder verschieden H oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere benachbarte Substituenten R¹ auch miteinander ein mono- oder polycyclisches aliphatisches oder aromatisches Ringsystem bilden;
m, n ist bei jedem Auftreten gleich oder verschieden 0, 1, 2 oder 3;
p ist 0, 1, 2, 3, 4 oder 5;
dabei sind die folgenden Verbindungen von der Erfindung ausgenommen:

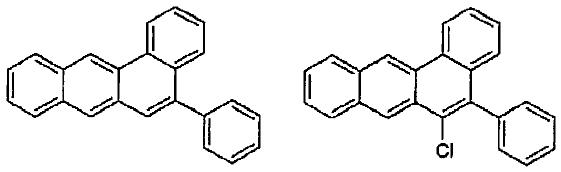

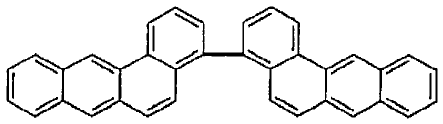

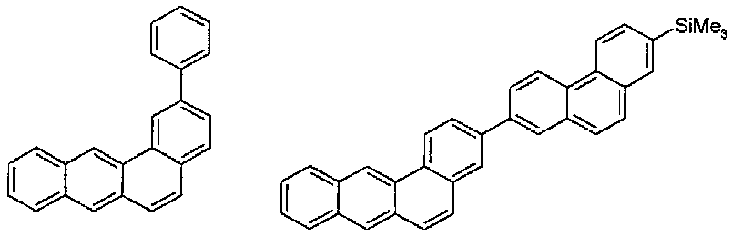

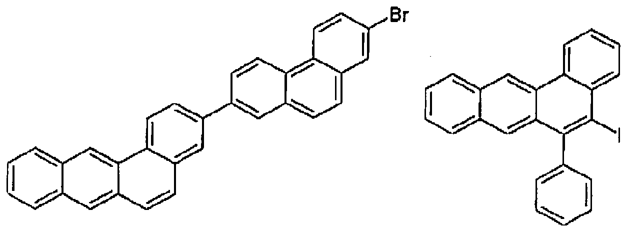

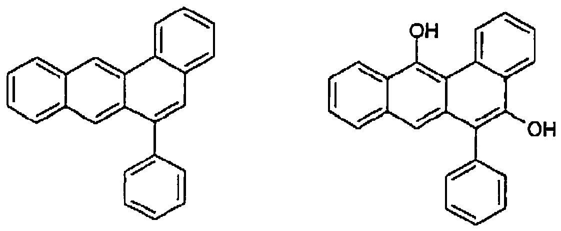

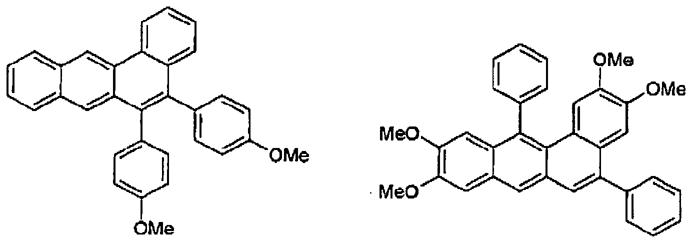

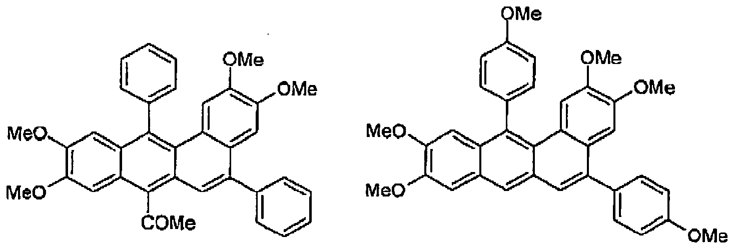

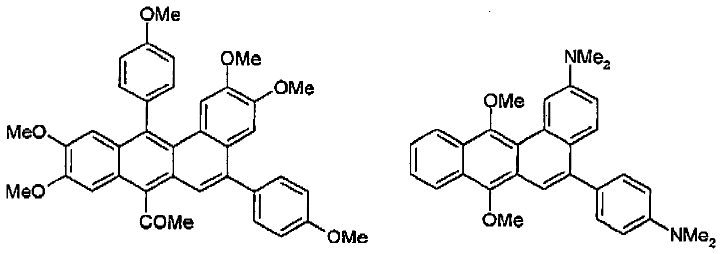

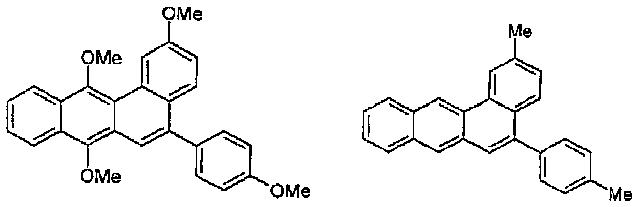

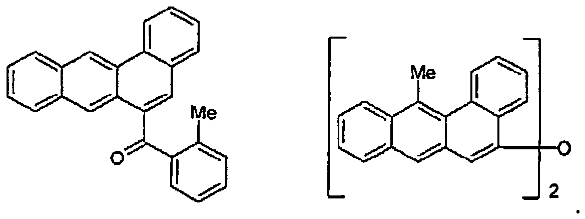

2. Verbindungen nach Anspruch 1 gemäß Formel (2), 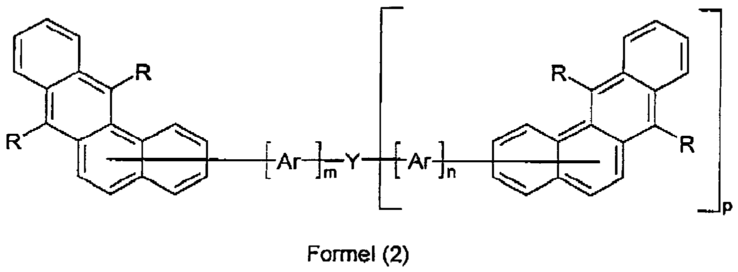 wobei die Gruppe Ar bzw. Y über eine der Positionen 2, 3, 4, 5 oder 6 des Benz[a]anthracens gebunden ist und wobei die Symbole und Indizes dieselbe Bedeutung haben, wie in Anspruch 1 beschrieben.

3. Verbindungen nach Anspruch 1 oder 2, ausgewählt aus den Formeln (3) bis (24), 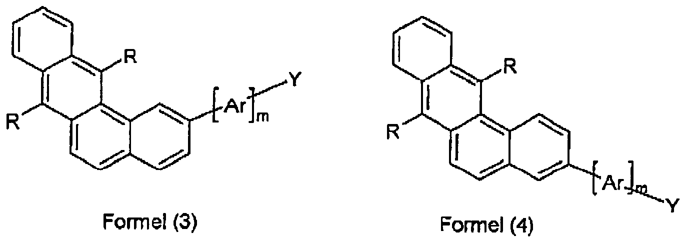 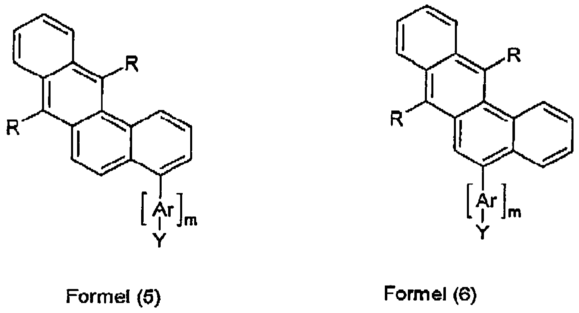 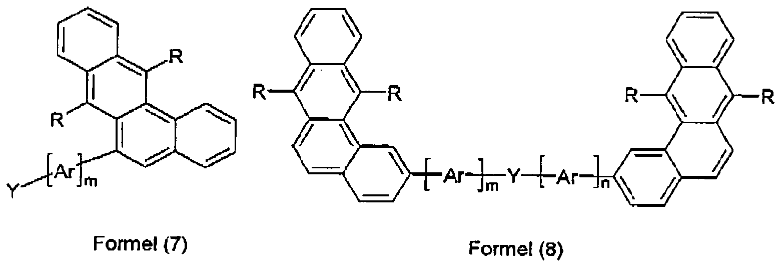 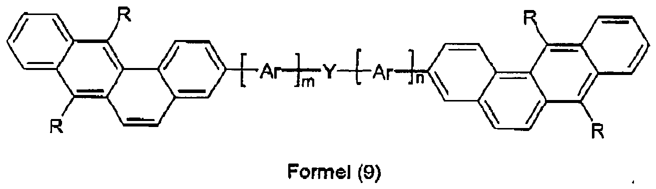 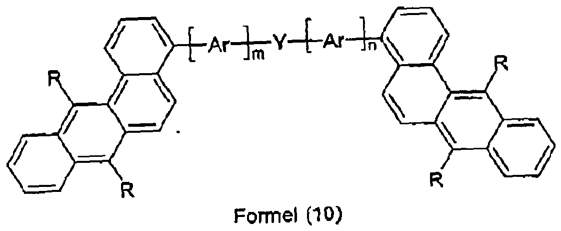 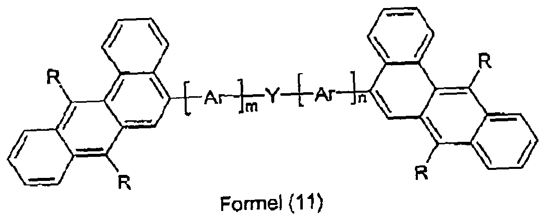 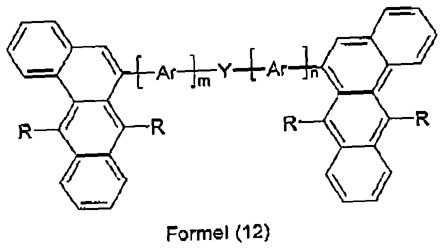 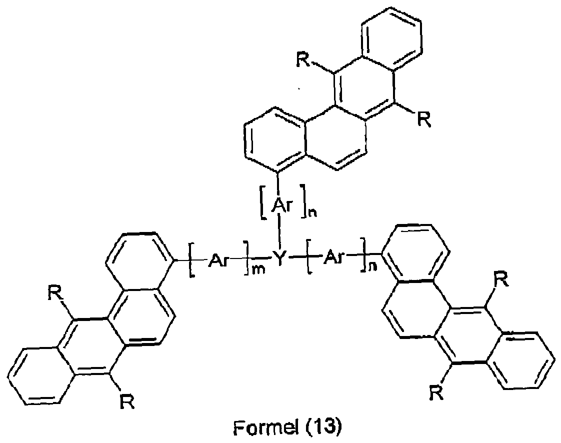 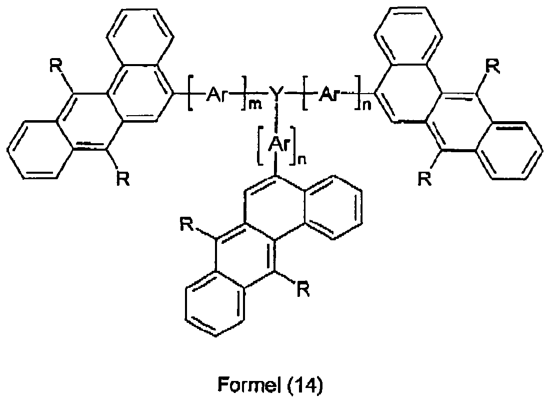 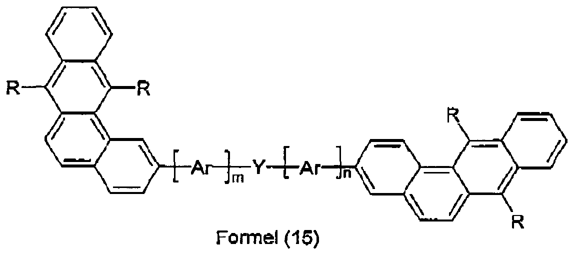 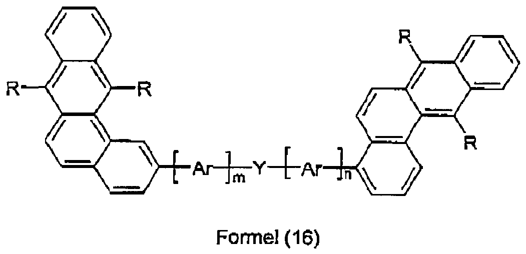 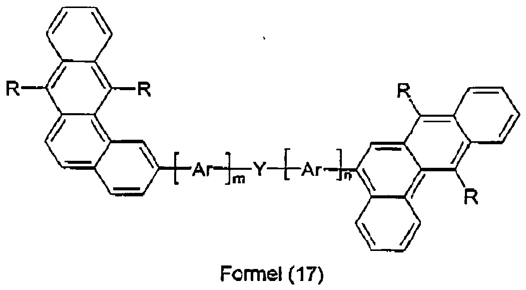 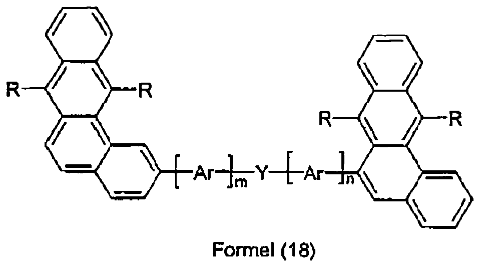 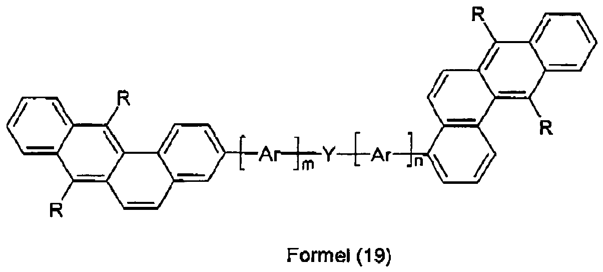 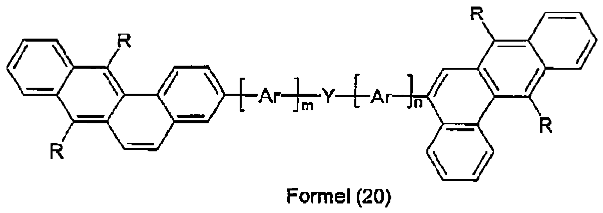 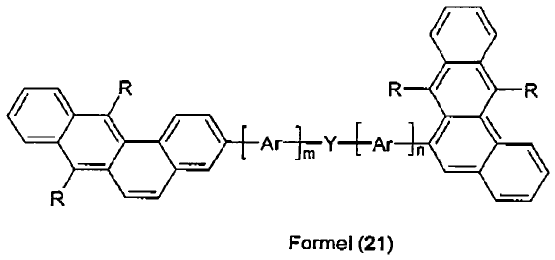 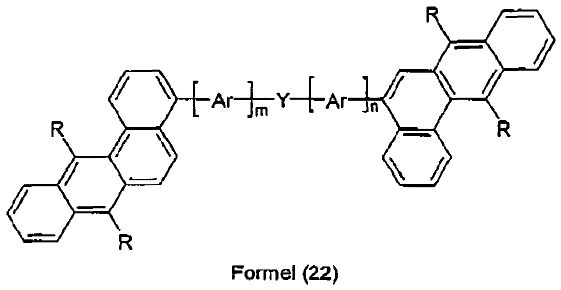 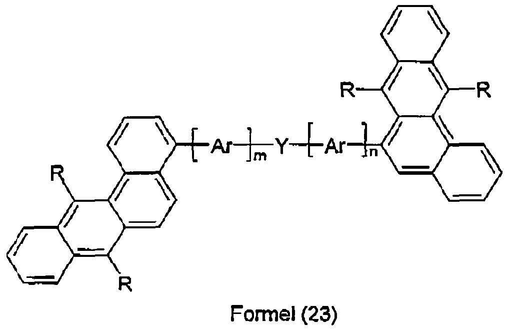 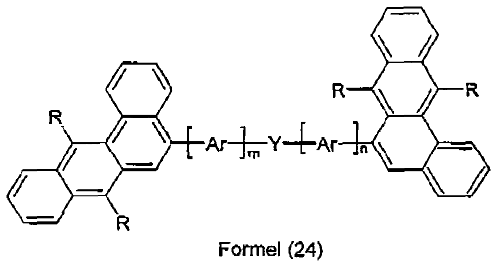 wobei die Symbole und Indizes dieselbe Bedeutung haben, wie unter Anspruch 1 beschrieben, wobei der Benz[a]anthracen-Grundkörper statt Wasserstoff auch Deuterium tragen kann und wobei die Gruppe Y in den Formeln (3) bis (7) für ein monovalentes aromatisches oder heteroaromatisches Ringsystem oder eine Gruppe N(Ar¹)₂ steht.

4. Verbindungen nach einem oder mehreren der Ansprüche 1 bis 3, **dadurch gekennzeichnet, dass** die Gruppe Y aus den Gruppen Benzol, Naphthalin, Anthracen, Carbazol, Phenanthren, Benzanthracen, Chrysen, Pyren, Phenanthrolin, Phenanthroimidazol, 1,3,5-Triazin und Benzimidazol aufgebaut ist oder, wenn p = 0 ist, eine Gruppe N(Ar¹)₂ darstellt.

5. Verbindungen nach einem oder mehreren der Ansprüche 1 bis 4, **dadurch gekennzeichnet, dass** die Gruppe Y für p = 0 bzw. in Verbindungen der Formel (3) bis (7) ausgewählt ist aus den Gruppen der folgenden Formeln (25) bis (33), 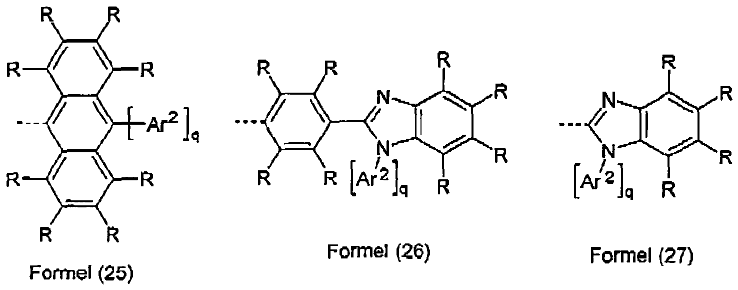 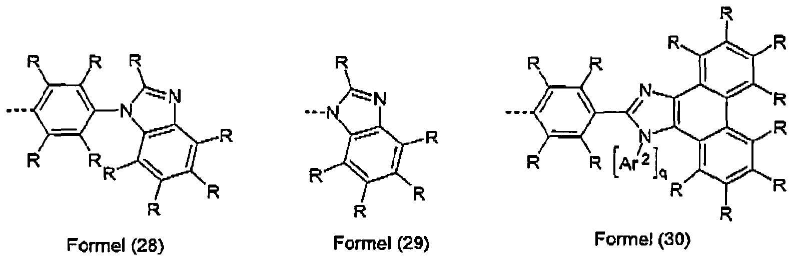 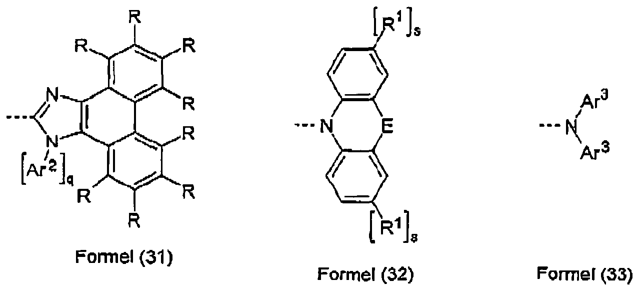 wobei R und R¹ die in Anspruch 1 aufgeführte Bedeutung haben und weiterhin gilt:
Ar² ist eine Aryl- oder Heteroarylgruppe mit 5 bis 16 aromatischen Ringatomen, bevorzugt Phenyl, 1-Naphthyl, 2-Naphthyl-, 9-Anthryl, Chrysenyl, 1-Pyrenyl, 2-Pyrenyl, 2-Phenanthrenyl, 3-Phenanthrenyl, 9-Phenanthrenyl, 2-Benzimidazol, 2-Fluorenyl, 2-Spirobifluorenyl, Fluoranthenyl, 2-Benz[a]anthracenyl, 3-Benz-[a]anthracenyl, 4-Benz[a]anthracenyl, 5-Benz[a]anthracenyl oder 6-Benz[a]anthracenyl, welche jeweils mit einem oder mehreren Resten R¹ substituiert sein kann, oder in Formel (25) eine Gruppe der Formel (32) oder (33);
Ar³ ist gleich oder verschieden bei jedem Auftreten eine Aryl- oder Heteroarylgruppe mit 5 bis 20 aromatischen Ringatomen oder eine Triarylamingruppe mit 15 bis 30 aromatischen Ringatomen, welche jeweils mit einem oder mehreren Resten R¹ substituiert sein kann, bevorzugt eine Aryl- oder Heteroarylgruppe mit 6 bis 14 aromatischen Ringatomen oder eine Triarylamingruppe mit 18 bis 30 aromatischen Ringatomen, bevorzugt mit 18 bis 22 aromatischen Ringatomen, welche jeweils mit einem oder mehreren Resten R¹ substituiert sein kann;
E steht für eine Einfachbindung, O, S, M(R¹) oder C(R¹)₂, wobei die beiden Reste R¹ durch Ringbildung auch ein Spirosystem aufspannen können;
q ist 1, 2 oder 3;
s ist bei jedem Auftreten gleich oder verschieden 0 oder 1.

6. Verbindungen nach einem oder mehreren der Ansprüche 1 bis 5, **dadurch gekennzeichnet, dass** in Verbindungen gemäß Formel (1) bis (24) das Symbol Ar, gleich oder verschieden bei jedem Auftreten, für eine Arylen- oder Heteroarylengruppe steht, welche ausgewählt ist aus 1,2-Phenylen, 1,3-phenylen, 1,4-Phenylen, 1,4-Naphthylen, 9,10-Anthrylen, 2,7-Phenanthrenylen, 3,6-Phenanthrenylen, 1,6-Pyrenylen, 2,7-Pyrenylen, 2,6-Pyridinylen, 2,5-Pyridinylen, 2,2'-Biphenyl, 3,3'-Biphenyl, 4,4'-Biphenyl, 2,7-Fluorenyl oder 2,7-Spirobifluorenyl.

7. Verbindungen nach einem oder mehreren der Ansprüche 1 bis 6, **dadurch gekennzeichnet, dass** in Verbindungen gemäß Formel (1) und Formel (2) mit p=1 bzw. gemäß Formel (8) bis (12) und gemäß Formel (15) bis (24) das Symbol Y für eine Einfachbindung oder eine bivalente Gruppe, ausgewählt aus C=O, O, NAr², POAr², O-B(Ar²)-O, einer bivalenten Alkylen- oder Alkylidengruppe mit 1 bis 6 C-Atomen oder einem bivalenten aromatischen oder heteroaromatischen Ringsystem mit 5 bis 14 aromatischen Ringatomen, steht; und dass in Verbindungen gemäß Formel (1) und Formel (2) mit p = 2 bzw. gemäß Formel (13) und (14) das Symbol Y für N oder ein trivalentes aromatisches oder heteroaromatisches Ringsystem mit 6 bis 14 aromatischen Ringatomen, insbesondere für 1,3,5-Benzol oder 1,3,5-Triazin, steht; und dass in Verbindungen gemäß Formel (1) und (2) mit p > 2 Y für ein entsprechend höhervalentes aromatisches oder heteroaromatisches Ringsystem mit 5 bis 14 aromatischen Ringatomen steht.

8. Verfahren zur Herstellung von Verbindungen nach einem oder mehreren der Ansprüche 1 bis 7, in denen die Gruppe Ar bzw. Y in 6-Position des Benz[a]anthracens gebunden ist, durch Reaktion eines gegebenenfalls substituierten 2-(2'-Arylacetylen)phenyl-naphthalins mit einem Elektrophil.

9. Verfahren zur Herstellung von Verbindungen nach einem oder mehreren der Ansprüche 1 bis 7, **dadurch gekennzeichnet, dass** ein Benz[a]anthracen, welches durch eine reaktive Abgangsgruppe, insbesondere Chlor, Brom, Iod, Triflat, Tosylat, Boronsäure oder Boronsäureester, substituiert ist, mit einem funktionalisierten Aromaten oder mit einem mono- oder disubstituierten Amin gekuppelt wird, insbesondere durch eine Suzuki-Kupplung unter Palladium-Katalyse oder durch eine palladiumkatalysierte Kupplung nach Hartwig-Buchwald,

10. Verbindungen gemäß Formel (34), 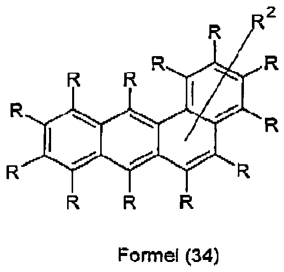 wobei R, R¹ und Ar¹ dieselbe Bedeutung haben, wie in Anspruch 1 beschrieben, und R² in der Position 2, 3, 4, 5 oder 6 des Benz[a]-anthracens gebunden ist und entsprechend an dieser Position keine Gruppe R gebunden ist, und weiterhin gilt:
R² steht für B(OR¹)₂ oder B(OAr¹)₂.

11. Oligomere, Polymere oder Dendrimere enthalten eine oder mehrere Verbindungen nach einem oder mehreren der Ansprüche 1 bis 7, wobei ein oder mehrere Reste R bzw. Ar bzw. Y Bindungen der Verbindung nach einem oder mehreren der Ansprüche 1 bis 7 zum Polymer, Oligomer oder Dendrimer darstellen.

12. Verwendung von Verbindungen nach einem oder mehreren der Ansprüche 1 bis 7 oder nach Anspruch 10 oder von Oligomeren, Dendrimeren oder Polymeren nach Anspruch 11 in elektronischen Vorrichtungen, insbesondere in organischen Elektrolumineszenzvorrichtungen.

13. Organische elektronische Vorrichtung, bevorzugt ausgewählt aus ausgewählt aus organischen Elektrolumineszenzvorrichtungen, organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Laserdioden (O-Laser) oder organischen Photorezeptoren, enthaltend mindestens eine Verbindung nach einem oder mehreren der Ansprüche 1 bis 7 oder nach Anspruch 10 bzw. mindestens ein Oligomer, Dendrimer oder Polymer nach Anspruch 11.

14. Organische Elektrolumineszenzvorrichtung nach Anspruch 13, **dadurch gekennzeichnet, dass** die Verbindung nach einem oder mehreren der Ansprüche 1 bis 10 als Hostmaterial für fluoreszierende Dotanden eingesetzt wird, wobei die fluoreszierenden Dotanden bevorzugt ausgewählt sind aus der Klasse der Monostyrylamine, der Distyrylamine, der Tristyrylamine, der Tetrastyrylamine, der Styrylphosphine, der Styrylether und der Arylamine, insbesondere der Arylamine enthaltend kondensierte aromatische Gruppen.

15. Organische Elektrolumineszenzvorrichtung nach Anspruch 13 oder 14, **dadurch gekennzeichnet, dass** die Verbindung nach einem oder mehreren der Ansprüche 1 bis 7 als emittierendes Material (Dotand), als Lochtransportmaterial, als Lochinjektionsmaterial oder als Elektronentransportmaterial eingesetzt wird.

## Claims

1. Uncharged compounds of the formula (1), 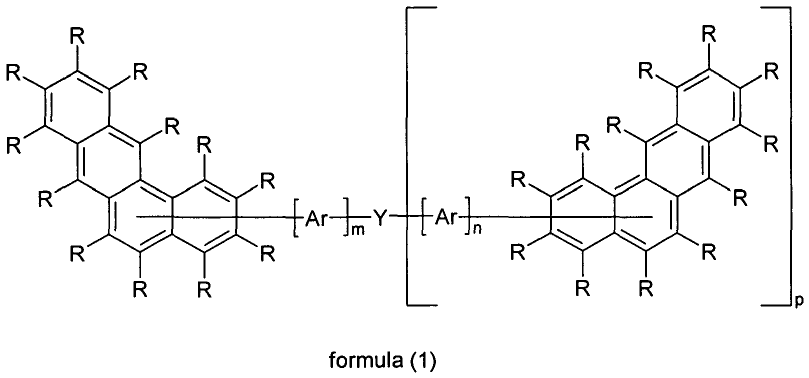 where the group Ar or Y is bonded via one of positions 2, 3, 4, 5 or 6 of the benz[a]anthracene and correspondingly no radical R is bonded at this position and where the following applies to the symbols and indices:
Ar is on each occurrence, identically or differently, a divalent aromatic or heteroaromatic ring system having 5 to 40 aromatic ring atoms, which may be substituted by one or more radicals R;
Y is, depending on the index p, a mono-, di-, tri-, tetra-, penta- or hexavalent aromatic or heteroaromatic ring system having 5 to 40 aromatic ring atoms, which may be substituted by one or more radicals R; or Y is, if p = 0, an N(Ar¹)₂, C(=O)Ar¹ or P(=O)(Ar¹)₂ group; or Y is, if p = 1, a single bond or C=O, O, S, SO, SO₂, NR¹, NAr¹, PAr¹, P(=O)Ar¹, P(=S)Ar¹, O-B(Ar¹)-O, O-BR¹-O, -CR¹=CR¹-, -C≡C-, an alkylene or alkylidene group having 1 to 20 C atoms, which may in each case also be substituted by one or more radicals R¹, where one or more non-adjacent CH₂ groups may be replaced by R¹C=CR¹, C≡C, Si(R¹)₂, Ge(R¹)₂, Sn(R¹)₂, C=O, C=S, C=Se, C=NR¹, P(=O)(R¹), SO, SO₂, NR¹, O, S or CONR¹ and where one or more H atoms may be replaced by F, Cl, Br, I, CN or NO₂; or Y is, if p = 2, equal to B, B₃O₃, CR¹, CAr¹, N, P, P=O or P=S;
R is, identically or differently on each occurrence, H, D, F, Cl, Br, I, CHO, N(Ar¹)₂, N(R¹)₂, C(=O)Ar¹, P(Ar¹)₂, P(=O)(Ar¹)₂, S(=O)Ar¹, S(=O)₂Ar¹, CR¹=CR¹Ar¹, CN, NO₂, Si(R¹)₃, B(OAr¹)₂, B(OR¹)₂, OSO₂R¹, OH, a straight-chain alkyl or thioalkoxy group having 1 to 40 C atoms or a straight-chain alkoxy group having 2 to 40 C atoms or a branched or cyclic alkyl, alkoxy or thioalkoxy group having 3 to 40 C atoms, each of which may be substituted by one or more radicals R¹, where one or more non-adjacent CH₂ groups may be replaced by R¹C=CR¹, C=C, Si(R¹)₂, Ge(R¹)₂, Sn(R¹)₂, C=O, C=S, C=Se, C=NR¹, P(=O)(R¹), SO, SO₂, NR¹, O, S or CONR¹ and where one or more H atoms may be replaced by F, Cl, Br, I, CN or NO₂, or an aromatic or heteroaromatic ring system having 5 to 60 aromatic ring atoms, which may in each case be substituted by one or more non-aromatic radicals R, or an aryloxy or heteroaryloxy group having 5 to 60 aromatic ring atoms, which may be substituted by one or more non-aromatic radicals R, or a combination of these systems; two or more adjacent substituents R here may also form a mono- or polycyclic aliphatic ring system with one another;
Ar¹ is on each occurrence, identically or differently, an aromatic or heteroaromatic ring system having 5 to 30 aromatic ring atoms, which may be substituted by one or more non-aromatic radicals R; two radicals Ar¹ which are bonded to the same nitrogen or phosphorus atom may also be linked to one another by a single bond or a bridge selected from B(R¹), C(R¹)₂, Si(R¹)₂, C=O, C=NR¹, C=C(R¹)₂, O, S, S=O, SO₂, N(R¹), P(R¹) and P(=O)R¹;
R¹ is on each occurrence, identically or differently, H or an aliphatic, aromatic and/or heteroaromatic hydrocarbon radical having 1 to 20 C atoms, in which, in addition, one or more H atoms may be replaced by F; two or more adjacent substituents R¹ here may also form a mono- or polycyclic aliphatic or aromatic ring system with one another;
m, n are on each occurrence, identically or differently, 0, 1, 2 or 3;
p is 0, 1, 2, 3, 4 or 5;
the following compounds are excluded from the invention:

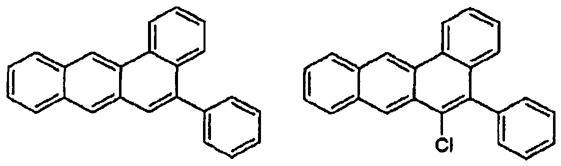

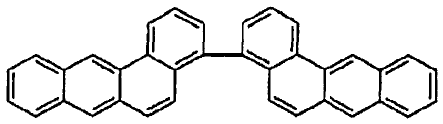

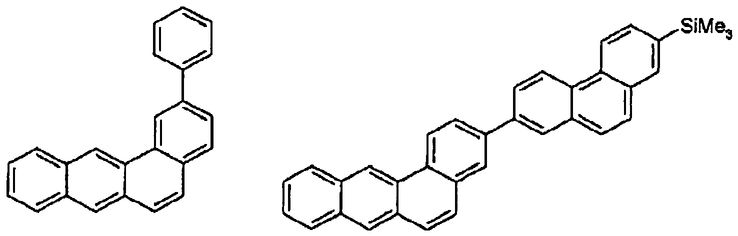

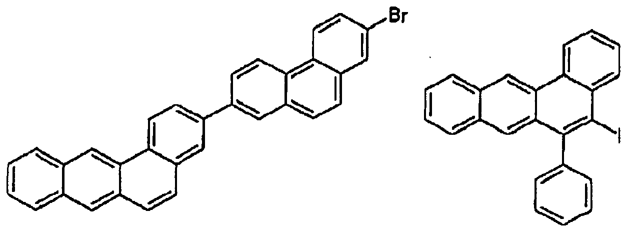

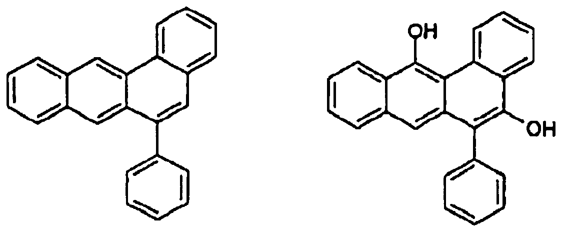

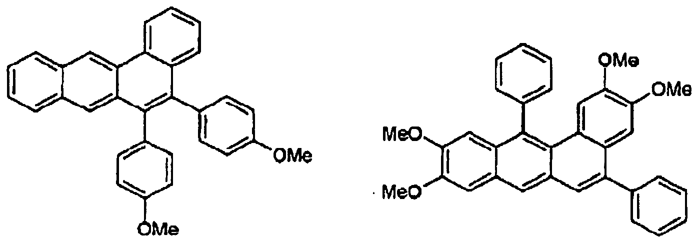

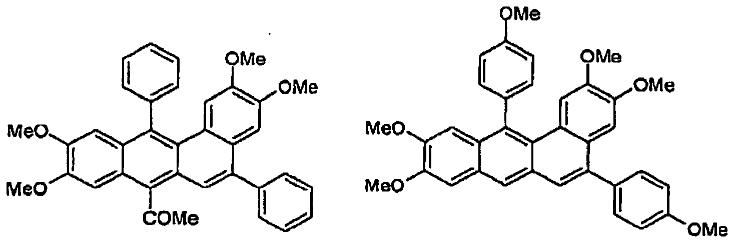

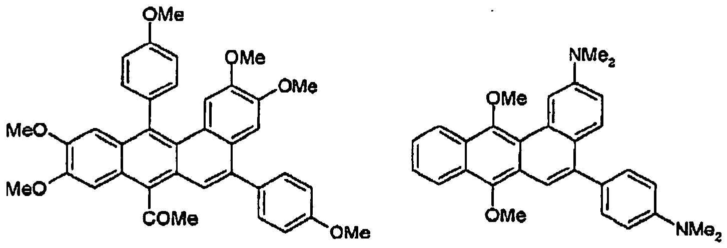

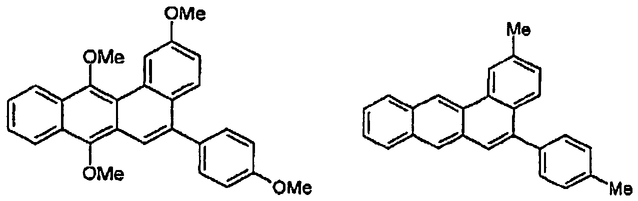

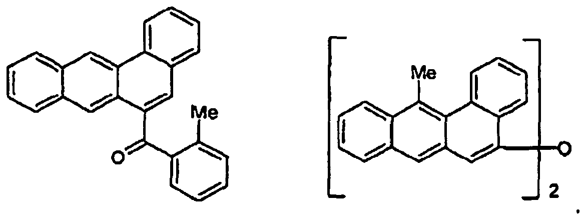

2. Compounds according to Claim 1 of the formula (2), 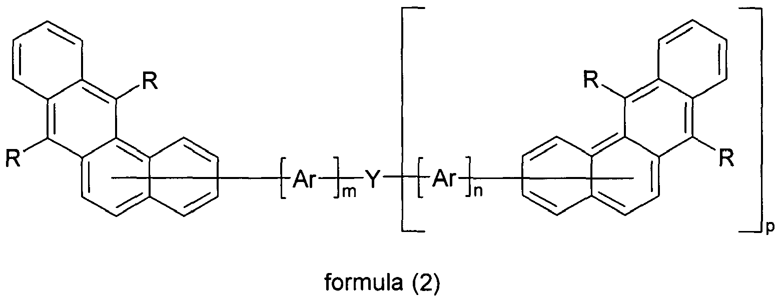 where the group Ar or Y is bonded via one of positions 2, 3, 4, 5 or 6 of the benz[a]anthracene and where the symbols and indices have the same meaning as described in Claim 1.

3. Compounds according to Claim 1 or 2, selected from formulae (3) to (24), 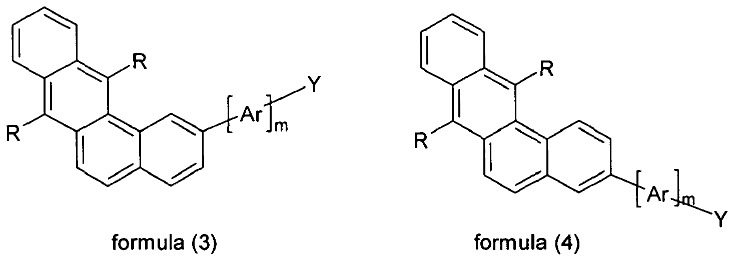 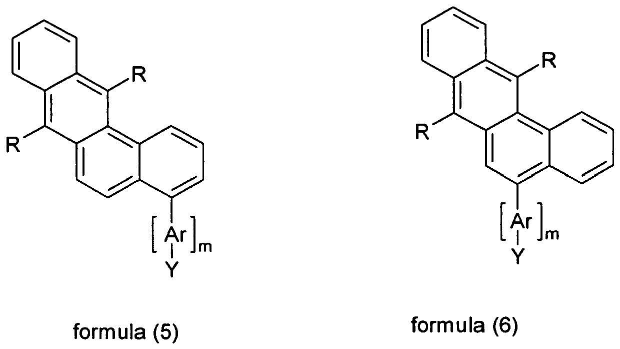 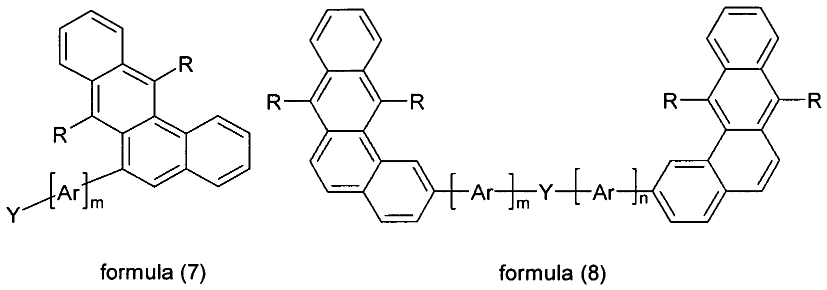 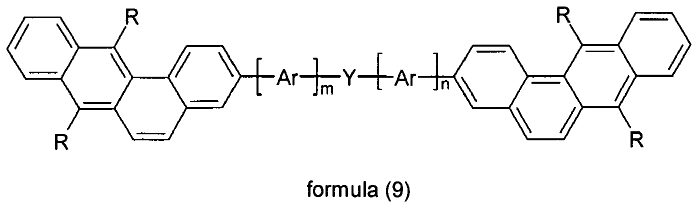 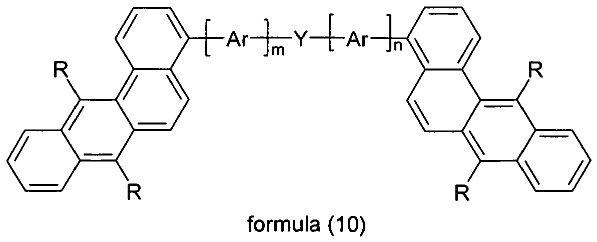 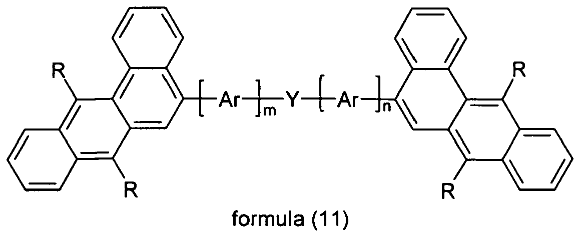 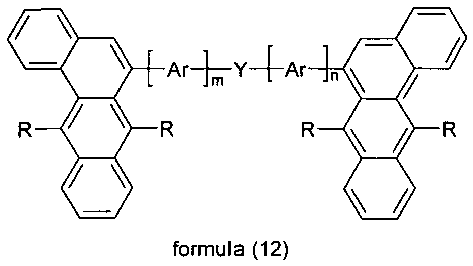 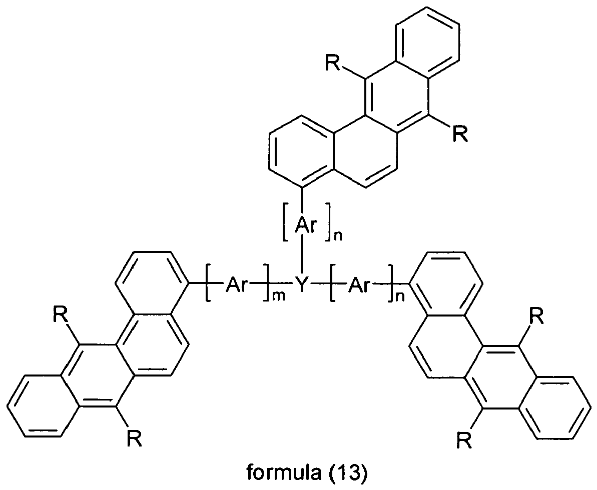 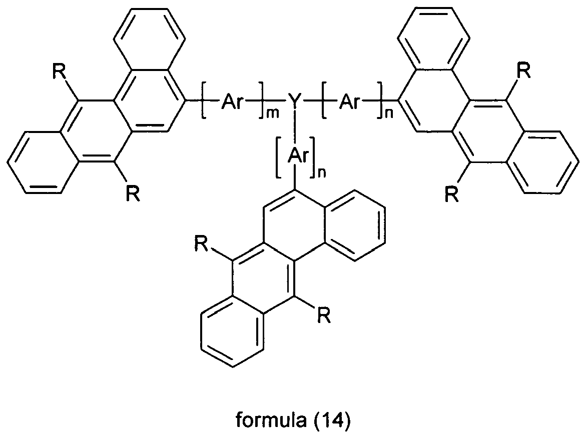 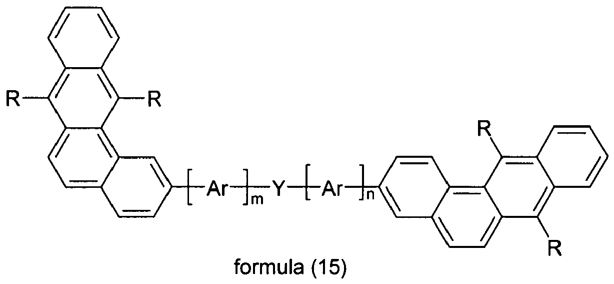 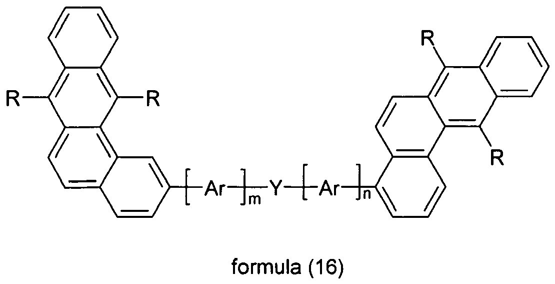 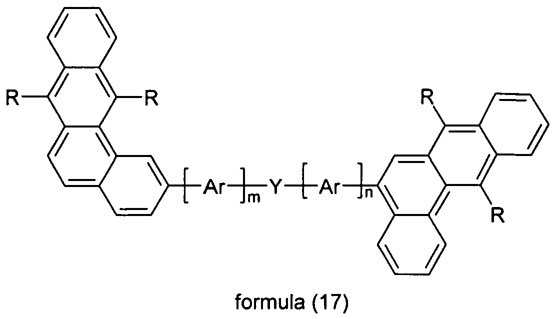 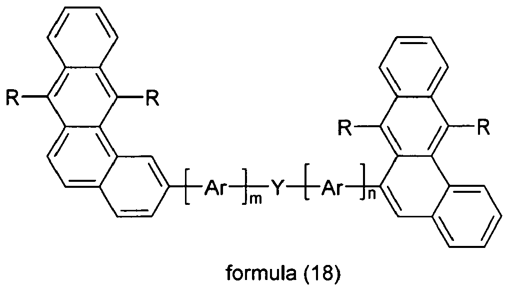 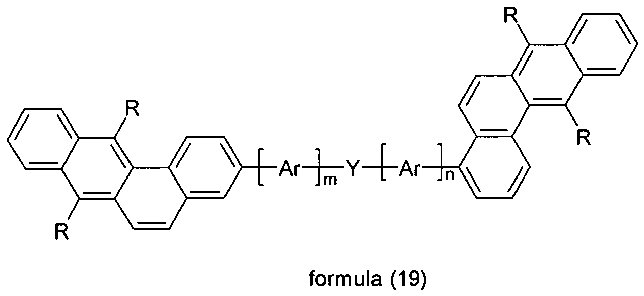 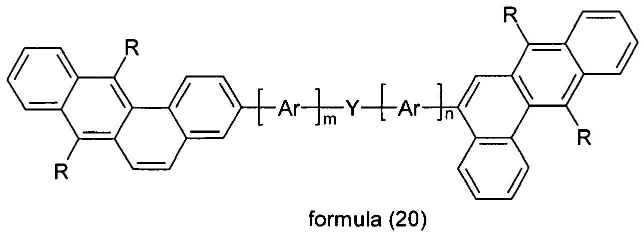 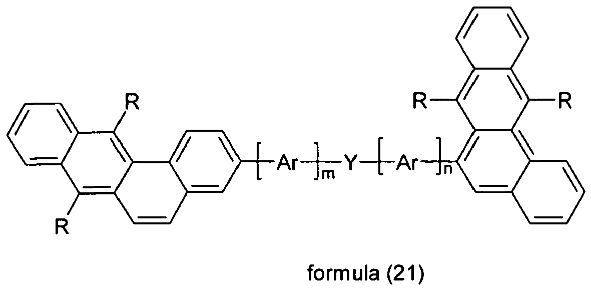 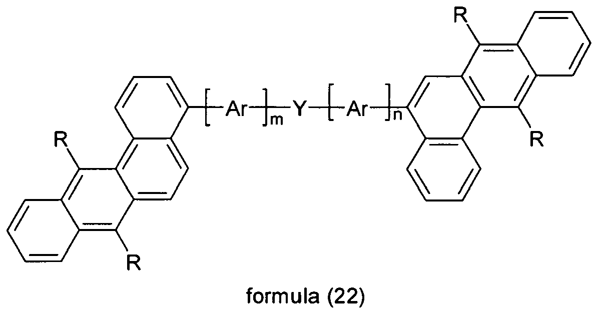 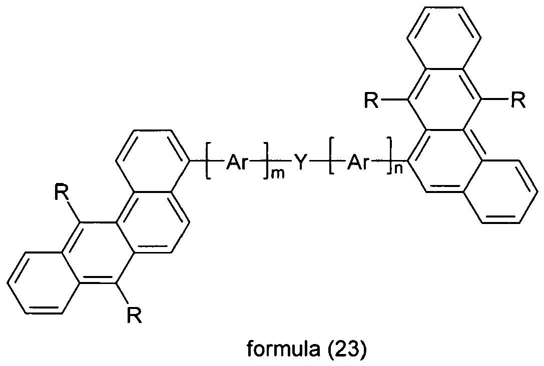 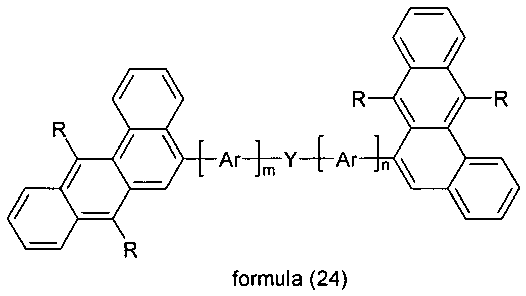 where the symbols and indices have the same meaning as described under Claim 1, where the benz[a]anthracene parent structure may also carry deuterium instead of hydrogen and where the group Y in formulae (3) to (7) stands for a monovalent aromatic or heteroaromatic ring system or an N(Ar¹)₂ group.

4. Compounds according to one or more of Claims 1 to 3, **characterised in that** the group Y is built up from the groups benzene, naphthalene, anthracene, carbazole, phenanthrene, benzanthracene, chrysene, pyrene, phenanthroline, phenanthroimidazole, 1,3,5-triazine and benzimidazole or, if p = 0, represents an N(Ar¹)₂ group.

5. Compounds according to one or more of Claims 1 to 4, **characterised in that** the group Y, for p = 0 or in compounds of the formulae (3) to (7), is selected from the groups of the following formulae (25) to (33), 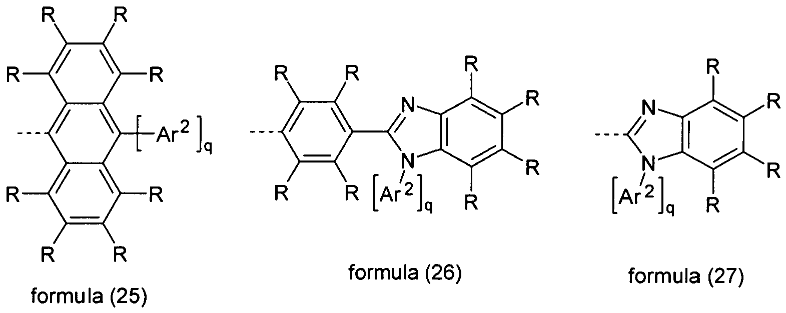 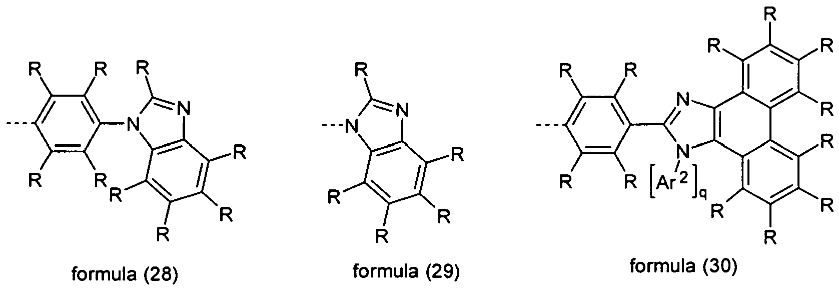 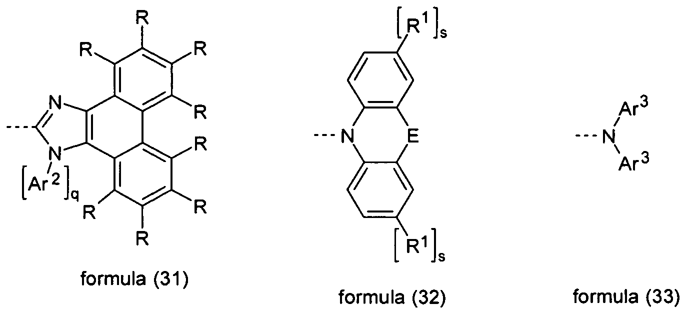 where R and R¹ have the meaning indicated in Claim 1 and furthermore:
Ar² is an aryl or heteroaryl group having 5 to 16 aromatic ring atoms, preferably phenyl, 1-naphthyl, 2-naphthyl, 9-anthryl, chrysenyl, 1-pyrenyl, 2-pyrenyl, 2-phenanthrenyl, 3-phenanthrenyl, 9-phenanthrenyl, 2-benzimidazole, 2-fluorenyl, 2-spirobifluorenyl, fluoranthenyl, 2-benz[a]anthracenyl, 3-benz[a]-anthracenyl, 4-benz[a]anthracenyl, 5-benz[a]anthracenyl or 6-benz[a]anthracenyl, each of which may be substituted by one or more radicals R¹, or, in formula (25), a group of the formula (32) or (33);
Ar³ is, identically or differently on each occurrence, an aryl or heteroaryl group having 5 to 20 aromatic ring atoms or a triarylamine group having 15 to 30 aromatic ring atoms, each of which may be substituted by one or more radicals R¹, preferably an aryl or heteroaryl group having 6 to 14 aromatic ring atoms or a triarylamine group having 18 to 30 aromatic ring atoms, preferably having 18 to 22 aromatic ring atoms, each of which may be substituted by one or more radicals R¹;
E stands for a single bond, O, S, N(R¹) or C(R¹)₂, where the two radicals R¹ may also form a spiro system through ring formation;
q is 1, 2 or 3;
s is on each occurrence, identically or differently, 0 or 1.

6. Compounds according to one or more of Claims 1 to 5, **characterised in that**, in compounds of the formulae (1) to (24), the symbol Ar stands, identically or differently on each occurrence, for an arylene or heteroarylene group selected from 1,2-phenylene, 1,3-phenylene, 1,4-phenylene, 1,4-naphthylene, 9,10-anthrylene, 2,7-phenanthrenylene, 3,6-phenanthrenylene, 1,6-pyrenylene, 2,7-pyrenylene, 2,6-pyridinylene, 2,5-pyridinylene, 2,2'-biphenyl, 3,3'-biphenyl, 4,4'-biphenyl, 2,7-fluorenyl or 2,7-spirobifluorenyl.

7. Compounds according to one or more of Claims 1 to 6, **characterised in that**, in compounds of the formula (1) and formula (2) where p = 1 or of the formulae (8) to (12) and of the formulae (15) to (24), the symbol Y stands for a single bond or for a divalent group selected from C=O, O, NAr², POAr², O-B(Ar²)-O, a divalent alkylene or alkylidene group having 1 to 6 C atoms or a divalent aromatic or heteroaromatic ring system having 5 to 14 aromatic ring atoms; and **in that**, in compounds of the formula (1) and formula (2) where p = 2 or of the formulae (13) and (14), the symbol Y stands for N or a trivalent aromatic or heteroaromatic ring system having 5 to 14 aromatic ring atoms, in particular for 1,3,5-benzene or 1,3,5-triazine; and **in that**, in compounds of the formulae (1) and (2) where p > 2, Y stands for a correspondingly polyvalent aromatic or heteroaromatic ring system having 5 to 14 aromatic ring atoms.

8. Process for the preparation of compounds according to one or more of Claims 1 to 7 in which the group Ar or Y is bonded in the 6-position of the benz[a]anthracene, by reaction of an optionally substituted 2-(2'-arylacetylene)phenylnaphthalene with an electrophile.

9. Process for the preparation of compounds according to one or more of Claims 1 to 7, **characterised in that** a benz[a]anthracene which is substituted by a reactive leaving group, in particular chlorine, bromine, iodine, triflate, tosylate, boronic acid or boronic acid ester, is coupled to a functionalised aromatic group or to a mono- or disubstituted amine, in particular by a Suzuki coupling with palladium catalysis or by a palladium-catalysed Hartwig-Buchwald coupling.

10. Compounds of the formula (34) 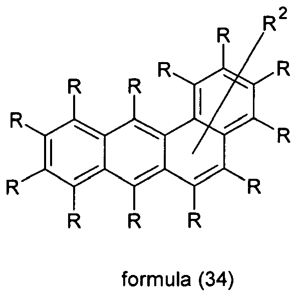 where R, R¹ and Ar¹ have the same meaning as described in Claim 1, and R² is bonded in position 2, 3, 4, 5 or 6 of the benz[a]anthracene and correspondingly no group R is bonded at this position, and furthermore:
R² stands for B(OR¹)₂ or B(OAr¹)₂.

11. Oligomers, polymers or dendrimers comprising one or more compounds according to one or more of Claims 1 to 7, where one or more radicals R or Ar or Y represent bonds from the compound according to one or more of Claims 1 to 7 to the polymer, oligomer or dendrimer.

12. Use of compounds according to one or more of Claims 1 to 7 or according to Claim 10 or of oligomers, dendrimers or polymers according to Claim 11 in electronic devices, in particular in organic electroluminescent devices.

13. Organic electronic device, preferably selected from organic electroluminescent devices, organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), light-emitting electrochemical cells (LECs), organic laser diodes (O-lasers) or organic photoreceptors, comprising at least one compound according to one or more of Claims 1 to 7 or according to Claim 10 or at least one oligomer, dendrimer or polymer according to Claim 11.

14. Organic electroluminescent device according to Claim 13, **characterised in that** the compound according to one or more of Claims 1 to 10 is employed as host material for fluorescent dopants, where the fluorescent dopants are preferably selected from the class of the monostyrylamines, the distyrylamines, the tristyrylamines, the tetrastyrylamines, the styrylphosphines, the styryl ethers and the arylamines, in particular the arylamines containing condensed aromatic groups.

15. Organic electroluminescent device according to Claim 13 or 14, **characterised in that** the compound according to one or more of Claims 1 to 7 is employed as emitting material (dopant), as hole-transport material, as hole-injection material or as electron-transport material.

## Revendications

1. Composés non chargés de la formule (1), 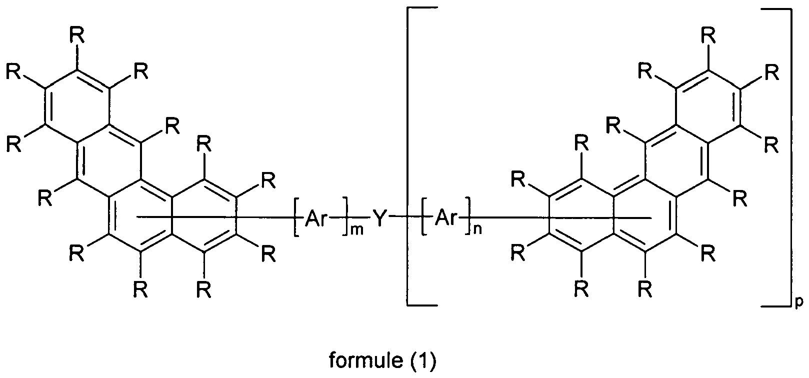 dans laquelle le groupe Ar ou Y est lié via l'une des positions 2, 3, 4, 5 ou 6 du benz[a]anthracène et en correspondance, aucun radical R n'est lié au niveau de cette position et dans laquelle ce qui suit s'applique aux symboles et indices :
Ar est, pour chaque occurrence, de manière identique ou différente, un système de cycle aromatique ou hétéroaromatique divalent comportant de 5 à 40 atomes de cycle aromatique, lesquels peuvent être substitués par un ou plusieurs radicaux R ;
Y est, en fonction de l'indice p, un système de cycle aromatique ou hétéroaromatique mono-, di-, tri-, tétra-, penta- ou hexavalent comportant de 5 à 40 atomes de cycle aromatique, lesquels peuvent être substitués par un ou plusieurs radicaux R ; ou Y est, si p = 0, un groupe N(Ar¹)₂, C(=O)Ar¹ ou P(=O)(Ar¹)₂ ; ou Y est, si p = 1, une liaison simple ou C=O, O, S, SO, SO₂, NR¹, NAr¹, PAr¹, P(=O)Ar¹, P(=S)Ar¹, O-B(Ar¹)-O, O-BR¹-O, -CR¹=CR¹-, -C≡C-, un groupe alkylène ou alkylidène comportant de 1 à 20 atomes de C, lesquels peuvent également dans chaque cas être substitués par un ou plusieurs radicaux R¹, où un ou plusieurs groupes CH₂ non adjacents peut/peuvent être remplacé(s) par R¹C=CR¹, C=C, Si(R¹)₂, Ge(R¹)₂, Sn(R¹)₂, C=O, C=S, C=Se, C=NR¹, P(=O)(R¹), SO, SO₂, NR¹, O, S ou CONR¹ et où un ou plusieurs atomes de H peut/peuvent être remplacé(s) par F, Cl, Br, I, CN ou NO₂ ; ou Y est, si p = 2, égal à B, B₃O₃, CR¹, CAr¹, N, P, P=O ou P=S ;
R est, de manière identique ou différente pour chaque occurrence, H, D, F, Cl, Br, I, CHO, N(Ar¹)₂, N(R¹)₂, C(=O)Ar¹, P(Ar¹)₂, P(=O)(Ar¹)₂, S(=O)Ar¹, S(=O)₂Ar¹, CR¹=CR¹Ar¹, CN, NO₂, Si(R¹)₃, B(OAr¹)₂, B(OR¹)₂, OSO₂R¹, OH, un groupe alkyle ou thioalcoxy en chaîne droite comportant de 1 à 40 atomes de C ou un groupe alcoxy en chaîne droite comportant de 2 à 40 atomes de C ou un groupe alkyle, alcoxy ou thioalcoxy ramifié ou cyclique comportant de 3 à 40 atomes de C, dont chacun peut être substitué par un ou plusieurs radicaux R¹, où un ou plusieurs groupes CH₂ non adjacents peut/peuvent être remplacé(s) par R¹C=CR¹, C=C, Si(R¹)₂, Ge(R¹)₂, Sn(R¹)₂, C=O, C=S, C=Se, C=NR¹, P(=O)(R¹), SO, SO₂, NR¹, O, S ou CONR¹ et où un ou plusieurs atomes de H peut/peuvent être remplacé(s) par F, Cl, Br, I, CN ou NO₂, ou un système de cycle aromatique ou hétéroaromatique comportant de 5 à 60 atomes de cycle aromatique, lesquels peuvent dans chaque cas être substitués par un ou plusieurs radicaux non aromatiques R, ou un groupe aryloxy ou hétéroaryloxy comportant de 5 à 60 atomes de cycle aromatique, lesquels peuvent être substitués par un ou plusieurs radicaux non aromatiques R, ou une combinaison de ces systèmes ; deux substituants R adjacents ou plus peuvent ici également former un système de cycle aliphatique mono- ou polycyclique l'un avec l'autre ou les uns avec les autres ;
Ar¹ est, pour chaque occurrence, de manière identique ou différente, un système de cycle aromatique ou hétéroaromatique comportant de 5 à 30 atomes de cycle aromatique, lesquels peuvent être substitués par un ou plusieurs radicaux non aromatiques R ; deux radicaux Ar¹ qui sont liés au même atome d'azote ou de phosphore peuvent également être liés l'un à l'autre par une liaison simple ou un pont choisi parmi B(R¹), C(R¹)₂, Si(R¹)₂, C=O, C=NR¹, C=C(R¹)₂, O, S, S=O, SO₂, N(R¹), P(R¹) et P(=O)R¹ ;
R¹ est, pour chaque occurrence, de manière identique ou différente, H ou un radical hydrocarbone aliphatique, aromatique et/ou hétéroaromatique comportant de 1 à 20 atomes de C, où, en outre, un ou plusieurs atomes de H peut/peuvent être remplacé(s) par F ; deux substituants R¹ adjacents ou plus peuvent ici également former un système de cycle aliphatique ou aromatique mono- ou polycyclique l'un avec l'autre ou les uns avec les autres ;
m, n sont, pour chaque occurrence, de manière identique ou différente, 0, 1, 2 ou 3 ; p est 0, 1, 2, 3, 4 ou 5 ;
les composés qui suivent sont exclus de l'invention :

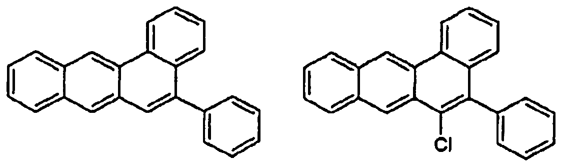

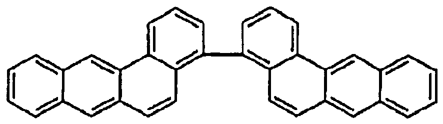

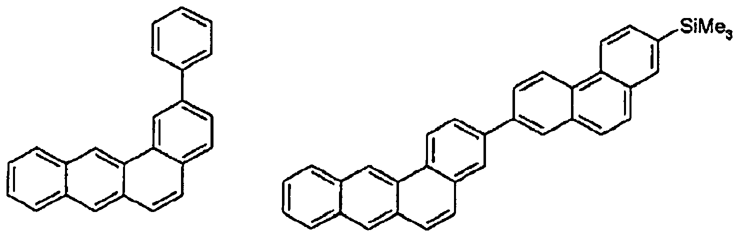

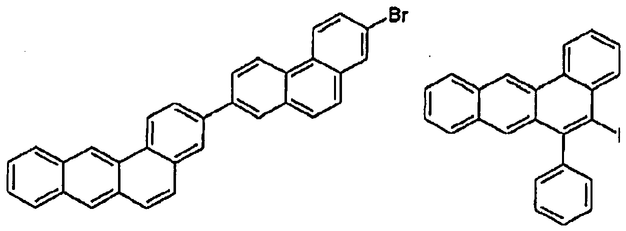

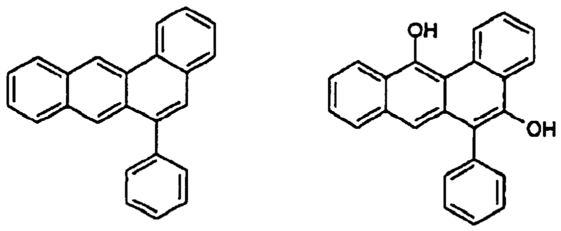

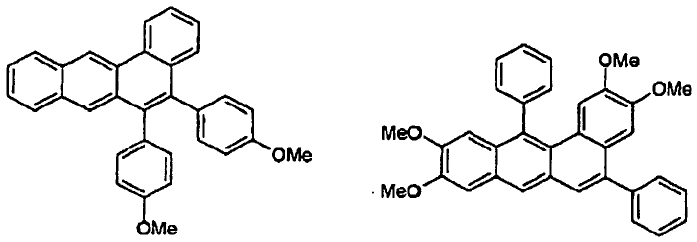

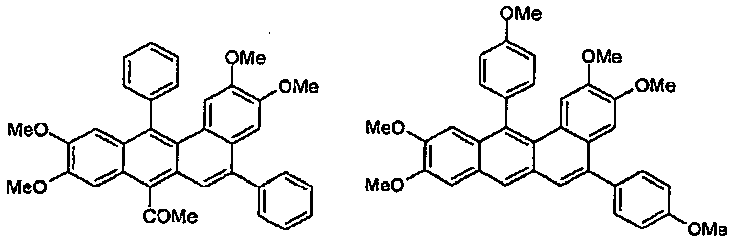

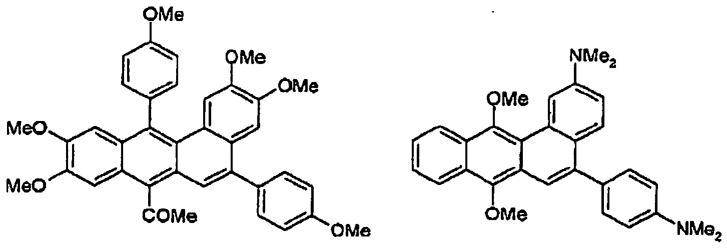

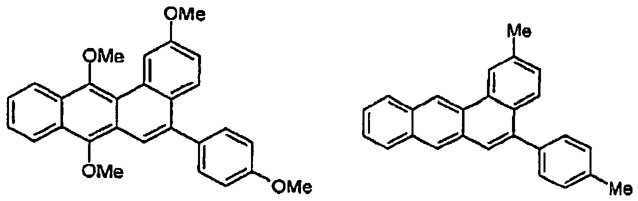

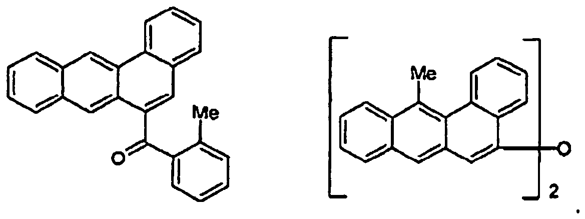

2. Composés selon la revendication 1 de la formule (2), 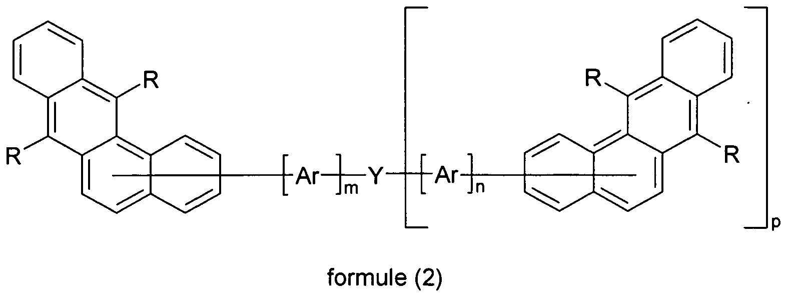 dans laquelle le groupe Ar ou Y est lié via l'une des positions 2, 3, 4, 5 ou 6 du benz[a]anthracène et dans laquelle les symboles et indices présentent la même signification que décrit selon la revendication 1.

3. Composés selon la revendication 1 ou 2, choisis parmi les formules (3) à (24), 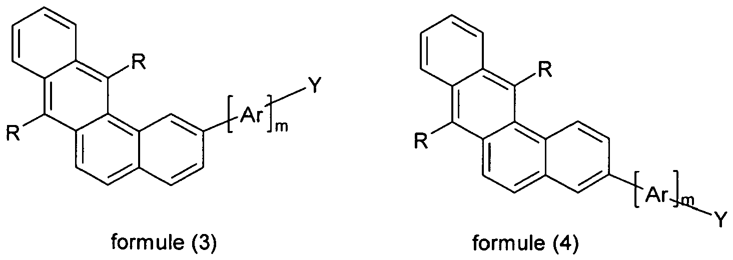 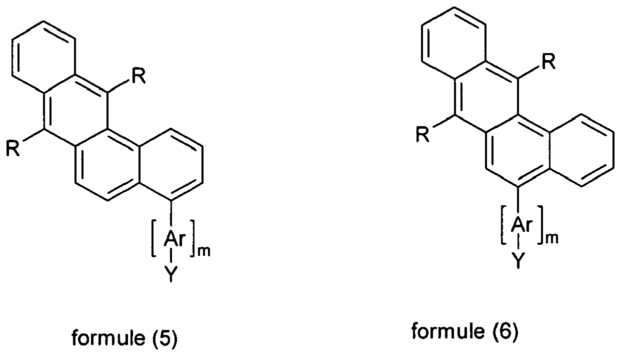 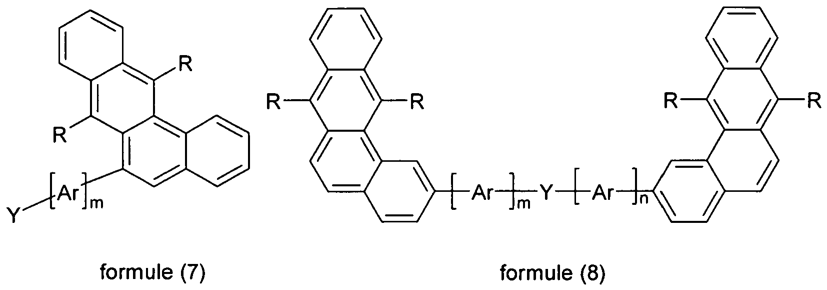 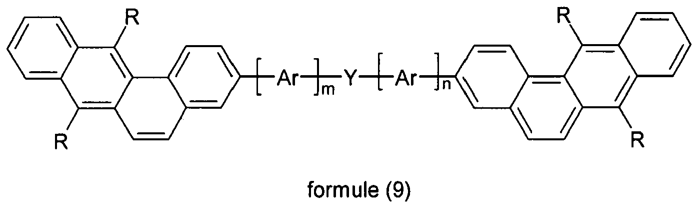 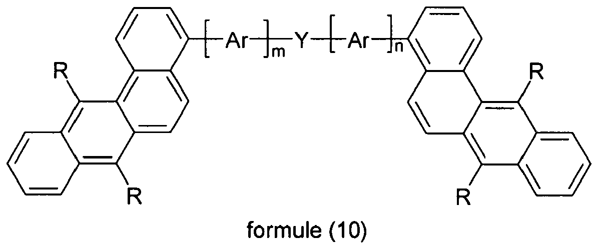 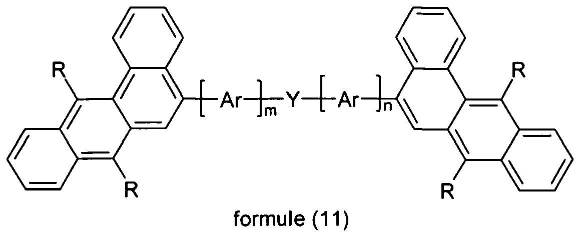 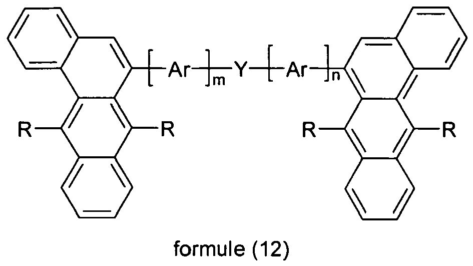 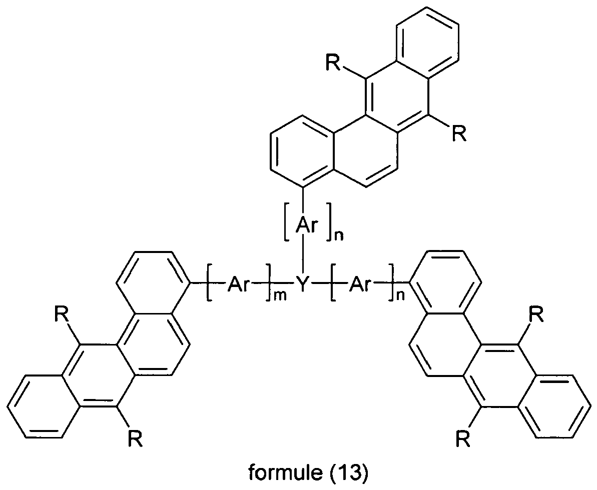 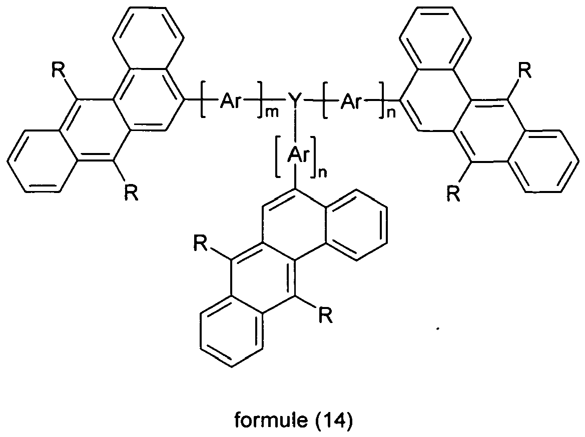 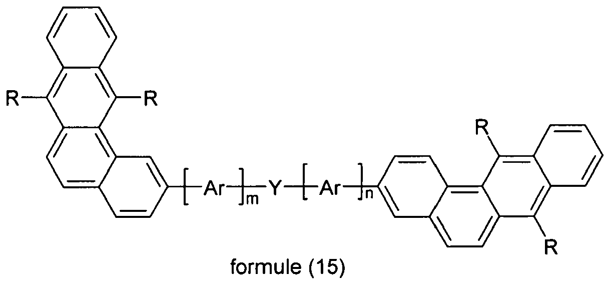 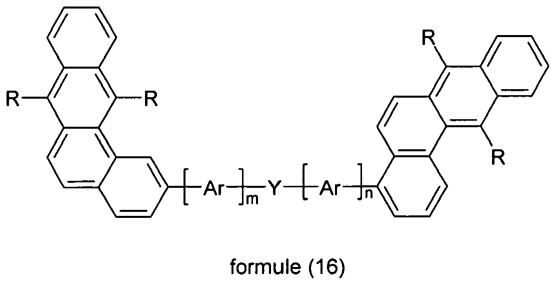 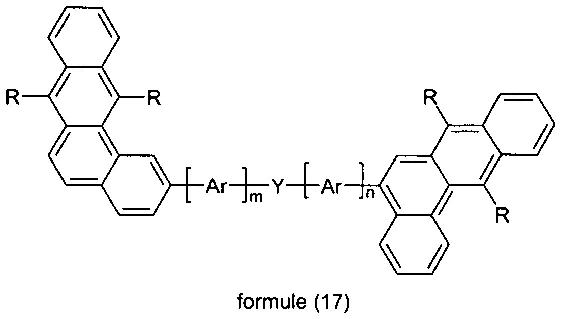 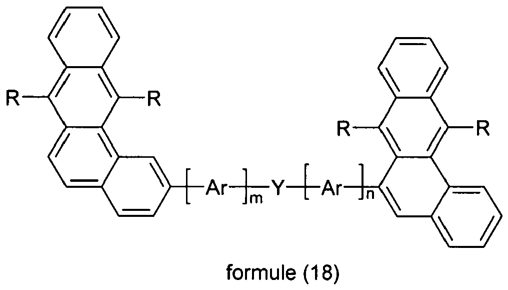 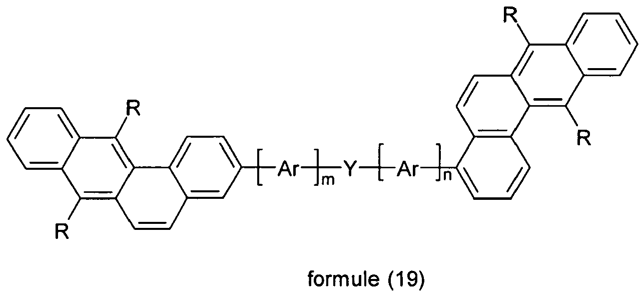 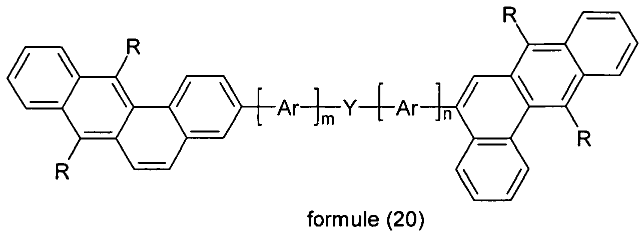 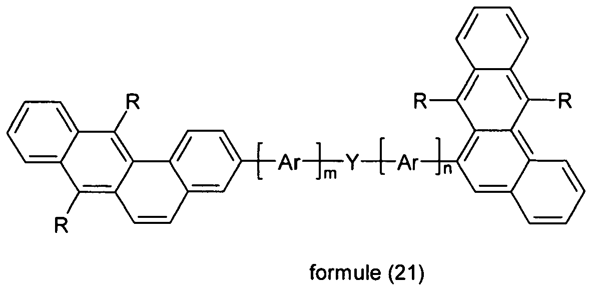 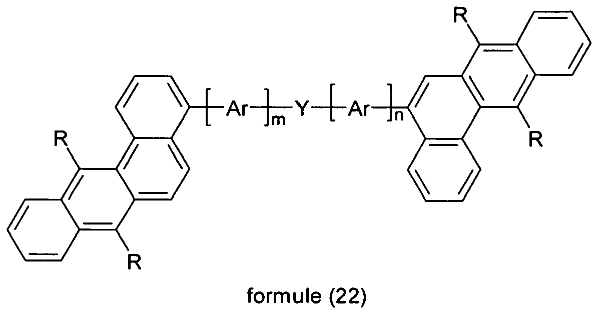 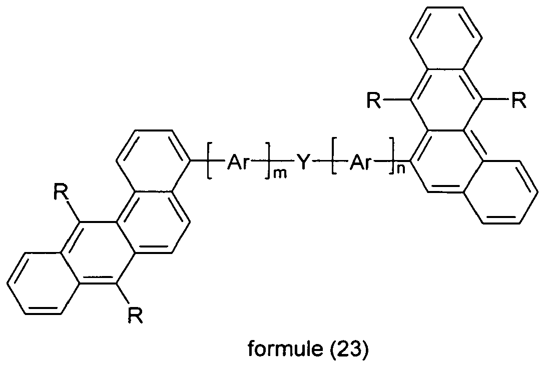 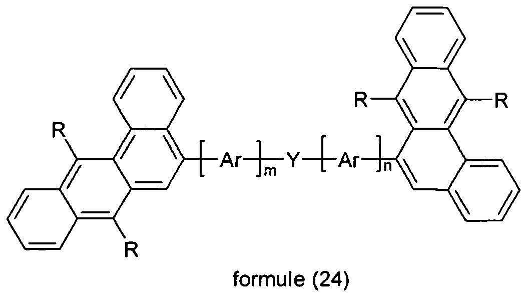 dans lesquelles les symboles et indices présentent la même signification que décrit selon la revendication 1, où la structure parente du benz[a]anthracène peut également être porteuse de deutérium en lieu et place de l'hydrogène et où le groupe Y dans les formules (3) à (7) représente un système de cycle aromatique ou hétéroaromatique monovalent ou un groupe N(Ar¹)₂.

4. Composés selon une ou plusieurs des revendications 1 à 3, **caractérisés en ce que** le groupe Y est élaboré à partir des groupes benzène, naphtalène, anthracène, carbazole, phénanthrène, benzanthracène, chrysène, pyrène, phénanthroline, phénanthroimidazole, 1,3,5-triazine et benzimidazole ou, si p = 0, représente un groupe N(Ar¹)₂.

5. Composés selon une ou plusieurs des revendications 1 à 4, **caractérisés en ce que** le groupe Y, pour p = 0 ou dans des composés des formules (3) à (7), est choisi parmi les groupes des formules qui suivent (25) à (33), 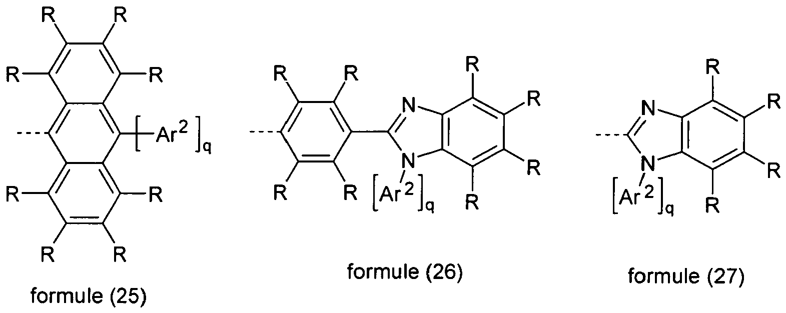 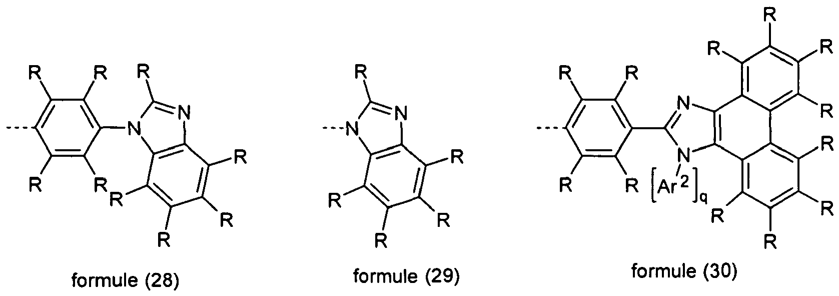 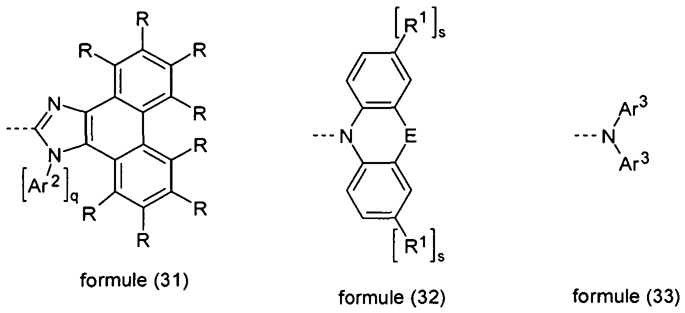 dans lesquelles R et R¹ présentent la signification indiquée selon la revendication 1 et en outre :
Ar² est un groupe aryle ou hétéroaryle comportant de 5 à 16 atomes de cycle aromatique, de préférence phényle, 1-naphtyle, 2-naphtyle, 9-anthryle, chrysényle, 1-pyrényle, 2-pyrényle, 2-phénanthrényle, 3-phénanthrényle, 9-phénanthrényle, 2-benzimidazole, 2-fluorényle, 2-spirobifluorényle, fluoranthényle, 2-benz[a]anthracényle, 3-benz[a]anthracényle, 4-benz-[a]anthracényle, 5-benz[a]anthracényle ou 6-benz[a]anthracényle, dont chacun peut être substitué par un ou plusieurs radicaux R¹, ou, dans la formule (25), un groupe de la formule (32) ou (33) ;
Ar³ est, de manière identique ou différente pour chaque occurrence, un groupe aryle ou hétéroaryle comportant de 5 à 20 atomes de cycle aromatique ou un groupe triarylamine comportant de 15 à 30 atomes de cycle aromatique, dont chacun peut être substitué par un ou plusieurs radicaux R¹, de préfé- rence un groupe aryle ou hétéroaryle comportant de 6 à 14 atomes de cycle aromatique ou un groupe triarylamine comportant de 18 à 30 atomes de cycle aromatique, de préférence comportant de 18 à 22 atomes de cycle aromatique, dont chacun peut être substitué par un ou plusieurs radicaux R¹;
E représente une liaison simple, O, S, N(R¹) ou C(R¹)₂, où les deux radicaux R¹ peuvent également former un système spiro par l'intermédiaire d'une formation de cycle ;
q est 1, 2 ou 3 ;
s est, pour chaque occurrence, de manière identique ou différente, 0 ou 1.

6. Composés selon une ou plusieurs des revendications 1 à 5, **caractérisés en ce que**, dans des composés des formules (1) à (24), le symbole Ar représente, de manière identique ou différente pour chaque occurrence, un groupe arylène ou hétéroarylène choisi parmi 1,2-phénylène, 1,3-phénylène, 1,4-phénylène, 1,4-naphtylène, 9,10-anthrylène, 2,7-phénanthrénylène, 3,6-phénanthrénylène, 1,6-pyrénylène, 2,7-pyrénylène, 2,6-pyridinylène, 2,5-pyridinylène, 2,2'-biphényle, 3,3'-biphényle, 4,4'-biphényle, 2,7-fluorényle ou 2,7-spirobifluorényle.

7. Composés selon une ou plusieurs des revendications 1 à 6, **caractérisés en ce que**, dans des composés de la formule (1) et de la formule (2) où p = 1 ou des formules (8) à (12) et des formules (15) à (24), le symbole Y représente une liaison simple ou un groupe divalent choisi parmi C=O, O, NAr², POAr², O-B(Ar²)-O, un groupe alkylène ou alkylidène divalent comportant de 1 à 6 atomes de C ou un système de cycle aromatique ou hétéroaromatique divalent comportant de 5 à 14 atomes de cycle aromatique ; et **en ce que**, dans des composés de la formule (1) et de la formule (2) où p = 2 ou des formules (13) et (14), le symbole Y représente N ou un système de cycle aromatique ou hétéroaromatique trivalent comportant de 5 à 14 atomes de cycle aromatique, en particulier 1,3,5-benzène ou 1,3,5-triazine ; et **en ce que**, dans des composés des formules (1) et (2) où p > 2, Y représente en correspondance un système de cycle aromatique ou hétéroaromatique polyvalent comportant de 5 à 14 atomes de cycle aromatique.

8. Procédé pour la préparation de composés selon une ou plusieurs des revendications 1 à 7, dans lequel le groupe Ar ou Y est lié à la position 6 du benz[a]anthracène, par réaction d'un 2-(2'-arylacétylène)phénylnaphtalène en option substitué avec un électrophile.

9. Procédé pour la préparation de composés selon une ou plusieurs des revendications 1 à 7, **caractérisé en ce qu'**un benz[a]anthracène qui est substitué par un groupe partant réactif, en particulier chlore, brome, iode, triflate, tosylate, acide boronique ou ester d'acide boronique, est couplé à un groupe aromatique fonctionnalisé ou à un amine mono- ou disubstitué, en particulier au moyen d'un couplage de Suzuki avec catalyse de palladium ou au moyen d'un couplage de Hartwig-Buchwald catalysé par palladium.

10. Composés de la formule (34) 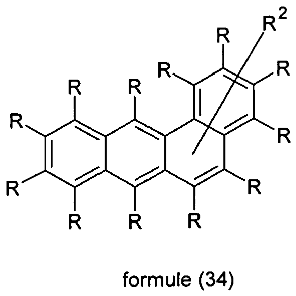 dans laquelle R, R¹ et Ar¹ présentent la même signification que décrit selon la revendication 1, et R² est lié à la position 2, 3, 4, 5 ou 6 du benz[a]anthracène et en correspondance, aucun groupe R n'est lié au niveau de cette position, et en outre :
R² représente B(OR¹)₂ ou B(OAr¹)₂.

11. Oligomères, polymères ou dendrimères comprenant un ou plusieurs composés selon une ou plusieurs des revendications 1 à 7, dans lesquels un ou plusieurs radicaux R ou Ar ou Y représentent des liaisons depuis le composé selon une ou plusieurs des revendications 1 à 7 sur le polymère, l'oligomère ou le dendrimère.

12. Utilisation de composés selon une ou plusieurs des revendications 1 à 7 ou selon la revendication 10 ou d'oligomères, de dendrimères ou de polymères selon la revendication 11 dans des dispositifs électroniques, en particulier dans des dispositifs électroluminescents organiques.

13. Dispositif électronique organique, de préférence choisi parmi des dispositifs électroluminescents organiques, des transistors à effet de champ organiques (O-FET), des transistors à film mince organiques (O-TFT), des transistors émetteurs de lumière organiques (O-LET), des circuits intégrés organiques (O-IC), des cellules solaires organiques (O-SC), des dispositifs à extinction de champ organiques (O-FQD), des cellules électrochimiques émettrices de lumière (LEC), des diodes laser organiques (O-laser) ou des photorécepteurs organiques, comprenant au moins un composé selon une ou plusieurs des revendications 1 à 7 ou selon la revendication 10 ou au moins un oligomère, un dendrimère ou un polymère selon la revendication 11.

14. Dispositif électroluminescent organique selon la revendication 13, **caractérisé en ce que** le composé selon une ou plusieurs des revendications 1 à 10 est utilisé en tant que matériau hôte pour des dopants fluorescents, où les dopants fluorescents sont de préférence choisis parmi la classe des monostyrylamines, des distyrylamines, des tristyrylamines, des tétrastyrylamines, des styrylphosphines, des styryléthers et des arylamines, en particulier des arylamines contenant des groupes aromatiques condensés.

15. Dispositif électroluminescent organique selon la revendication 13 ou 14, **caractérisé en ce que** le composé selon une ou plusieurs des revendications 1 à 7 est utilisé en tant que matériau d'émission (dopant), en tant que matériau de transport de trous, en tant que matériau d'injection de trous ou en tant que matériau de transport d'électrons.
